(19) Europäisches Patentamt
European Patent Office
Office européen des brevets

(11) Publication number: **0 320 992 B1**

# (12) EUROPEAN PATENT SPECIFICATION

(45) Date of publication of patent specification: **27.07.94**

(51) Int. Cl.[5]: **C07D 487/04**, C07D 495/14, C07D 519/00, C07F 7/10, A61K 31/55, //(C07D487/04, 249:00,243:00),(C07D495/14, 333:00,249:00,243:00), (C07D519/00,495:00,471:00)

(21) Application number: **88121165.0**

(22) Date of filing: **16.12.88**

(54) **Triazolodiazepine derivatives.**

(30) Priority: **18.12.87 US 134726**
**03.08.88 US 227948**

(43) Date of publication of application:
**21.06.89 Bulletin 89/25**

(45) Publication of the grant of the patent:
**27.07.94 Bulletin 94/30**

(84) Designated Contracting States:
**AT BE CH DE ES FR GB GR IT LI LU NL SE**

(56) References cited:
**EP-A- 0 240 899**
**WO-A-88/09333**

(73) Proprietor: **F. HOFFMANN-LA ROCHE AG**
**Postfach 3255**
**CH-4002 Basel(CH)**

(72) Inventor: **Walser, Armin**
**19 Crane Avenue**
**West Caldwell, N.J.(US)**

(74) Representative: **Lederer, Franz, Dr. et al**
**Lederer, Keller & Riederer**
**Patentanwälte**
**Prinzregentenstrasse 16**
**D-80538 München (DE)**

**EP 0 320 992 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compounds of the general formula

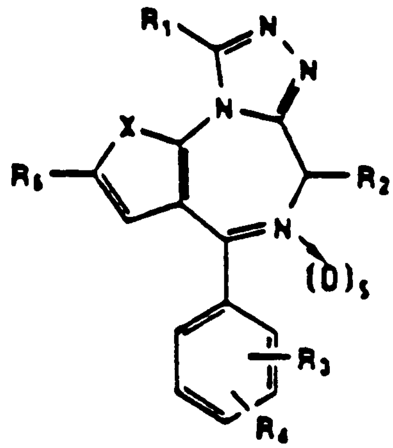

I

wherein X is -CH = CH- or S;

$R_1$ is lower alkyl, lower alkoxy or trifluoromethyl;

$R_2$ is hydrogen, lower alkyl, lower alkoxy, hydroxy or lower alkanoyloxy;

$R_3$ and $R_4$, independently, are hydrogen, chlorine, fluorine, lower alkyl or lower alkoxy;

$R_5$ is a radical of the formula $R_6$-$(CH_2)_n$-C≡C-or $R_7$-O-$(CH_2)_m$-C≡C-.

$R_6$ and $R_7$ are aryl or a heterocyclic radical; n is an integer from 0 to 2; m is an integer from 1 to 2 and s is an integer from 0 to 1; with the proviso that, when s is 1, $R_2$ cannot be hydroxy, lower alkoxy or lower alkanoyloxy; that, when n is 0, $R_6$ must be attached through a carbon to carbon bond, and that $R_7$ is always attached through a carbon to oxygen bond,
and, when at least one asymmetric centre is present, their enantiomers and racemates, and pharmaceutically acceptable acid addition salts thereof.

The compounds of formula I exhibit activity as platelet activating factor (PAF) antagonists and are, therefore, useful in disease states characterized by excess platelet activating factor or for the prevention and treatment of cardiovascular diseases, pulmonary diseases, immunological disorders, inflammatory diseases, dermatological disorders, shocks or transplant rejections.

As used herein, the term "lower alkyl", denotes a straight or branched chain saturated hydrocarbon group containing from 1 to 7 carbon atoms, preferably from 1 to 4 carbon atoms, for example, methyl, ethyl, propyl, isopropyl, butyl, t-butyl, neopentyl, pentyl, heptyl, and the like. The term "lower alkoxy" denotes an alkyl ether group in which the alkyl group is as described above, for example, methoxy, ethoxy, propoxy, pentoxy and the like.

The term "aryl" preferably denotes phenyl or naphthyl or phenyl or phenyl or naphthyl mono-, di- or trisubstituted by chlorine, fluorine, lower alkyl or lower alkoxy.

The term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted, preferably mono- or disubstituted, by lower alkyl, lower alkoxy, oxo, hydroxy, chlorine or fluorine. It is understood that heterocyclic refers to a carbocyclic moiety in which one or more of the carbon atoms are replaced, independently, by oxygen, nitrogen or sulfur.

Exemplary of monocyclic 5- or 6-membered aromatic heterocyclic radicals are pyridinyl, imidazolinyl, thienyl, 2-chlorothienyl, furyl, pyrimidinyl, oxazolinyl or the like.

Exemplary of monocyclic 5-, 6- or 7-membered non-aromatic heterocyclic radicals are

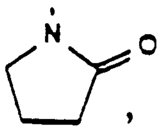, 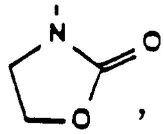, 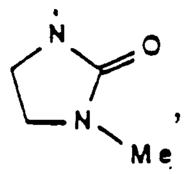, 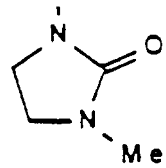,

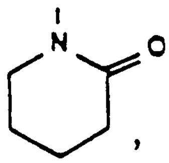, 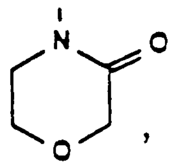, 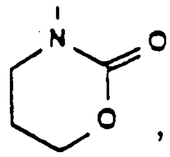, 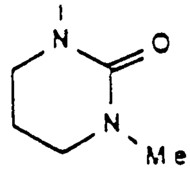,

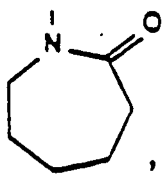, 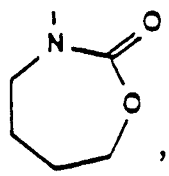, 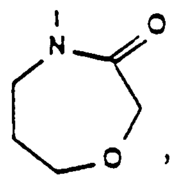,

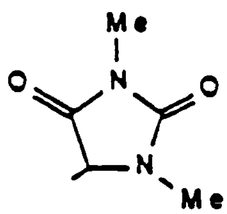, 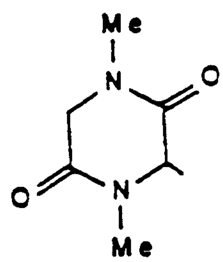,

and the like.

Exemplary of bicyclic heterocyclic radicals are:

(a) 5,5-ringsystems:

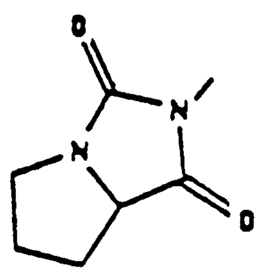

and the like;

(b) 6,5-ringsystems:

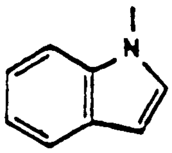, 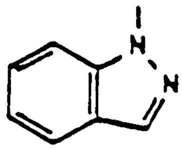, 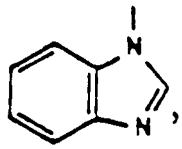, 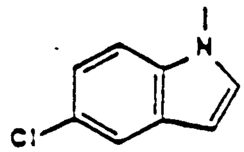,

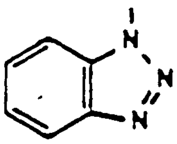, 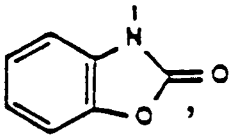, 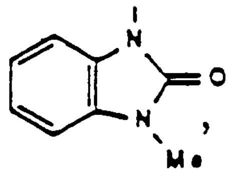, 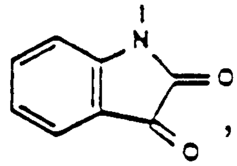,

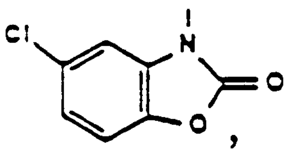, 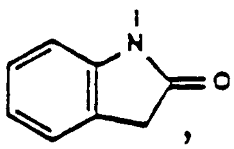, 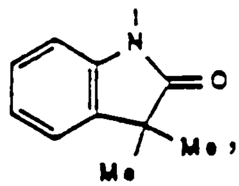, 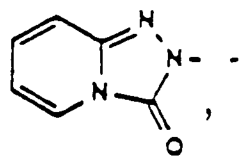,

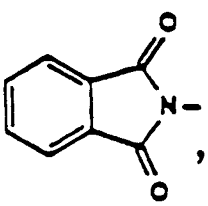, 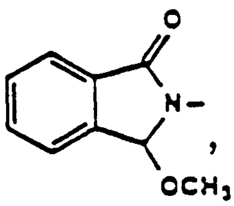, 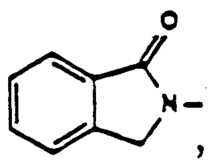, 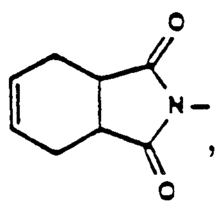,

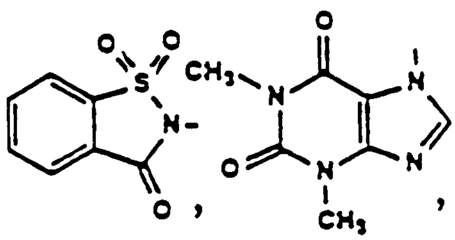, , 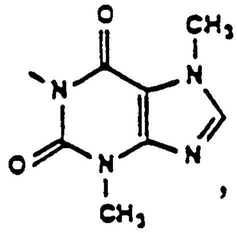, 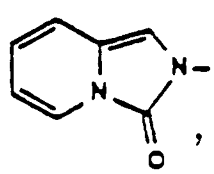,

and the like;

(c) 6,6 ringsystems:

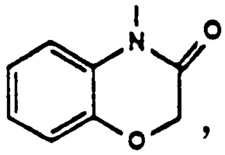, 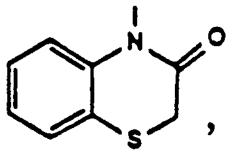, 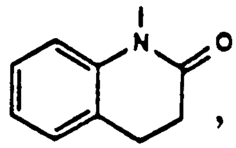, 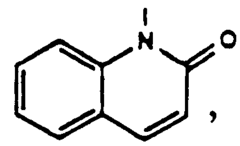,

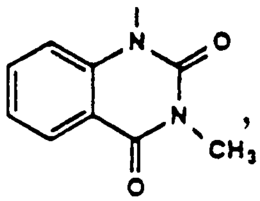, 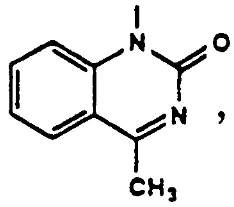, 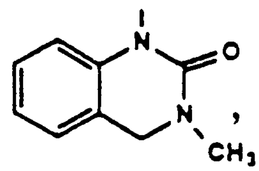, 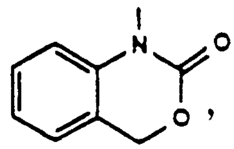,

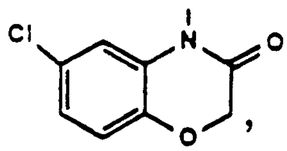, 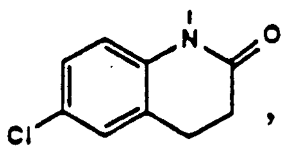, 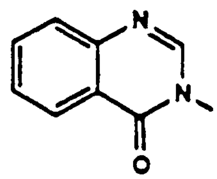, 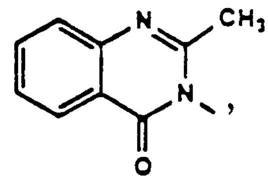,

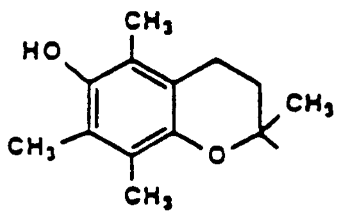

and the like; and

(d) 6,7-ringsystems:

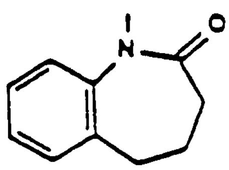, 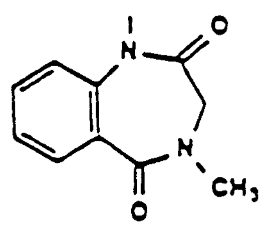, 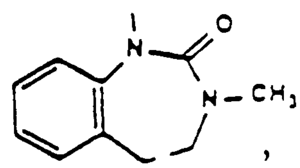,

and the like.

Exemplary of tricyclic heterocyclic radicals are 5,5,6-, 5,6,6-, 6,6,6- and 6,6,7-ringsystems:

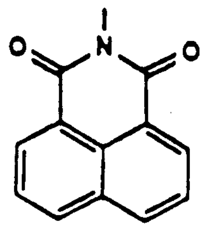,
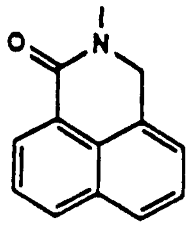,
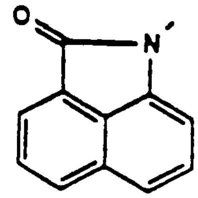,
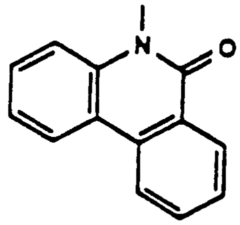,
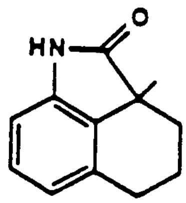,
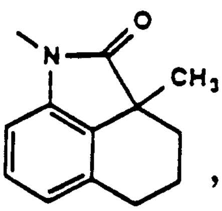,
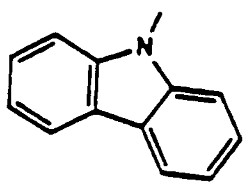,
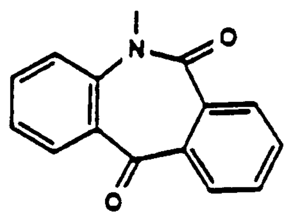,
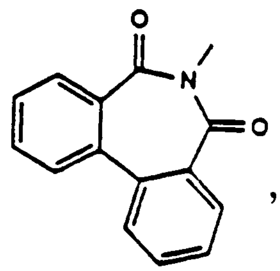,
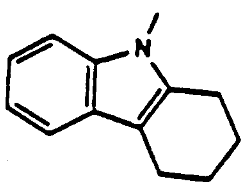
or
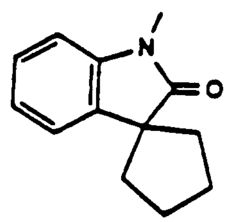

and the like.

As used herein. and as is evident from the nomenclature and structures utilized throughout the specification, the structural representation -≡ is -C≡CH and -≡- is -C≡C-.

A preferred group of compounds of formula I are those wherein $R_1$ is methyl or ethyl; $R_2$ is hydrogen; $R_3$ is fluorine or chlorine, $R_4$ is hydrogen, s is 0 and X and $R_5$ are as previously described except that n is 1 or 2.

A more preferred group of compounds of formula I are those wherein $R_1$ is methyl, $R_2$ is hydrogen, $R_3$ is fluorine or chlorine at the 2-position of the phenyl moiety, $R_4$ is hydrogen, s is 0, $R_5$ is $R_6$-$(CH_2)_n$-C≡C- or $R_7$-O-$(CH_2)_m$-C≡C-, m and n are 1, $R_6$ is a bi- or tricyclic heterocyclic radical and $R_7$ is aryl.

A most preferred group of compounds of formula I are those wherein X is S, $R_1$ is methyl, $R_2$ is hydrogen, $R_3$ is chlorine and at the 2-position of the phenyl moiety, $R_4$ is hydrogen, s is 0, $R_5$ is $R_6$-$(CH_2)_n$-C≡C-, n is 1 and $R_6$ is

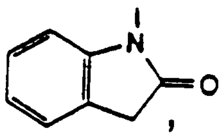, 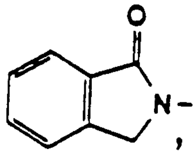, 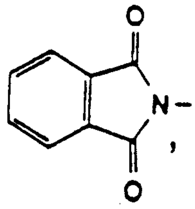,

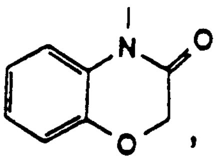, 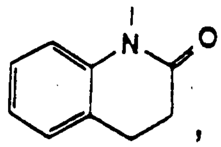, 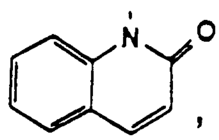,

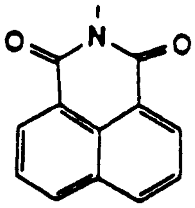, 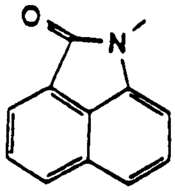, 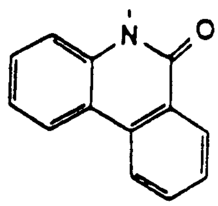 and

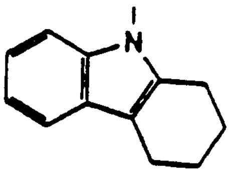

Most preferred compounds of the invention are:

5-{3-[4-(2-chlorophenyl)-9-methyl-6H-thieno-[3,2-f][1,2,4]triazolo[4,3-a][1,4]diazepin-2-yl]-2-propynyl}-phenanthridin-6(5H)-one;

4-(2-chlorophenyl)-2-[3-(1,2,3,4-tetrahydro-9H-carbazol-9-yl)-1-propynyl]-9-methyl-6H-thieno-[3,2-f]-[1,2,4]triazolo[4,3-a][1,4]diazepine;

1-{3-[4-(2-chlorophenyl)-9-methyl-6H-thieno[3,2,-f][1,2,4]triazolo[4,3-a][1,4]diazepin-2-yl]-2-propynyl}-3,4-dihydro-2-(1H)-quinolinone;

2-[3-[4-(2-chlorophenyl)-9-methyl-6H-thieno[3,2-f][1,2,4]triazolo[4,3-a] [1,4]diazepin-2-yl]-2-propynyl]-1H-benz[de]isoquinoline-1,3(2H)-dione;

1-[3-[4-(2-chlorophenyl)-9-methyl-6H-thieno[3,2-f][1,2,4]triazolo[4,3-a] [1,4]diazepin-2-yl]-2-propynyl]-benz[cd]indol-2(1H)-one; and

4-[3-[4-(2-chlorophenyl)-9-methyl-6H-thieno[3,2-f][1,2,4]triazolo[4,3-a] [1,4]diazepin-2-yl]-2-propynyl]-2H-1,4-benzoxazin-3(4H)-one.

Other preferred compounds of the invention are:

1-[3-[4-(2-chlorophenyl)-9-methyl-6H-thieno[3,2-f][1,2,4]triazolo[4,3-a] [1,4]diazepin-2-yl]-2-propynyl]-1H-indole-2,3-dione;

1-[3-[4-(2-chlorophenyl)-9-methyl-6H-thieno[3,2-f][1,2,4]triazolo[4,3-a] [1,4]diazepin-2-yl]-2-propynyl]-1,3-dihydro-2H-indol-2-one;

2-[3-[4-(2-chlorophenyl)-9-methyl-6H-thieno[3,2-f][1,2,4]triazolo[4,3-a] [1,4]diazepin-2-yl]-2-propynyl]-1,2,4-triazolo[4,3-a]pyridin-3(2H)-one;

2-[3-[4-(2-chlorophenyl)-9-methyl-6H-thieno[3,2-f][1,2,4]triazolo[4,3-a] [1,4]diazepin-2-yl]-2-propynyl]-1,2-benzisothiazol-3(2H)-one 1,1-dioxide;

4-(2-chlorophenyl)-2-[3-(1H-indazol-1-yl)-1-propynyl]-9-methyl-6H-thieno[3,2-f][1,2,4]triazolo[4,3-a][1,4]-diazepine;

2-[3-(1H-benzimidazol-1-yl)-1-propynyl]-4-(2-chlorophenyl)-9-methyl-6H-thieno[3,2-f][1,2,4]triazolo[4,3-a]-[1,4]diazepine;

2-[3-[6-(2-fluorophenyl)-1-methyl-4H-[1,2,4]triazolo[4,3-a][1,4]benzodiazepin-8-yl]-2-propynyl]-1H-isoindole-1,3(2H)-dione; and

4-[3-[6-(2-fluorophenyl)-1-methyl-4H-[1,2,4]triazolo[4,3-a][1,4]benzodiazepin-8-yl]-2-propynyl]-2H-1,4-benzoxazin-3(4H)-one.

Exemplary compounds of formula I of the invention are:

2-[3-(1H-Benztriazol-1-yl)-1-propynyl]-4-(2-chlorophenyl)-9-methyl-6H-thieno[3,2-f][1,2,4]triazolo[4,3-a]-[1,4]diazepine;

4-[3-[6-(2-fluorophenyl)-1-methyl-4H-[1,2,4]triazolo[4,3-a][1,4]benzodiazepin-8-yl]-2-propynyl]-2H-1,4-benzothiazin-3(4H)-one;

2-[3-[(1-ethyl-6-(2-fluorophenyl)-4H-[1,2,4]triazolo[4,3-a][1,4]benzodiazepin-8-yl]-2-propynyl]-1H-isoindole-1,3(2H)-dione;

2-[3-[6-(2-chlorophenyl)-1-methyl-4H-[1,2,4]triazolo[4,3-a][1,4]benzodiazepin-8-yl]-2-propynyl]-1H-isoindole-1,3(2H)-dione;

2-[3-[6-(2-chlorophenyl)-1-methyl-4H-[1,2,4]triazolo[4,3-a][1,4]benzodiazepin-8-yl]-2-propynyl]-1H-benz-[de]isoquinolin-1,3(2H)-dione;

2-[3-[6-(2-fluorophenyl)-1-methyl-4H-[1,2,4]triazolo[4,3-a][1,4]benzodiazepin-8-yl]-2-propynyl]-1H-benz-[de]isoquinolin-1,3(2H)-dione;

3-[3-[6-(2-fluorophenyl)-1-methyl-4H-[1,2,4]triazolo[4,3-a][1,4]benzodiazepin-8-yl]-2-propynyl]-4(3H)-quinazolinone;

3-[3-[6-(2-fluorophenyl)-1-methyl-4H-[1,2,4]triazolo[4,3-a][1,4]benzodiazepin-8-yl]-2-propynyl]-2-methyl-4(3H)-quinazolinone;

2-[3-[6-(2-fluorophenyl)-1-methyl-4H-[1,2,4]triazolo[4,3-a][1,4]benzodiazepin-8-yl]-2-propynyl]-2,3-dihydro-1H-isoindol-1-one;

rac-2-[3-[6-(2-fluorophenyl)-1-methyl-4H-[1,2,4]triazolo[4,3-a][1,4]benzodiazepin-8-yl]-2-propynyl]-2,3-dihydro-3-methoxy-1H-isoindol-1-one;

2-[3-[6-(2-fluorophenyl)-1-methyl-4H-[1,2,4]triazolo[4,3-a][1,4]benzodiazepin-8-yl]-2-propynyl]-1,2,4-triazolo[4,3-a]pyridin-3(2H)-one;

2-[3-[6-(2-fluorophenyl)-1-methyl-4H-[1,2,4]triazolo[4,3-a][1,4]benzodiazepin-8-yl]-2-propynyl]-1,2-benzisothiazol-3(2H)-one 1,1-dioxide;

2-[3-[6-(2-fluorophenyl)-1-methyl-4H-[1,2,4]triazolo[4,3-a][1,4]benzodiazepin-8-yl]-2-propynyl]-tetrahydro-1H-pyrrolo[1,2-c]imidazole-1,3-(2H)-dione;

1-[3-[6-(2-fluorophenyl)-1-methyl-4H-[1,2,4]triazolo[4,3-a][1,4]benzodiazepin-8-yl]-2-propynyl]-1,3-dihydro-2H-indol-2-one;

2-[3-[6-(2-fluorophenyl)-1-methyl-4H-[1,2,4]triazolo[4,3-a][1,4]benzodiazepin-8-yl]-2-propynyl]-3a,4,7,7a-tetrahydro-1H-isoindole-1,3(2H)-dione;

1-[3-[6-(2-fluorophenyl)-1-methyl-4H-[1,2,4]triazolo[4,3-a][1,4]benzodiazepin-8-yl]-2-propynyl]-3,4-dihydro-4-methyl-1H-1,4-benzodiazepine-2,5-dione

1-[3-[6-(2-fluorophenyl)-1-methyl-4H-[1,2,4]triazolo[4,3-a][1,4]benzodiazepin-8-yl]-2-propynyl]-3,7-dihydro-3,7-dimethyl-1H-purine-2,6-dione;

2-[4-[6-(2-fluorophenyl)-1-methyl-4H-[1,2,4]triazolo[4,3-a][1,4]benzodiazepin-8-yl]-2-butynyl]-1H-isoindole-1,3(2H)-dione;

8-[3-(1H-benzimidazol-1-yl)-1-propynyl]-6-(2-chlorophenyl)-1-methyl-4H-[1,2,4]triazolo[4,3-a][1,4]-benzodiazepine;

8-[3-(1H-benzimidazol-1-yl)-1-propynyl]-6-(2-fluorophenyl)-1-methyl-4H-[1,2,4]triazolo[4,3-a][1,4]-benzodiazepine;

6-(2-fluorophenyl)-8-[3-(1H-indol-1-yl)-1-propynyl]-1-methyl-4H-[1,2,4]triazolo[4,3-a][1,4]benzodiazepine;

6-(2-chlorophenyl)-8-[3-(1H-indol-1-yl)-1-propynyl]-1-methyl-4H-[1,2,4]triazolo[4,3-a][1,4]benzodiazepine;

6-(2-fluorophenyl)-8-[3-(1H-indazol-1-yl)-1-propynyl]-1-methyl-4H-[1,2,4]triazolo[4,3-a][1,4]-benzodiazepine;

3-[3-[6-(2-fluorophenyl)-1-methyl-4H-[1,2,4]triazolo[4,3-a][1,4]benzodiazepin-8-yl]-2-propynyl]-2,3-dihydro-1,3-benzoxazol-2-one;

3-[3-[6-(2-chlorophenyl)-1-methyl-4H-[1,2,4]triazolo[4,3-a][1,4]benzodiazepin-8-yl]-2-propynyl]-2,3-dihydro-1,3-benzoxazol-2-one;

1-[3-[6-(2-fluorophenyl)-1-methyl-4H-[1,2,4]triazolo[4,3-a][1,4]benzodiazepin-8-yl]-1,3-dihydro-3-methyl-benzimidazol-2(2H)-one;

3-[3-[4-(2-chlorophenyl)-9-methyl-6H-thieno[3,2-f][1,2,4]triazolo[4,3-a][1,4]diazepin-2-yl]-2-propynyl]-2,3-dihydro-1,3-benzoxazol-2-one;

1-[3-[4-(2-chlorophenyl)-9-methyl-6H-thieno[3,2-f][1,2,4]triazolo[4,3-a][1,4]diazepin-2-yl]-2-propynyl]-1,3-dihydro-3-methyl-benzimidazol-2(2H)-one;

4-[3-[4-(2-chlorophenyl)-9-methyl-6H-thieno[3,2-f][1,2,4]triazolo[4,3-a][1,4]diazepin-2-yl]-2-propynyl]-2H-1,4-benzothiazin-3(4H)-one;

2-[3-[4-(2-chlorophenyl)-9-methyl-6H-thieno[3,2-f][1,2,4]triazolo[4,3-a][1,4]diazepin-2-yl]-2-propynyl]-1H-isoindol-1,3(2H)-dione;

3-[3-[4-(2-chlorophenyl)-9-methyl-6H-thieno[3,2-f][1,2,4]triazolo[4,3-a][1,4]diazepin-2-yl]-4(3H)-quinazolinone;

3-[3-[4-(2-chlorophenyl)-9-methyl-6H-thieno[3,2-f][1,2,4]triazolo[4,3-a][1,4]diazepin-2-yl]-2-propynyl]-1,3-dihydro-1-methylquinazolin-2,4-dione;

1-[3-[4-(2-chlorophenyl)-9-methyl-6H-thieno[3,2-f][1,2,4]triazolo[4,3-a][1,4]diazepin-2-yl]-2-propynyl]-1,3-dihydro-3-methylquinazolin-2,4(2H,4H)-dione;

2-[3-[4-(2-chlorophenyl)-9-methyl-6H-thieno[3,2-f][1,2,4]triazolo[4,3-a][1,4]diazepin-2-yl]-2-propynyl]-2,3-dihydro-1H-isoindol-1-one;

2-[3-[4-(2-chlorophenyl)-9-methyl-6H-thieno[3,2-f][1,2,4]triazolo[4,3-a][1,4]diazepin-2-yl]-2-propynyl]-tetrahydro-1H-pyrrolo[1,2-c]imidazole-1,3(2H)-dione;

2-[3-[4-(2-chlorophenyl)-9-methyl-6H-thieno[3,2-f][1,2,4]triazolo[4,3-a][1,4]diazepin-2-yl]-2-propynyl]-3a,4,7,7a-tetrahydro-1H-isoindole-1,3(2H)-dione;

1-[3-[4-(2-chlorophenyl)-9-methyl-6H-thieno[3,2-f][1,2,4]triazolo[4,3-a][1,4]diazepin-2-yl]-2-propynyl]-3,4-dihydro-4-methyl-1H-1,4-benzodiazepine-2,5-dione;

2-[4-[4-(2-chlorophenyl)-9-methyl-6H-thieno[3,2-f][1,2,4]triazolo[4,3-a][1,4]diazepin-2-yl]-3-butynyl]-1H-isoindole-1,3(2H)-dione;

1-[3-[4-(2-chlorophenyl)-9-methyl-6H-thieno[3,2-f][1,2,4]triazolo[4,3-a][1,4]diazepin-2-yl]-2-propynyl]-2-(1H)-quino linone;

1-[3-[4-(2-chlorophenyl)-9-methyl-6H-thieno[3,2-f][1,2,4]triazolo[4,3-a][1,4]diazepin-2-yl]-2-propynyl]-3,4-dihydro-2(1H)-quinolinone;

1-[3-[4-(2-chlorophenyl)-9-methyl-6H-thieno[3,2-f][1,2,4]triazolo[4,3-a][1,4]diazepin-2-yl]-2-propynyl]-4-methyl-2(1H)-quinazolinone;

1-[3-[4-(2-chlorophenyl)-9-methyl-6H-thieno[3,2-f][1,2,4]triazolo[4,3-a][1,4]diazepin-2-yl]-2-propynyl]-1,4-dihydro-2H-3,1-benzoxazin-2-one;

1-[3-[4-(2-chlorophenyl)-9-methyl-6H-thieno[3,2-f][1,2,4]triazolo[4,3-a][1,4]diazepin-2-yl]-2-propynyl]-3,4-dihydro-3-methyl-2(1H)-quinazolinone;

1-[3-[4-(2-chlorophenyl)-9-methyl-6H-thieno[3,2-f][1,2,4]triazolo[4,3-a][1,4]diazepin-2-yl]-2-propynyl]-3,7-dihydro-3,7-dimethyl-1H-purin-2,6-dione;

7-[3-[4-(2-chlorophenyl)-9-methyl-6H-thieno[3,2-f][1,2,4]triazolo[4,3-a][1,4]diazepin-2-yl]-2-propynyl]-1,3-dihydro-1,3-dimethyl-1H-purine-2,6-dione;

5-[3-[4-(2-chlorophenyl)-9-methyl-6H-thieno[3,2-f][1,2,4]triazolo[4,3-a][1,4]diazepin-2-yl]-2-propynyl]-dibenz[b,e]azepine-6,11(5H)-dione;

2-[3-(9H-carbazol-9-yl)-1-propynyl]-4-(2-chlorophenyl)-9-methyl-6H-thieno[3,2-f][1,2,4]triazolo[4,3-a][1,4]-diazepine;

6-(2-chlorophenyl)-1-methyl-8-(3-phenoxy-1-propynyl)-4H-[1,2,4]triazolo[4,3-a][1,4]benzodiazepine;

6-(2-chlorophenyl)-1-methyl-8-[3-(1-naphthyloxy)-1-propynyl]-4H-[1,2,4]triazolo[4,3-a][1,4]-benzodiazepine;

6-(2-chlorophenyl)-1-methyl-8-[3-(3-pyridinyloxy-)-1-propynyl]-4H-[1,2,4]triazolo[4,3-a][1,4]-benzodiazepine;

6-(2-fluorophenyl)-1-methyl-8-[3-(2-pyrimidyloxy)-1-propynyl]-4H-[1,2,4]triazolo[4,3-a][1,4]-benzodiazepine;

4-(2-chlorophenyl)-9-methyl-2-(3-phenoxy-1-propynyl)-6H-thieno[3,2-f][1,2,4]triazolo[4,3-a][1,4]diazepine.

4-(2-chlorophenyl)-9-methyl-2-[3-(3-pyridyloxy-1-propynyl)-6H-thieno[3,2-f][1,2,4]triazolo[4,3-a][1,4]-diazepine;

4-(2-chlorophenyl)-9-methyl-2-[3-(2-pyrimidyloxy)-1-propynyl]-6H-thieno[3,2-f][1,2,4]triazolo[4,3-a][1,4]-diazepine;

6-(2-fluorophenyl)-1-methyl-8-[3-(8-quinolinyloxy)-1-propynyl]-4H-[1,2,4]triazolo[4,3-a][1,4]-benzodiazepine;

6-(2-chlorophenyl)-1-methyl-8-(2-thienylethynyl)-4H-triazolo[4,3-a][1,4]benzodiazepine;

6-(2-fluorophenyl)-1-methyl-8-(5-pyrimidinyl)ethynyl-4H-triazolo[4,3-a][1,4]benzodiazepine;

6-(2-fluorophenyl)-1-methyl-8-(2-pyridylethynyl)-4H-triazolo[4,3-a][1,4]benzodiazepine;

4-(2-chlorophenyl)-9-methyl-2-(2-thienylethynyl)-6H-thieno[3,2-f][1,2,4]triazolo[4,3-a][1,4]diazepine; and

4-(2-chlorophenyl)-9-methyl-2-(1-naphthylethynyl)-6H-thieno[3,2-f][1,2,4]triazolo[4,3-a][1,4][diazepine.

The compounds of formula I and their pharmaceutically acceptable acid addition salts can be prepared, in accordance with the invention, by

a) reacting a compound of the general formula

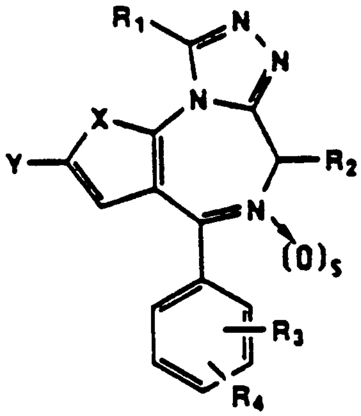

II

wherein X, $R_1$, $R_2$, $R_3$, $R_4$ and s are as defined above, and Y is bromo or iodo, with a compound of the general formula

$R_6$-$(CH_2)_n$-C≡C-H (IIIa) or $R_7$-O-$(CH_2)_n$-C≡C-H (IIIb)

wherein $R_6$, $R_7$, n and m are as defined above,

or

b) reacting a compound of the general formula

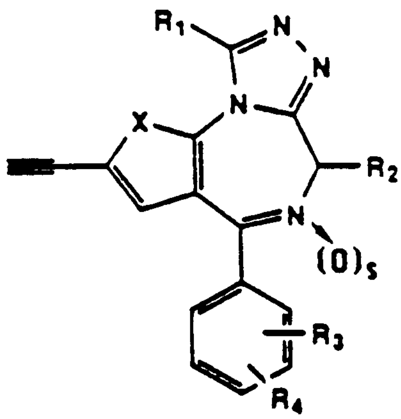

V

wherein X, $R_1$, $R_2$, $R_3$, $R_4$ and s are as defined above, with a compound of the general formula

$R_6$-Y

wherein $R_6$ and $R_7$ are as defined above and Y is as defined above,

or

c) for the manufacture of a compound of formula I, wherein s is 1 and $R_5$ is other than a moiety containing a basic nitrogen atom, reacting a compound of formula I, wherein s is 0 and $R_5$ is other than a moiety containing a basic nitrogen atom, with a peroxy acid, or

d) for the manufacture of a compound of formula I, wherein s is 0 and $R_2$ is lower alkanoyloxy, reacting a compound of formula I, wherein s is 1 and $R_2$ is hydrogen, with a lower alkanoic acid anhydride, or

e) for the manufacture of a compound of formula I, wherein $R_2$ is hydroxy, hydrolyzing a compound of formula I, wherein $R_2$ is lower alkanoyloxy, and

f) if desired, converting a compound of the general formula I obtained into a pharmaceutically accpetable acid addition salt.

More particularly, the compounds of formula I can be prepared as hereinafter described in Reaction Schemes I and II.

REACTION SCHEME I

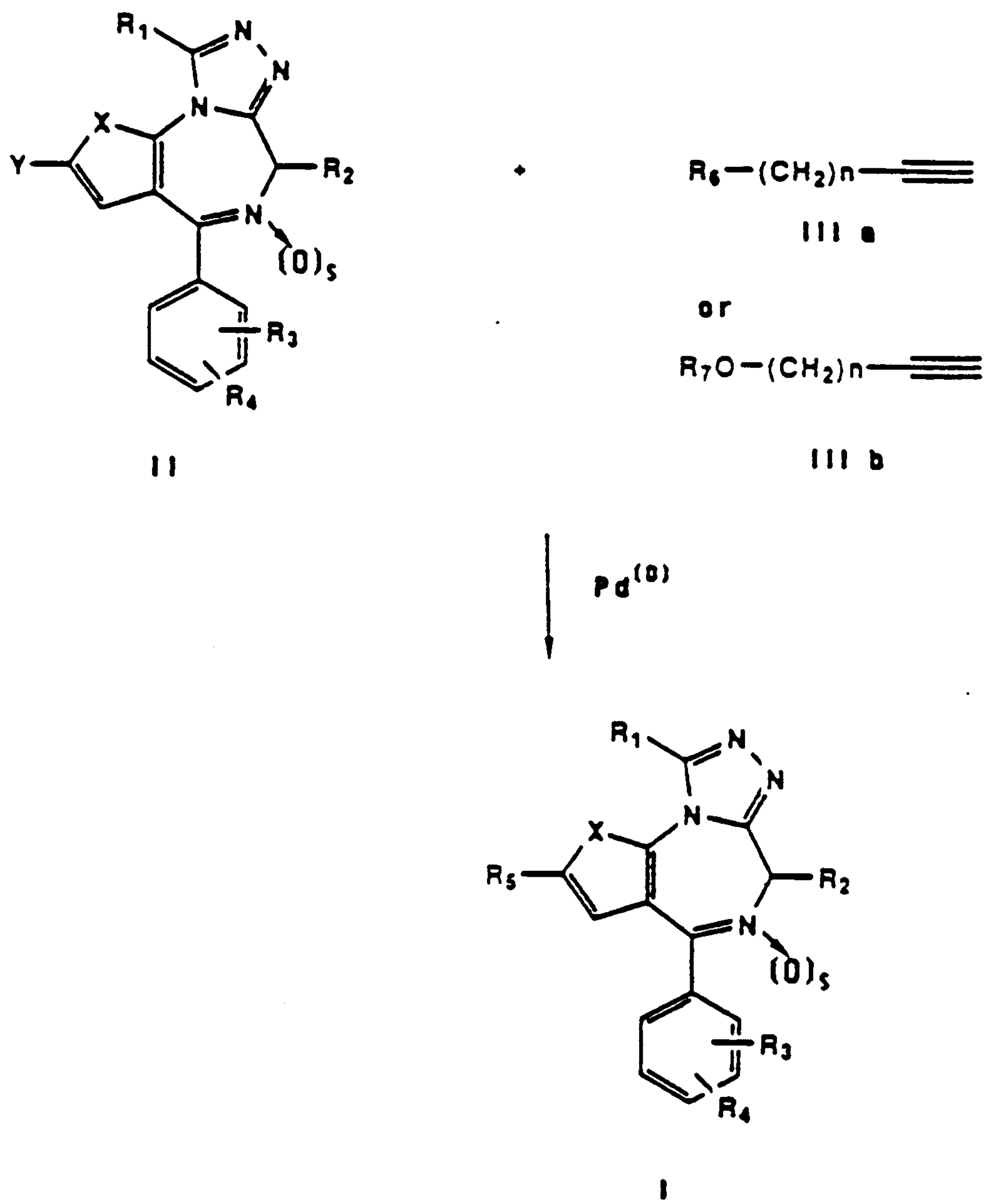


II

+

III a

or

III b

I

wherein $R_1$, $R_2$, $R_3$, $R_4$, $R_5$, $R_6$, $R_7$, X and s are as previously described, and Y is bromo or iodo.

In Reaction Scheme I, a triazolothieno- or a triazolobenzodiazepine of formula II, wherein Y is bromo or iodo, is reacted with a terminal acetylene of formula IIIa or IIIb utilizing transition metal catalysis according to procedures known in the art, to yield the corresponding compound of formula I according to process variant a).

The reaction of a bromo or preferably an iodo compound of structure II with an acetylene of formula IIIa or IIIb is carried out in an inert solvent, preferred solvents are acetonitrile, tetrahydrofuran and dimethylformamide, at a temperature in the range of from room temperature to about 100 °C, depending on the nature of Y and X in formula II, in the presence of a palladium catalyst, for example, bis(triphenylphosphine)-palladium dichloride or diacetate, optionally in the presence of a catalytical amount of cuprous iodide and an excess of a proton acceptor, such as, triethylamine.

Alternatively, a compound of formula I, wherein s is 0 and $R_5$ is other than a moiety containing a basic nitrogen atom, can be converted according to process variant c), if desired, to the corresponding N-oxide by treatment with a peroxy acid such as m-chloroperoxybenzoic acid, peroxyacetic acid and the like in an inert solvent such as methylene chloride, chloroform, acetic acid and the like, at a temperature in the range of from about 0° to 80°.

In addition, a compound of formula I, wherein $R_2$ is lower alkanoyloxy, can also be prepared according to process variant d) by treating the corresponding N-oxide according to known procedures, for example, with a lower alkanoic acid anhydride such as acetic anhydride, at a temperature in the range of from about 50° to about 100°, optionally in the presence of pyridine.

A compound of formula I, wherein $R_2$ is hydroxy, can also be prepared according to process variant e) by hydrolysis of a corresponding compound of formula I, wherein $R_2$ is lower alkanoyloxy.

The resulting product of formula I can be isolated by conventional methods for example, chromatography or crystallization.

The starting materials of formula II are known compounds or can be prepared in analogy to published procedures. This applies also to the acetylene compounds of formulas IIIa and IIIb. The acetylenes of formula IIIa, wherein n is 1, are conveniently prepared by alkylation of the corresponding heterocyclic system with propargyl bromide following known methods. The compounds of formula IIIa, wherein n is 2, can be similarly prepared by alkylation of the corresponding heterocyclic ring system, for example, with 4-tosyloxy-1-butyne.

It is noted that when a compound of formula I and/or formula II possess an asymmetric carbon, it may be convenient to utilize an enantiomer in place of the racemic mixture as the starting materials.

REACTION SCHEME II

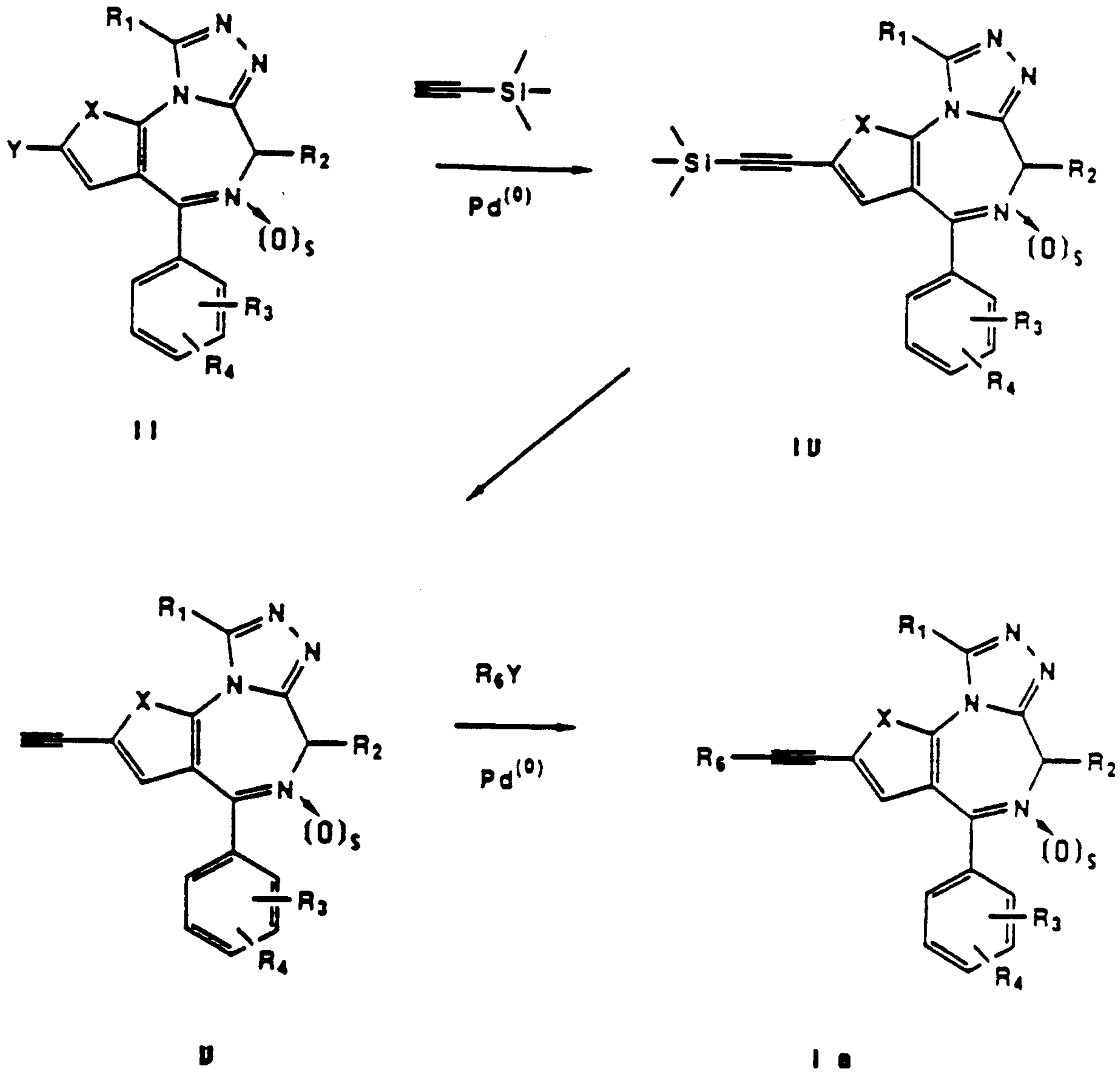


II IV V Ia

wherein $R_1$, $R_2$, $R_3$, $R_4$, $R_6$, Y and s are as previously described.

In Reaction Scheme II, a compound of formula I, wherein n is zero, can alternately be prepared as set forth. A compound of formula II, wherein Y is bromo or iodo, is coupled by palladium catalysis with trimethylsilylacetylene to yield the corresponding product of formula IV. The reaction parameters are essentially the same as described above for Reaction Scheme I. More particularly, a compound of formula IV is desilylated by treatment with an aqueous alkali solution to yield the corresponding ethynyl compound of formula V. The conversion of a compound of formula IV to a compound of formula V is carried out by hydrolysis, preferably by treatment with an aqueous alkali hydroxide solution in a water miscible solvent, such as, alcohol, tetrahydrofuran, dioxane or the like with the exclusion of oxygen. The temperature at which the reaction is carried out is not critical, but a temperature in the range of from about 0° to 100°C is preferred. A resulting compound of formula V is subjected to another palladium catalized coupling with an aryl or heteroaryl halide $R_6Y$, wherein Y is either bromo or iodo and $R_6$ is aryl or a heterocyclic radical, according to process variant b).

The resulting compound of formula Ia can be isolated by known procedures, for example, crystallization or chromatography.

The compounds of formula I can form acid addition salts with strong inorganic or organic acids. Thus, they form pharmaceutically acceptable acid addition salts with both pharmaceutically acceptable organic

and inorganic acids, for example, with hydrohalic acids, such as, hydrochloric acid, hydrobromic acid, hydroiodic acid, other mineral acids, such as, sulfuric acid, phosphoric acid, perchloric acid or the like, alkyl and mono-aryl sulfonic acids such as, ethanesulfonic acid, toluenesulfonic acid, benzenesulfonic acid, or the like. Non-pharmaceutically acceptable acid addition salts of a compound of formula I can be converted into pharmaceutically acceptable acid addition salts via conventional metathetic reactions whereby the non-pharmaceutically acceptable anion is replaced by a pharmaceutically acceptable anion; or alternatively, by neutralizing the non-pharmaceutically acceptable acid addition salt and then reacting the so-obtained free base with a reagent yielding a pharmaceutically acceptable acid addition salt.

The compounds of formula I exhibit activity as platelet activing factor (PAF) antagonists and are, therefore, useful in disease states characterized by excess platelet activating factor or for the prevention and treatment of cardiovascular diseases, pulmonary diseases, immunological disorders inflammatory diseases, dermatological disorders, shocks or transplant rejections.

The useful activity of the compounds of formula I can be demonstrated by the following procedures:

## Binding Assay

### a) Assay

The binding assay was done in 400 µl polyethylene microcentrifuge tubes (Beckman) containing 50 µl of an oil mixture of 2 parts of Siliconol AR200 (Serva) and 1 part of Silicone Fluid (Arthur H. Thoma). Buffer, standards, or analogs (150 µl total volume) were added to the tubes. Radiolabelled $^3$H-PAF (50 µl) was then added to the tubes. The reaction was started by the addition of 50 µl of dog platelets (2 x $10^7$ platelets). The tubes were capped, inverted several times to mix, and incubated for 10 minutes at room temperature. The platelets were separated from the incubation mixture by centrifuging for 1 minute in a Beckman Microfuge B centrifuge. The tip of the microfuge tube was cut off, and the platelets were washed out of the tip with 200 µl of 50% methanol (Burdick and Jackson). Aquasol (NEN, 10 ml) was added and the radioactivity in the samples was determined using an LS 8100 Beckman liquid scintillation counter linked to a Techtran tape recorder. Data was processed through an in-house computer system. Alternatively, radioactivity was determined using a Searle Mark III liquid scintillation counter linked to a Iso-Data microprocessor. Results are set forth in Tables I and II.

### b. Preparation of Platelets

Blood was collected from anesthesized or unanesthesized dogs into 50 ml plastic centrifuge tubes containing 3.8% sodium citrate as the anticoagulant (1 volume of citrate/9 volumes of blood). The red cells were removed by centrifugation for 15 minutes at 600 rpm (100-125g) at room temperature. An aliquot of the supernatant platelet rich plasma (RPR) was saved for cell counting and the remainder was acidified to pH 6.5 with 0.15 M citric acid. The platelet pellet was obtained after a 10 minute centrifugation at 2000 rpm (1000g) at room temperature. Washed platelets were prepared by resuspending the platelet pellet once with PBS containing 1 mM EDTA, centrifuging as noted, and then resuspending the platelets in 0.1% BSA-PBS. An aliquot of the washed platelets was counted. Platelets used for binding assays were diluted to 2 x $10^7$ platelets/assay tube (4 x $10^8$ platelets/ml). Platelet counting was done using a Royco Cell-Crit 921.

## PAF Induced Bronchoconstriction Assay

Male guinea pigs (Hartley Strain, 400-500g were anesthetized with urethane (2 g/kg, i.p.). Each animals' trachea was cannulated and the guinea pigs were respirated using a Harvard small animal rodent respirator (3.0 cc stroke volume, 40 breaths per min.). Tracheal pressure was recorded from a cannula inserted in the trachea and connected to a Statham Pressure transducer.

The jugular vein was cannulated for administering compounds. Spontaneous breathing was arrested with succinylcholine (1.2 mg/kg, i.v.) administered 2 minutes prior to intravenous injection of platelet activating factor (PAF). Since propranolol has been shown to enhance bronchoconstrictor responses, all animals were pretreated five minutes prior to challenge with propranolol (0.1 mg/kg, i.v.).

For the intravenous testing, the guinea pig is given a 1-minute pretreatment with propranolol at a dose of 0.1 mg/kg intravenously. The test compound is administered with a 1 minute pretreatment prior to intravenous challenge with PAF. The animal is then challenged with a 1 µg/kg intravenous dose of PAF and the change in tracheal pressure is measured.

For the oral testing, the procedure includes a 1-hour pretreatment period with the test compound administered through an oral gavage tube. Propranolol or succinylcholine and PAF are administered intravenously, and the change in tracheal pressure is measured.

The change in tracheal pressure is determined by subtracting the steady state baseline achieved after administration of succinylcholine from the peak bronchoconstriction seen after challenge with PAF. The mean is calculated for each test compound and compared to the mean of the control animals to give the percent inhibition of bronchoconstriction. The standard error is calculated as the standard error of the mean.

The results obtained are set forth in Tables 1 and 2 which follow:

TABLE I

TRIAZOLOTHIENODIAZEPINES

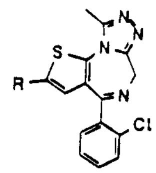

| R | PAF-binding IC-50 (nM) | Bronchoconstriction ID-50, mg/kg i.v. | p.o. 2 hr |
|---|---|---|---|
| 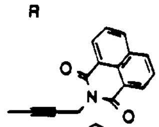 | 12 | < 0.1 | 0.025 |
| 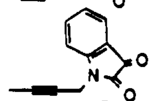 | 7 | < 0.1 | 0.060 |
| 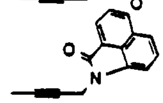 | 5 | < 0.1 | 0.042 |
| 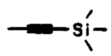 | 20 | 0.28 | |
|  | 10 | 0.02 | (17 %)* |
| 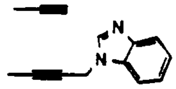 | 4.5 | | 0.12 |
| 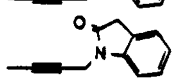 | 3.0 | | 0.05 |
| 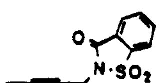 | 4 | | (27%)* |
| 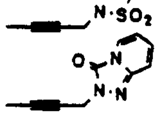 | 11 | | (79%)* |
| 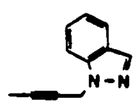 | 1.0 | 0.002 | 0.015 |
| 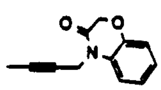 | 0.7 | 0.007 | 0.019 |
| 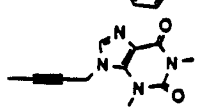 | 20 | 0.043 | (27%)* |
| 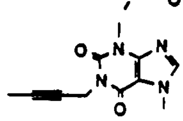 | 7.0 | 0.018 | (38%)* |
| 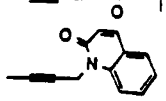 | 2.0 | 0.006 | 0.016 |
| 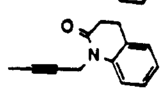 | 0.2 | 0.004 | 0.015 |

TABLE I (con't)

| R | PAF-binding IC-50 (nM) | Bronchoconstriction ID-50, mg/kg i.v. | p.o. 2 hr |
|---|---|---|---|
| 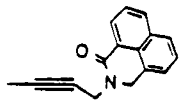 | 3.5 | 0.01 | 0.009 |
| 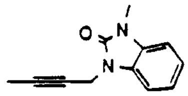 | 0.3 | 0.004 | 0.043 |
| 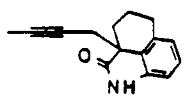 | 0.7 | 0.008 | (24 %)* |
| 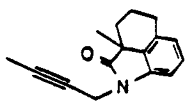 | 12 | 0.013 | 0.03 |
| 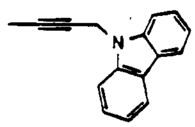 | 120 | 0.016 | 0.016 |
| 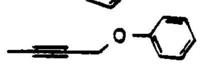 | 3.0 | 0.006 | (67 %)* |
| 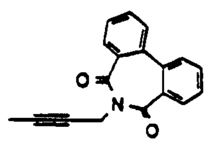 | 51 | 0.01 | 0.015 |
| 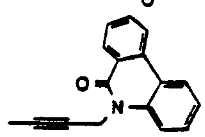 | 13 | 0.016 | 0.012 |
| 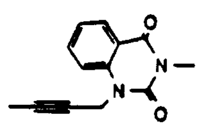 | 0.6 | 0.007 | 0.017 |
| 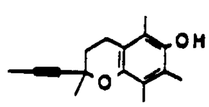 | 45 | 0.027 | |
| 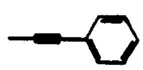 | 100 | >0.1 | |
| 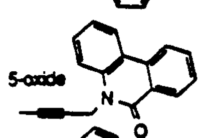 5-oxide | 15 | 0.017 | |
| 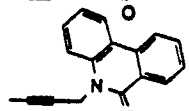 6-hydroxy | 25 | 0.022 | |

TABLE I (con't)

| R | PAF-binding IC-50 (nM) | Bronchoconstriction ID-50, mg/kg i.v. | p.o. 2 hr |
|---|---|---|---|
| 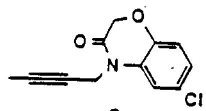 | < 1 | 0.004 | 0.049 |
| 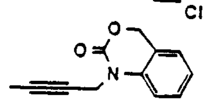 | 4.0 | 0.007 | 0.076 |
| 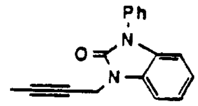 | < 1 | 0.008 | 0.015 |
| 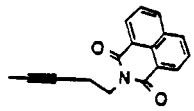 | 10 | | (66%)* |
| 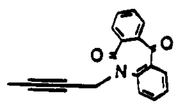 | 6 | 0.008 | 0.026 |
| 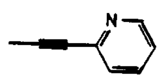 | 250 | 0.056 | (5 %)* |
| 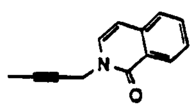 | 1.0 | 0.007 | 0.029 |
| 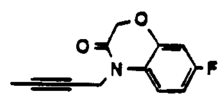 | 4 | 0.005 | 0.016 |
| 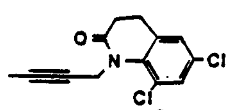 | < 1 | 0.006 | 0.021 |
| 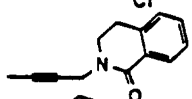 | 5 | 0.008, | 0.016 |
| 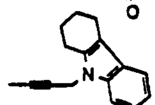 | 7 | 0.005 | 0.006 |
| 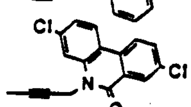 | | 0.008 | |
| 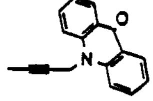 | | 0.01 | |

Numbers as percentages indicate % inhibition of bronchoconstriction at a dose of 0.3 mg/kg p.o.

TABLE II

**TRIAZOLOBENZODIAZEPINES**

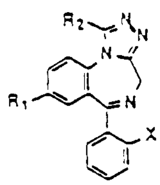

| R1 | R2 | X | PAF-binding IC-50 (nM) | Bronchoconstriction ID-50, mg/kg i.v. | p.o. 2 hr |
|---|---|---|---|---|---|
| 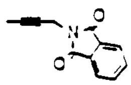 | Me | Cl | 20 | 0.019 | 1.4 |
| 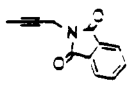 | Me | F | 10 | 0.013 | 0.52 |
| 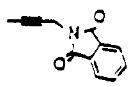 | Et | F | 100 | 0.054 | 1.5 |
| 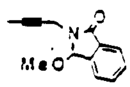 | Me | F | 100 | (99%)* | (100%)* |
| 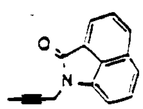 | Me | F | 20 | (99%)* | (57%)* |
| 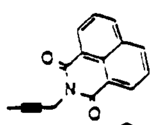 | Me | Cl | 15 | | (99%)* |
| 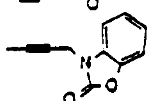 | Me | Cl | 20 | | (91%)* |
| 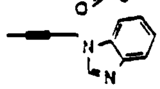 | Me | Cl | 15 | | |
| 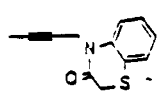 | Me | F | 7 | (99%)* | (99%)* |
| 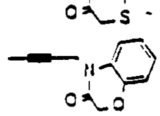 | Me | F | 5 | (100%)* | (98%)* |
| 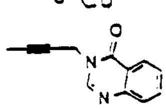 | Me | F | 70 | | |

* Number in percentages indicate % inhibition of bronchoconstriction at 0.1 mg/kg i.v. and 3 mg/kg p.o.

TABLE II (con't)

| R1 | R2 | X | PAF-binding IC-50 (nM) | Bronchoconstriction ID-50, mg/kg i.v. | p.o. 2 hr |
|---|---|---|---|---|---|
| 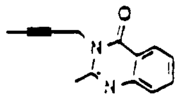 | Me | F | 50 | | |
| 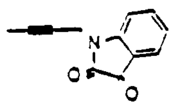 | Me | Cl | 350 | (100%)* | |
| 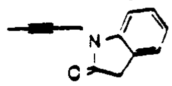 | Me | Cl | < 10 | | |
| 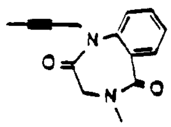 | Me | F | 160 | | |
| 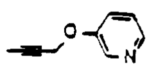 | Me | Cl | 130 | 0.005 | -3 |
| 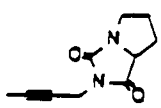 | Me | F | 200 | | (99 %)* |
| 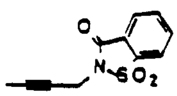 | Me | F | 150 | | |
| 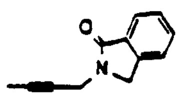 | Me | F | 225 | | (100 %)* |
| 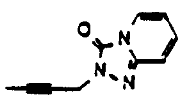 | Me | F | 350 | | (43 %)* |
| 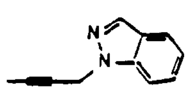 | Me | F | < 10 | | |
| 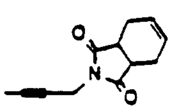 | Me | F | 50 | | (99 %)* |

TABLE II (con't)

| R1 | R2 | X | PAF-binding IC-50 (nM) | Bronchoconstriction ID-50, mg/kg i.v. | p.o. 2 hr |
|---|---|---|---|---|---|
| 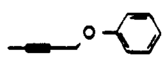 | Me | Cl | < 10 | | (37 %)** |
| 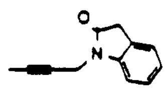 | Me | F | < 10 | | (32 %)** |
| 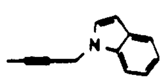 | Me | F | | | >> 0.3 |
| 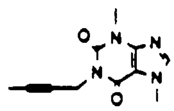 | Me | F | | | >> 0.3 |
| 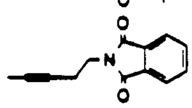 | Me | F | | | (54 %)** |
| 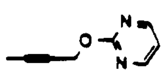 | Me | F | | | |
| 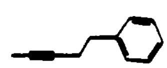 | Me | F | < 1 | 0.006 | >>0.3 |
| 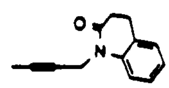 | Me | F | 20 | 0.015 | 0.44 |
| 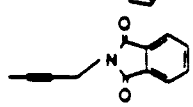 | $CF_3$ | F | 5.0 | 0.44 | 0.30 |
| 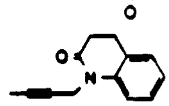 4-methyl (S) | Me | F | 6.0 | 0.008 | |
| 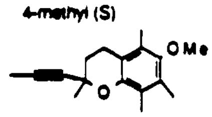 | Me | F | 70 | 0.03 | |

** numbers in percentages indicate % inhibition of bronchoconstriction at an oral dose of 0.3 mg/kg.

It is to be understood that formula I as used herein, includes racemates and enantiomers, when one or more asymmetric carbons are present in a compound of formula I.

The compounds of formula I and the pharmaceutically acceptable salts thereof can be administered by methods well known in the art. Thus, a compound of formula I, or a salt thereof can be administered either singly or together with other pharmaceutical agents, for example, antihistamines, mediator release inhibitors, methyl xanthines, beta agonists or antiasthmatic steroids such as prednisone and prednisolone, orally,

parenterally, rectally, or by inhalation, for example, in the form of an aerosol, micropulverized powder or nebulized solution. For oral administration they can be administered in the form of tablets or capsules, for example, in admixture with talc, starch, milk sugar or other inert ingredients, that is, pharmaceutically acceptable carriers, or in the form of aqueous solutions, suspensions, elixirs or aqueous alcoholic solutions, for example, in admixture with sugar or other sweetening agents, flavoring agents, colorants, thickeners and other conventional pharmaceutical excipients. For parenteral administration, they can be administered in solutions or suspension, for example, as an aqueous or peanut oil solution or suspension using excipients and carriers conventional for this mode of administration.

For administration as aerosols, they can be dissolved in a suitable pharmaceutically acceptable solvent, for example, ethyl alcohol or combinations of miscible solvents, and mixed with a pharmaceutically acceptable propellant. Such aerosol compositions are packaged for use in a pressurized container fitted with an aerosol valve suitable for release of the pressurized composition. Preferably, the aerosol valve is a metered valve, that is one which on activation releases a predetermined effective dose of the aerosol composition.

In the practice of the invention, the dose of a compound of formula I or a salt thereof to be administered and the frequency of administration will be dependent on the potency and duration of activity of the particular compound of formula I or salt to be administered and on the route of administration, as well as the severity of the condition, age of the mammal to be treated and the like. Oral doses of a compound of formula I or a salt thereof contemplated for use in practicing the invention are in the range of from about 0.5 to about 1000/mg per day, preferably about 0.5 to about 100 mg per day, preferably about 0.5 to about 10 mg either as a single dose or in divided doses.

Furthermore, since those compounds of formula I of the invention, wherein $R_2$ is other than hydrogen, possess an asymmetric centre, they are ordinarily obtained as racemic mixtures. It is to be understood that when $R_6$ and $R_7$ are a heterocyclic group, that group may also have one or more asymmetric centres, and the resulting racemates, enantiomers and diastereomers also form part of this invention. The resolution of such racemates into the optically active isomers can be carried out by known procedures. Some racemic mixtures can be precipitated as eutectics and can thereafter be separated. Chemical resolution is, however, preferred. By this method, diastereomers are formed from the racemic mixture of a compound of formula I, with an optically active resolving agent. The formed diastereomers are separated by selective crystallization or chromatography and converted to the corresponding optical isomer. Thus, the invention covers the racemates of the compounds of formula I as well as their optically active isomers (enantiomers).

The examples which follow further illustrate the invention. All temperatures are in degrees Centrigrade, unless otherwise specified.

Example 1

a) A mixture of 31 g (0.08 mol) of 1,3-dihydro-5-(2-fluorophenyl)-7-iodo-1,4-benzodiazepin-2(2H)-one [ref. G. F. Field and L. H. Sternbach, Swiss patents 561,706; May 1975; and 562,222; April 1975], 20 g (0.09 mol) of phosphorus pentasulfide, 20 g of sodium bicarbonate and 300 ml of diglyme was stirred and heated to 80-85 °C. for 3 hours. The reaction mixture was then poured onto ice and diluted with water. After stirring for 30 minutes, the solid yellow product was filtered off, washed with water, 2-propanol and little ether. It was sucked dry in the funnel and further dried in vacuum to leave 26 g (80%) of 1,3-dihydro-5-(2-fluorophenyl)-7-iodo-1,4-benzodiazepine-2(2H)-thione which was further transformed as described below. Pure material was obtained by recrystallization from tetrahydrofuran/ethanol and had m.p. 242-244 °C.

b) Hydrazine, 3 ml, was added to a suspension of 8 g of the above thione in 40 ml of 2-propanol and 100 ml of tetrahydrofuran. After stirring for 15 minutes at room temperature, the reaction mixture was filtered over 20 g of silica gel using tetrahydrofuran for elution. The filtrate was evaporated and the residue was crystallized from ether to yield 6.7 g (83 %) of 5-(2-fluorophenyl)-2-hydrazino-7-iodo-3H-1,4-benzodiazepine with m.p. 179-181 °C.

c) A mixture of 4 g of the above hydrazino compound, 20 ml of triethyl orthoacetate, 30 ml of toluene and 4 g of silica gel was heated to reflux with stirring for 3 hours. The silica gel was filtered off and washed with ethanol. The filtrate was evaporated and the residue was crystallized from methylene chloride/ethyl acetate to yield 3.9 g (92 %) of 6-(2-fluorophenyl)-8-iodo-1-methyl-4H-[1,2,4]triazolo[4,3-a]-[1,4]benzodiazepine with m.p. 235-238 °C.

d) A mixture of 1.68 g (4 mmol) of 6-(2-fluorophenyl)-8-iodo-1-methyl-4H-[1,2,4]triazolo[4,3-a][1,4]-benzodiazepine, 0.88 g (4.8 mmol) of N-propargylphthalimide, 2 ml of triethylamine, 0.36 g of triphenylphosphine, 0.08 g of cuprous iodide and 40 ml of dimethylformamide was stirred and degassed by a

slow stream of argon for 15 minutes. At this point, 0.12 g of palladium acetate was added and the mixture was stirred under argon for 16 hours at room temperature. The reaction mixture was partitioned between 200 ml of methylene chloride and 100 ml of saturated aqueous sodium bicarbonate solution. The organic phase was separated, dried over sodium sulfate and evaporated under reduced pressure, at the end azeotropically with xylene. The crude product was chromatographed over 120 g of silica gel (Merck 70-230 mesh) using 5 % (V/V) of ethanol in methylene chloride. The clean fractions of product were combined and evaporated and the residue was crystallized from ethyl acetate to yield 1.6 g (84 %) of 2-[3-[6-(2-fluorophenyl)-1-methyl-4H-[1,2,4]triazolo[4,3-a][1,4]-benzodiazepin-8-yl]-2-proynyl]-1H-isoindole-1,3(2H)-dione with m.p. 253-255 ° C.

Example 2

a) Iodine monochloride, 15 ml (21 g), was added to a solution of 23 g (0.1 mol) of (2-aminophenyl)(2-chlorophenyl)methanone [ref., E. Reeder and L. H. Sternbach, US patent 3,371,085; Feb. 1968] in 500 ml of methylene chloride cooled to -60 ° C. After stirring with cooling for 5 hours, the cooling bath was removed and the temperature of the reaction mixture was allowed to reach O ° C. Following the addition of 300 ml of aqueous sodium bisulfite solution, the two phase system was stirred for 10 minutes. The organic phase was separated, dried over sodium sulfate and evaporated. The residue was crystallized from ether/hexane to yield 20 g (56 %) of (2-amino-5-iodophenyl) (2-chlorophenyl)methanone with m.p. 120-122 ° C.

b) Bromoacetyl bromide, 15 ml, was added to a solution of 52g (0.145 mol) of (2-amino-5-iodophenyl)(2-chlorophenyl) methanone in 300 ml of methylene chloride cooled to 0 ° C. A 10% aqueous solution of sodium carbonate, 150 ml, was added slowly with stirring and the two phase system was stirred in the cold for 30 minutes. The organic layer was separated, washed with water and dried over sodium sulfate. The solution was filtered and evaporated. Crystallization of the residue from methylene chloride/ether yielded 61g (90%) of 2-bromo-N-[2-(2-chloro- benzoyl)-4-iodophenyl] acetamide with m.p.150-152 ° C. A solution of 50g of this material in 1 liter of methylene chloride was added to 800 ml of liquid ammonia with dry-ice cooling. After refluxing for 16 hours, the cooling was discontinued and the ammonia was allowed to evaporate. The remaining solution was washed with water, dried over sodium sulfate and evaporated under reduced pressure. The residue was dissolved in 1 liter of ethanol and the solution was heated to reflux for 30 minutes after the addition of 15 ml of acetic acid. The crystals separated from the cooled reaction mixture were collected to leave 38g (89%) of 5-(2-chlorophenyl)-1,3-dihydro-7-iodo-2H-1,4-benzodiazepin-2-one which melted at 260-262 ° C after recrystallization from tetrahydrofuran/ethanol.

c) A solution of 15.7 g (0.04 mol) of 5-(2-chlorophenyl)-7-iodo-1,3-dihydro-2H-1,4-benzodiazepin-2-one in 350 ml of tetrahydrofuran was cooled to -30 ° C. Potassium t-butoxide, 4.9 g (0.044 mol) was added and stirring under nitrogen was continued for 30 minutes at -10 to -5 ° C. Diethyl chlorophosphate, 6,6 ml, was then added and the mixture was stirred at this temperature for another 30 minutes. Following the addition of 3.4 g of acetyl hydrazine, stirring without cooling was continued for 1 hour and 150 ml of n-butanol was added. The tetrahydrofuran was distilled out of the reaction mixture over a period of 45 minutes. The residue was partitioned between water and toluene. The organic phase was washed with brine, dried over sodium sulfate and evaporated down to a small volume. The precipitated crystals were collected to leave 14 g of crude product which was purified by chromatography over 250 g of silica gel using 5% ethanol (V/V) in methylene chloride. The clean fractions were combined and evaporated. Crystallization from tetahydrofuran/ethanol gave 8.5g (49%) of 6-(2-chlorophenyl)-8-iodo-1-methyl-4H-[1,2,4] triazolo[4,3-a]-[1,4]benzodiazepine with m.p. 290-292 ° C.

d) Reaction of 6-(2-Chlorophenyl)-8-iodo-1-methyl-4H-[1,2,4]triazolo[4,3-a][1,4]benzodiazepine with N-propargylphthalimide as described in example 1d gave the desired 2-[3-[6-(2-chlorophenyl)-1-methyl-4H-[1,2,4]triazolo[4,3-a] [1,4]benzodiazepin-8-yl]-2-propynyl]-1H-isoindole-1,3(2H)-dione semihydrate, which was purified by chromatography and crystallized from methanol/ethyl acetate to give off-white crystals with m.p. 248-250 ° C.

Example 3

a) A mixture of 1 g of 5-(2-fluorophenyl)-7-iodo-2-hydrazino-7-iodo-3H-1,4-benzodiazepine (see example 1b), 5 ml of triethyl orthopropionate and 10 ml of xylene was heated to reflux for 1 hour. The solvents were partially distilled over and the residue was diluted with hexane. The precipitated crystals were collected and recrystallized from methanol/ethyl acetate to leave 1.05 g (97 %) of 1-ethyl-6-(2-fluorophenyl)-8-iodo-4H-[1,2,4]triazolo[4,3-a][1,4]benzodiazepine with m.p. 209-211 ° C.

b) A mixture of 435 mg (1 mmol) of 1-ethyl-6-(2-fluorophenyl)-8-iodo-4H-[1,2,4]triazolo[4,3-a][1,4]-benzodiazepine, 220 mg of N-propargylphthalimide, 80 mg of triphenylphosphine, 20 mg of cuprous iodide, 0.5 ml of triethylamine and 10 ml of dimethylformamide was stirred and degassed with a stream of argon for 10 minutes. Palladium acetate, 30 mg, was then added and stirring under argon was continued for 48 hours. The reaction mixture was partitioned between methylene chloride and saturated aqueous sodium bicarbonate solution. the organic phase was dried and evaporated under reduced pressure, at the end azeotropically with xylene. The residue was chromatographed over 30g silica gel (Merck 70-230 mesh) using 5 % (V/V) of ethanol in methylene chloride. Crystallization of the combined clean fractions from ethyl acetate yielded 0.41g of 2-[3-[1-ethyl-6-(2-fluorophenyl)-4H-[1,2,4]triazolo[4,3-a] [1,4]benzodiazepin-8-yl]-2-propynyl]-1H-isoindole-1,3(2H)-dione semihydrate with m.p. 216-219 °C.

<u>Example 4</u>

6-(2-Chlorophenyl)-8-iodo-1-methyl-4H-[1,2,4]triazolo[4,3-a][1,4] benzodiazepine was reacted with 1-(2-propynyl)-1H-indole-2,3-dione [ref. A. Lindquist, P. Lagerstrom and R. Dahlbom, Acta Pharm. Suecica <u>9</u>, 99 (1972)] as described in Example 3b. The crude product was purified by chromatography over 40 fold amount of silica gel using 5% (V/V) of ethanol in methylene chloride. Crystallization from ethyl acetate gave yellow crystals of 1-[3-[6-(2-chlorophenyl)-1-methyl-4H-[1,2,4]triazolo[4,3,-a][1,4]benzodiazepin-8-yl]-2-propynyl]-1H-indole-2,3-dione with m.p. 210-212 °C. These crystals contained according to microanalysis and pmr-spectrum 0.25 molar amounts of ethyl acetate.

<u>Example 5</u>

6-(2-Chlorophenyl)-8-iodo-1-methyl-4H-[1,2,4]triazolo[4,3-a] [1,4]benzodiazepine was reacted with 2-(2-propynyl)-1H-benz[de]isoquinoline-1,3(2H)-dione as described in example 3b. The crude product was chromatographed over 40 fold amount of silica gel using 4% (V/V) of ethanol in methylene chloride for elution. Crystallization from methylene chloride/ether and recrystallization from tetrahydrofuran/ethanol gave 2-[3-[6-(2-chlorophenyl)-1-methyl-4H-[1,2,4]triazolo[4,3-a] [1,4]benzodiazepin-8-yl-2-proynyl]-1H-benz[de]-isoquinoline-1,3(2H)-dione as colorless crystals with m.p. 213-215 °C., containing 0.66 molar amounts of water according to pmr-spectrum and analysis.

The acetylenic reaction component was prepared as follows:

Potassium t-butoxide, 6.2 g (0.055 mol) was added to a solution of 9.9 g (0.05 mol) of naphthalimide in 50 ml of dimethylformamide cooled to -20 °C. After stirring in the cold for 1 hour, 5 ml (0.055 mol) of propargyl bromide in 20 ml of dimethylformamide was added and the mixture was allowed to warm to room temperature. It was then heated to 45 °C. for 45 minutes. After cooling, 15 ml of glacial acetic acid was added and the product was precipitated by addition of water. The solids were collected and recrystallized from ethyl acetate to leave 10 g (84 %) of colorless crystals of 2-(2-propynyl)-1H-benz[de] isoquinoline-1,3-(2H)-dione with m.p. 235-237 °C.

<u>Example 6</u>

6-(2-Chlorophenyl)-8-iodo-1-methyl-4H-[1,2,4]triazolo[4,3-a][1,4]benzodiazepine was reacted with 1-(2-propynyl)-1H-benzimidazole [ref. I. I. Popov. P. V. Tkachenko and A. M. Simonov, Khim. Geterots. Soedin. 551, (1973)] as described in example 3b. The product was isolated by chromatography over 40 fold amount of silica gel using 5 %(V/V) of ethanol in methylene chloride. Crystallization from ethanol yielded 8-[3-(1H-benzimidazol-1-yl)-1-propynyl]-6-(2-chlorophenyl)-1-methyl-4H-[1,2,4]triazolo[4,3-a][1,4]benzodiazepine semihydrate as colorless crystals with m.p. 165-168 °C. Analytical and spectroscopic data indicated a semihydrate.

<u>Example 7</u>

Reaction of 6-(2-chlorophenyl)-8-iodo-1-methyl-4H-[1,2,4]triazolo[4,3-a][1,4]benzodiazepine was reacted with 3-(2-propynyl)- 2,3-dihydro-1,3-benzoxazol-2-one [ref. A. Lindquist et al., Acta Pharm. Suecica <u>9</u>, 99 (1972)] yielded after chromatography over 40 fold amount of silica gel with 3% (V/V) of ethanol in methylene chloride and crystallization from ethanol colorless crystals of 3-[3-[6-(2-chlorophenyl)-1-methyl-4H-[1,2,4]triazolo[4,3-a][1,4]benzodiazepin-8-yl]-2-propynyl]-2,3-dihydro-1,3-benzoxazol-2-one hydrate with m.p. 158-160 °C.

Example 8

6-(2-Chlorophenyl)-8-iodo-1-methyl-4H-[1,2,4]triazolo[4,3-a][1,4]benzodiazepine was reacted with 1,3-dihydro-1-(2-propynyl)-2H-indol-2-one [ref. A. Lindquist et al., Acta Pharm. Suecica 9, 99 (1972)] as described in example 3b. The product was isolated and purified by chromatography over the 40 fold amount of silica gel using 5% (V/V) of ethanol in methylene chloride. Crystallization from ethanol gave 1-[3-[6-(chlorophenyl)-1-methyl-4H-[1,2,4]triazolo[4,3-a][1,4]benzodiazepin-8-yl]-2-propynyl]-1,3-dihydro-2H-indol-2-one with m.p. 141-143 °C and containing 0.33 mol of ethanol and 0.66 mol of water.

Example 9

A mixture of 0.84 g (2 mmol) of 6-(2-fluorophenyl)-8-iodo-1-methyl-4H-[1,2,4]triazolo[4,3-a][1,4]-benzodiazepine, 0.5 g (2.4 mmol) of 1-(2- propynyl)benz[cd]indol-2(1H)-one, 90 mg of triphenylphosphine, 20 mg of cuprous iodide, 1 ml of triethylamine and 20 ml of dimethylformamide was degassed with a slow stream of argon for 15 minutes. Palladium acetate, 30 mg, was then added and the mixture was stirred under argon for five hours at room temperature. The reaction mixture was partitioned between methylene chloride and saturated aqueous sodium bicarbonate solution. The organic phase was dried and evaporated and the residue was chromatographed over 40 g of silica gel using 5 % (V/V) of ethanol in methylene chloride for elution. Crystallization of the clean fractions from methanol/ethyl acetate yielded 1-[3-[6-(2-fluorophenyl)-1-methyl-4H-[1,2,4]triazolo[4,3-a][1,4]benzodiazepin-8-yl]-2-propynyl]benz[cd]indole-2(IH)-one as light yellow crystals with m.p. 224-226 °C.

The acetylenic starting material was prepared as follows:

Potassium-t-butoxide, 6,17 g (0.055 mol) was added to a solution of 8.46 g (5 mmol) of benz[cd]indol-2-(1H)-one in 100 ml of dimethylformamide. After stirring for 10 minutes at room temperature, 4.9 ml (0.055 mol) of propargyl bromide was added and the mixture was stirred for 1 hour at room temperature. The reaction mixture was acidified with acetic acid and partitioned between methylene chloride and saturated aqueous sodium bicarbonate solution. The organic layer was dried and evaporated and the residue was crystallized from tetrahydrofuran/ethanol to leave 8g (77%) of 1-(2-propynyl)benz[cd]indol-2(1H)-one with m.p. 183-186 °C. The compound was recrystallized for analysis twice from methylene chloride/ ethyl acetate and had m.p. 185-187 °C.

Example 10

Reaction of 0.84 g of 6-(2-fluorophenyl)-8-iodo-1-methyl-4H-[1,2,4]triaolo [4,3-a][1,4]benzodiazepine with 0.53 g (2.6 mmol) of 4-(2-propynyl)-2H-1,4-benzothiazin-3(4H)-one [ref. R. N. Prasad and K. Tietje, Can. J. Chem. 44, 1247 (1966)] as described in Example 9 yielded after chromatographic purification (5 % ethanol in methylene chloride on silica gel) and crystallization from ethyl acetate 0.5 g (51%) of yellowish crystals of 4-[3-[6-(2-fluorophenyl)-1-methyl-4H-[1,2,4]triazolo[4,3-a] [1,4]benzodiazepin-8-yl]-2-propynyl]-2H-1,4-benzothiazin-3(4H)-one with m.p. 203-206 °C. These crystals contained according to pmr-spectrum and analysis 0.166 molar amounts of ethyl acetate.

Example 11

4-[3-[6-(2-Fluorophenyl)-1-methyl-4H-[1,2,4]triazolo[4,3-a][1,4]benzodiazepin-8-yl]-2-propynyl]-2H-1,4-benzoxazin-3(4H)-one was similarly obtained by coupling 0.84 g of 6-(2-fluorophenyl)-8-iodo-1-methyl-4H-[1,2,4]triazolo [4,3-a][1,4]benzodiazepine with 4-(2-propynyl)-2H-1,4-benzoxazin-3(4H)-one [ref. A. Lindquist et al., Acta Pharm. Suecica 9, 99 (1972)] as described in Example 9. The product was isolated and purified by chromatography and was crystallized from ethyl acetate to give 0.55 g (56%) of light yellow crystals with m.p. 238-240 °C. These crystals contained 0.166 mol of ethyl acetate according to spectral and analytical data.

Example 12

Reaction of 0.84 g (2 mmol) fo 6-(2-fluorophenyl)-8-iodo-1-methyl-4H-[1,2,4]triazolo[4,3-a][1,4]-benzodiazepine with 0.48 g (2.6 mmol) of 3-(2-propynyl)-4(3H)-quinazolinone [ref. J. Maillard et al., Chimie There. 3, 202 (1967)] as described in example 9 yielded 0.6 g (59 %) of off-white product, crystallized from ethyl acetate. The crystals of 3-[3-[6-(2-fluorophenyl)-1-methyl-4H-[1,2,4]triazolo[4,3-a] [1,4]benzodiazepin-8-yl]-2-propynyl]-4(3H)-quinazolinone with m.p. 199-201 °C contained 1 mol of water and 0.166 molar

amounts of ethyl acetate.

Example 13

3-[3-[6-(2-Fluorophenyl)-1-methyl-4H-[1,2,4]triazolo[4,3-a][1,4]benzodiazepin-8-yl]-2-propynyl]-2-methyl-4(3H)-quinazolinone was obtained by coupling 6-(2-fluorophenyl)-8-iodo-1-methyl-4H-[1,2,4]triazolo[4,3-a]-[1,4]benzodiazepine with 2-methyl-3-(2-propynyl)-4(3H)-quinazolinone [ref. B. Danielsson, L. Kronberg and B. Akerman, Acta Pharm. Suecica, 6, 379, (1969)] as described in ex. 9. It was isolated in 57 % yield and crystallized from ethyl acetate, m.p. 241-244 °C with decomposition. The crystals contained 0.66 molar amounts of water.

Example 14

Reaction of 6-(2-fluorophenyl)-8-iodo-1-methyl-4H-[1,2,4]triazolo[4,3-a][1,4]benzodiazepine with 2,3-dihydro-2-(2-propynyl)-1H-isoindol-1-one [ref. J. I. Neumeyer, U. V. Moyer, J. A. Richman, F. J. Rosenberg and D. G. Teiger, J. Med. Chem. 10, 615 (1967)] gave after chromatographic purification as described in example 9 and crystallization from ethyl acetate colorless crystals of 2-[3-[6-(2-fluorophenyl)-1-methyl-4H-[1,2,4]triazolo[4,3-a][1,4]benzodiazepin-8-yl]-2-propynyl]-2,3-dihydro-1H-isoindol-1-one with m.p. 165-168 °C. According to spectral and analytical data, these crystals contained 0.5 molar amounts of water and traces of ethyl acetate.

Example 15

rac-2,3-Dihydro-2-[3-[6-(2-fluorophenyl)-1-methyl-4H-[1,2,4]triazolo[4,3-a][1,4]benzodiazepin-8-yl]-2-propynyl] -3-methoxy-1H-isoindol-1-one was prepared as described in example 1d by reacting 6-(2-fluorophenyl)-8-iodo-1-methyl-4H-[1,2,4] triazolo[4,3-a][1,4]benzodiazepine with rac. 2,3-dihydro-3-methoxy-2-(2-propynyl)-1H-isoindol-1-one. The product was not obtained in crystalline state and was characterized spectroscopically. For testing the compound was precipitated from tetrahydrofuran by addition of hexane and the resulting amorphous powder was dried under vacuum. Nmr ($CDCl_3$): 2.64 (s, 3, $CH_3$), 2.96 (s, 3, OMe), 4.1 (d,1) and 5.54 (d,1) (AB-system J = 7 Hz, $CH_2$), 4,2 (d,1) and 4.88 (d,1) (AB-system, J = 9 Hz, $CH_2$ of propynyl), 6.07 (s,1,C3-H), 6,9-8.0 (m, 11, aromatic H) ppm.

The acetylenic reaction component was prepared as follows:

A solution of 2 g of 2,3-dihydro-3-hydroxy-2-(2-propynyl)-1H-isoindol-1-one in 20 ml of thionyl chloride was allowed to sit at room temperature over night. The reagent was evaporated azeotropically with toluene under reduced pressure. The residue was dissolved in 20 ml of methanol and the solution was treated with 5 ml of triethylamine. After heating on the steam bath for 5 minutes, the mixture was evaporated and the residue was partitioned between methylene chloride and saturated aqueous sodium bicarbonate solution. The organic layer was separated, dried and evaporated. The residue was crystallized from ether/hexane to leave 0.8 g of rac. 2,3-dihydro-3-methoxy-2-(2-propynyl)-1H-isoindol-1-one as colorless crystals with m.p. 85-87 °C.

The starting material was prepared as follows:

A mixture of 10 g of N-propargylphthalimide and 2 g of sodium borohydride in 100 ml of ethanol was heated on the steam bath for 15 minutes with stirring. The resulting solution was concentrated under reduced pressure to one third of the volume and the product was crystallized by addition of ice and saturated sodium bicarbonate solution. The precipitated crystals of rac. 2,3-dihydro-3-hydroxy-2-(2-propynyl)-1H-isoindol-1-one were collected by filtration, washed with water and sucked dry. After drying in vacuum they had m.p. 157-159 °C.

Example 16

2-[3-[6-(2-Fluorophenyl)-1-methyl-4H-[1,2,4]triazolo[4,3-a][1,4]benzodiazepin-8-yl]-2-propynyl]-1,2,4-triazolo[4,3-a] pyridin-3(2H)-one was obtained as described in example 9 by coupling of 6-(2-fluorophenyl)-8-iodo--1-methyl-4H-[1,2,4] triazolo[4,3-a][1,4]benzodiazepine with 2-(2-propynyl)- 1,2,4-triazolo[4,3-a]-pyridin-3(2H)-one. The product was purified by chromatography in the usual fashion and crystallized from ethyl acetate/ethanol. Recrystallization from ethanol gave light yellow crystals with m.p. 170-173 °C. They contained 0.66 molar amounts of water.

The required acetylene was prepared as follows:

Potassium-t-butoxide, 3 g (2.6 mmol), was added to a solution of 3.25 g (2.4 mmol) of 1,2,4-triazolo

[4,3-a]pyridin-3(2H)-one in 75 ml of dimethylformamide. After stirring under nitrogen for 15 minutes, 2.35 ml (2.6 mmol) of propargyl bromide was added and stirring at room temperature was continued for 1 hour. The solvent was evaporated under reduced pressure, at the end azeotropically with xylene. The residue was extracted with methylene chloride and the solution was evaporated. Chromatographic purification of the residue on silica gel (5 % ethanol in methylene chloride) and crystallization of the clean fractions from methanol yield 1.7 g of colorless crystals of 2-(2-propynyl)-1,2,4-triazolo[4,3-a]pyridin-3(2H)-one with m.p. 126-128 ° C.

Example 17

6-(2-Fluorophenyl)-1-methyl-8-[3-(1H-indazol-1-yl)--1-propynyl]-4H-[1,2,4]triazolo[4,3-a][1,4]-benzodiazepine was obtained as described in example 9 by reaction of 6-(2-fluorophenyl)-8-iodo-1-methyl-4H-[1,2,4] triazolo[4,3,-a][1,4]benzodiazepine with 1-(2-propynyl)-1H-indazole[ref. P. V. Tkachenko, I. I. Popov, A. M. Simonov and Yu. V. Medvedov, Khim. Geterotsikl. Soedin. 11, 1542 (1975)]. The chromatographically isolated product was crystallized from ethyl acetate to give yellowish crystals with m.p. 148-151 ° C.

Example 18

Coupling of 6-(2-fluorophenyl)-8-iodo-1-methyl-4H-[1,2,4]triazolo[4,3-a][1,4]benzodiazepine with 1,3-dihydro-1-(2-propynyl)-2H-indol-2-one [ref. A. Lindquist et al., Acta Pharm. Suecica, 9, 99 (1972)] as described in Example 9 gave colorless crystals of 1-[3-[6-(2-fluorophenyl)-1-methyl-4H-[1,2,4]triazolo[4,3-a]-[1,4]benzodiazepin-8-yl]-2-propynyl]-1,3-dihydro-2H-indol-2-one hydrate from ethyl acetate, which had m.p. 233-235 ° C.

Example 19

2-[3-[6-(2-Fluorophenyl)-1-methyl-4H-[1,2,4]triazolo[4,3-a][1,4]benzodiazepin-8-yl]-2-propynyl]-1,2-benzisothiazol-3(2H)-one 1,1-dioxide semihydrate was prepared according to the procedure of example 9 by reacting 6-(2-fluorophenyl)-8-iodo-1-methyl4H-[1,2,4]triazolo[4,3-a][1,4]benzodiazepine with 2-(2-propynyl)-1,2-benzisothiazol-3(2H)-one 1,1-dioxide [ref. R. Granger and J. Giroux, French patent 1,273,867, Feb. 1962; C. A. 57, 7285i (1963)]. The product was isolated and purified by chromatography and crystallized from methylene chloride/ ethyl acetate to yield colorless crystals with m.p. 238-240 ° C.

Example 20

Coupling of 6-(2-fluorophenyl)-8-iodo-1-methyl-4H-[1,2,4]triazolo[4,3-a][1,4]benzodiazepine with 2-(2-propynyl)-tetrahydro-1H-pyrrolo[1,2-c] imidazole-1,3(2H)-dione under the conditions described in Example 9 yielded after chromatographic purification and crystallization from ethanol off-white crystals of 2-[3-[6-(2-fluorophenyl)-1-methyl-4H-[1,2,4]triazolo[4,3-a] [1,4]benzodiazepin-8-yl]-2-propynyl]-tetrahydro-1H-pyrrolo-[1,2-c]imidazole-1,3(2H)-dione semihydrate with m.p. 158-161 ° C.

The acetylenic reaction component was prepared as follows:

Potassium-t-butoxide, 1,23 g (11 mmol), was added to a solution of 1.4 g (10 mmol) of tetrahydro-1H-pyrrolo [1,2-c]imidazole-1,3(2H)-dione (L-proline-hydantoin) [ref. T. Suzuki, K. Igarashi, K. Hase and K. Tuzimura, Agr. Biol. Chem., 37, 411 (1973)] in 20 ml of dimethylformamide. After stirring for 10 minutes at room temperature, 1 ml (11 mmol) of propargyl bromide was added and stirring under nitrogen was continued for 2 hours. The reaction mixture was acidified with acetic acid and evaporated under reduced pressure. The residue was slurried with methylene chloride and filtered. The filtrate was evaporated and the residue was chromatographed over 45 g of silica gel using 5 % (V/V) of ethanol in methylene chloride. The clean fractions were combined and evaporated to leave 2-(2-propynyl)-tetrahydro-1H-pyrrolo[1,2-c]-imidazole-1,3(2H)-dione as a colorless, viscous oil. NMR($CDCl_3$): 1.72 (m,1,C6-H), 1,9-2,4 (m,3,C6-H,C7-H), 2.22 (t,1,J = 1.5Hz, acetylenic H), 3.24 (m,1,C5-H), 3.70 (m,1,C5-H), 4.11 (dd, 1, J = 4Hz and 3.5 Hz, C7a-H), 4.23 (d,2,J = 1.5Hz, $CH_2$) ppm.

Example 21

2-[3-[6-(2-Fluorophenyl)-1-methyl-4H-[1,2,4]triazolo[4,3-a][1,4]benzodiazepin-8-yl]-2-propynyl]-3a,4,7,7a-tetrahydro-1H-isoindole-1,3(2H)-dione was obtained by reaction of 6-(2-fluorophenyl)-8-iodo-1-methyl-4H-

[1,2,4]triazolo[4,3-a][1,4]benzodiazepine with N-propargyl-tetrahydrophthalimide [ref. W. E. Hahn and A. Sokolowska, Soc. Sci. Lodz. Acta Chim. 18, 187 (1974)] following the procedure described in example 1. The product was isolated chromatographically and was crystallized from ethanol to give colorless crystals with m.p. 259-261 ° C. According to analytical data, these crystals contained 0.33 molar amounts of water.

Example 22

Coupling of 6-(2-fluorophenyl)-8-iodo-1-methyl-4H-[1,2,4]triazolo[4,3-a] [1,4]benzodiazepine with 3,4-dihydro-4-methyl-1-(2-propynyl)-1H-1,4-benzodiazepine-2,5-dione as described in example 9 gave after chromatography and crystallization from ethyl acetate colorless crystals of 1-[3-[6-(2-fluorophenyl)-1-methyl-4H-[1,2,4]triazolo[4,3-a][1,4]benzodiazepin-8-yl]-2-propynyl]-3,4-dihydro-4-methyl-1H-1,4-benzodiazepine-2,5-dione with m.p. 179-182 ° C. The crystals contained according to analytical and NMR-data 0.16 mol of ethyl acetate and 0.66 mol of water.

The required acetylene was prepared as follows:

Propargyl bromide, 2,6 g (22 mmol), was added to a mixture of 3.8 g (20 mmol) of 3,4-dihydro-4-methyl-1H-1,4-benzodiazepine-2,5(2H)-dione [ref. M. Uskokovic and W. Wenner, US patent 3,261,828, July 1966], 3.4 g of barium oxide and 100 ml of dimethylformamide. After stirring at room temperature for 2 hours, the reaction mixture was partitioned between water and methylene chloride. The organic phase was separated, washed with water, dried and evaporated. The residue was dissolved in ethyl acetate and the product was crystallized by addition of hexane to give 3,4-dihydro-4-methyl-1-(2-propynyl-1H-1,4-benzodiazepine-2,5(2H)-dione as colorless crystals with m.p. 148-150 ° C.

Example 23

Coupling of 6-(2-chlorophenyl)-8-iodo-1-methyl-4H-[1,2,4]triazolo[4,3-a] [1,4]benzodiazepine with 1-(3-pyridinyloxy)-2-propyne [ref. J. Bruhn, J. Zsindely, H. Schmid and G. Frater, Helv. Chim. Acta 61, 2542 (1978)] as described in example 1 gave after chromatography and crystallization from ethanol/ether colorless crystals of 6-(2-chlorophenyl)-1-methyl-8-[3-(3-pyridinyloxy)-1-propynyl]-4H-[1,2,4]triazolo[4,3-a]-[1,4]benzodiazepine with m.p. 128-130 ° C. These crystals contained 0.66 molar amounts of water according to analytical data.

Example 24

6-(2-Chlorophenyl)-1-methyl-8-(3-phenoxy-1-propynyl)-4H-[1,2,4]triazolo [4,3-a][1,4]benzodiazepine was obtained by coupling 6-(2-chloro-phenyl)-8-iodo-1-methyl-4H-[1,2,4]triazolo[4,3-a][1,4]benzodiazepine with 1-phenoxy-2-propyne as described in example 1. Chromatographic isolation and crystallization from ethyl acetate gave colorless crystals with m.p. 160-162 ° C.

Example 25

a) A solution of 54.8 g of 5-(2-chlorophenyl)-1,3-dihydro-2H-thieno[2,3-e][1,4]diazepin-2-one [NL patent 7,205,730, Nov. 1972, Hoffman-La Roche & Co., AG, Basle] in 350 ml of acetic acid and 350 ml of methanol was treated with 64.4g of iodine monochloride and 16.2g of sodium acetate. The mixture was stirred for 15 minutes at room temperature. A0solution of 65 g of sodium bisulfite in 350 ml of water was then added and stirring was continued for 10 minutes. The mixture was neutralized by addition of 500 ml of conc. ammonia and 1 kg of ice. The precipitated product was filtered off and washed with water and ethanol. Recrystallization from tetrahydrofuran/ethanol gave off-white crystals of 5-(2-chlorophenyl)-1,3-dihydro-7-iodo-2H-thieno[2,3-e][1,4]diazepin-2-one with m.p. 229-231 °.

b) A mixture of 70 g of 5-(2-chlorophenyl)-1,3-dihydro-7-iodo-2H-thieno[2,3-e][1,4]diazepin-2-one, 43,3 g of phosphorus pentasulfide, 45 g of sodium bicaronate and 700 ml of diglyme was stirred and heated to 70-80 ° for 2 hours. After cooling to room temperature, a mixture of water and crushed ice was added and stirring was continued for 15 minutes. The precipitated 5-(2-chlorophenyl)-1,3-dihydro-7-iodo-2H-thieno[2,3-e][1,4]diazepine-2-thione was collected by filtration, washed with water and sucked dry.

c) A mixture of 64.4g of 5-(2-chlorophenyl)-1,3-dihydro-7-iodo-2H-thieno[2,3-e][1,4]diazepin-2-thione, 650 ml of tetrahydrofuran and 65 ml of hydrazine was stirred at room temperature for 30 minutes. The solvent was evaporated under reduced pressure and the residue was stirred with 275 ml of methylene chloride and 275 ml of water for 15 minutes. The precipitated crystalline material was filtered off and washed with water and ether. This crude 5-(2-chlorophenyl)-2-hydrazino-7-iodo-2H-thieno[2,3-e][1,4]-

diazepine was combined with 375 ml of ethyl acetate, 170 ml of triethyl orthoacetate and a few crystals of para-toluenesulfonic acid and the mixture was heated on the steam bath for 30 minutes. The product crystallized during this process and was collected after cooling. Recrystallization from methylene chloride/ethanol gave colorless crystals of 4-(2-chlorophenyl)-2-iodo-9-methyl-6H-thieno[3,2-f][1,2,4]-triazolo[4,3-a][1,4]diazepine with m.p. 254-256°.

d) A mixture of 0.88 g (2 mmol) of 4-(2-chlorophenyl)-2-iodo-9-methyl-6H-thieno[3,2-f][1,2,4]triazolo[4,3-a][1,4] diazepine, 565 mg (2.4 mmol) of 2-(2-propynyl)-1H-benz[de]isoquinolin-1,3(2H)-dione, 1 ml of triethylamine, 20 mg of cuprous iodide, 90 mg of triphenylphosphine and 20 ml of dimethylformamide was degassed by a slow stream of argon for 15 minutes. Palladium acetate, 30 mg, was then added and the mixture was stirred at room temperature under argon for 20 hours. Thin layer chromatography indicated practically complete reaction after 5 hours. The reaction mixture was partitioned between methylene chloride and saturated aqueous sodium bicarbonate solution. The organic layer was separated, dried over sodium sulfate and evaporated under reduced pressure, at the end azeotropically with xylene to remove the residual dimethylformamide. The residue was chromatographed over 40 g silica gel using 5 %(V/V) of ethanol in methylene chloride for elution. The clean fractions were combined and evaporated. Crystallization from methanol/ethyl acetate gave 0.58 g (53%) of 2-[3-[4-(2-chlorophenyl)-9-methyl-6H-thieno[3,2-f][1,2,4]triazolo[4,3-a][1,4]diazepin-2-yl]-2-propynyl]-1H-benz[de]isoquinoline-1,3-(2H)-dione with m.p. 188-192°C. A different crystalline modification with m.p. 252-254°C was also observed.

Example 26

1-[3-[4-(2-Chlorophenyl)-9-methyl-6H-thieno[3,2-f][1,2,4]triazolo[4,3-a][1,4]diazepin-2-yl]-2-propynyl]-1H-indole-2,3-dione was prepared as described in Example 25d by coupling of 4-(2-chlorophenyl)-2-iodo-9-methyl-6H-thieno[3,2-f][1,2,4]triazolo[4,3-a] [1,4]diazepine with 1-(2-propynyl)-1H-indole-2,3-dione [ref. A. Lindquist et al., Acta Pharm. Suecica 9, 99 (1972)]. Chromatographic isolation and crystallization from methanol/ethyl acetate gave orange crystals with m.p. 185-190°C with foaming at 130-140°C. These crystals contained according to analytical and spectral data molar amounts of ethyl acetate.

Example 27

Reaction of 4-(2-chlorophenyl)-2-iodo-9-methyl-6H-thieno[3,2-f][1,2,4]triazolo[4,3-a][1,4]diazepine with 1-(2-propynyl)-benz[cd]indol-2(1H)-one as described in Example 25d yielded after chromatographic purification and slow crystallization from ethyl acetate yellow crystals with m.p. 202-205°C. These crystals contained according to analytical data 0.75 mol of water. Treatment of this product with ethanolic hydrogen chloride and addition of ethyl acetate gave a crystalline hydrochloride of 1-[3-[4-(2-chlorophenyl)-9-methyl-6H-thieno[3,2-f][1,2,4]triazolo[4,3-a][1,4]diazepin-2-yl]-2-propynyl]-benz[cd]indol-2(1H)-one with m.p. 219-222°C.

Example 28

1-[3-[4-(2-Chlorophenyl)-9-methyl-6H-thieno[3,2-f][1,2,4]triazolo[4,3-a][1,4]diazepin-2-yl]-2-propynyl]-1,3-dihydro-2H-indol-2-one was prepared by reacting 4-(2-chlorophenyl)--2-iodo-9-methyl-6H-thieno[3,2-f][1,2,4]-triazolo [4,3-a][1,4]diazepine with 1,3-dihydro-1-(2-propynyl)-2H-indol-2-one [ref. A. Lindquist et al., Acta Pharm. Suecica, 9, 99 (1972)] as described in Example 25d. It was isolated by chromatography and crystallized from ethyl acetate to give yellowish crystals with m.p. 203-206°C. These crystals contained according to NMR-spectral and analytical data 0.66 molar amounts of water and traces of ethyl acetate.

Example 29

Coupling of 4-(2-chlorophenyl)-2-iodo-9-methyl-6H-thieno[3,2-f][1,2,4] triazolo[4,3-a][1,4]diazepine with 1-(2-propynyl)-1H-benzimidazole [ref. I. I. Popov et al., Khim. Geterosikl. Soedin., 551, (1973)] yielded after chromatographic isolation and crystallization from ethanol/hexane off-white crystals with m.p. 215-217°C. The crystals of 2-[3-(1H-benzimidazol-1-yl)-1-propynyl]-4-(2-chlorophenyl)-9-methyl-6H-thieno[3,2-f][1,2,4]-triazolo[4,3-a][1,4]diazepine contained 0.66 molar amounts of water based on the analytical data.

Example 30

2-[3-[4-(2-Chlorophenyl)-9-methyl-6H-thieno[3,2-f][1,2,4]triazolo[4,3-a][1,4]diazepin-2-yl]-2-propynyl]-1,2,4-triazolo [4,3-a]pyridin-3(2H)-one was obtained by coupling 4-(2-chlorophenyl)-2-iodo-9-methyl-6H-thieno[3,2-f][1,2,4]triazolo[1,4] diazepine with 2-(2-propynyl)-1,2,4-triazolo[4,3-a] pyridin-3(2H)-one as described in Example 25d. It was isolated by chromatography using 7.5 % (V/V) of ethanol in methylene chloride for elution. Crystallization from ethanol gave 0.55 g (55%) of yellow crystals with m.p. 220-223 °C. According to analytical and NMR-spectral data, these crystals contained 0.25 molar amounts of ethanol.

Example 31

2-[3-[4-(2-Chlorophenyl)-9-methyl-6H-thieno[3,2-f][1,2,4]triazolo[4,3-a][1,4]diazepin-2-yl]-2-propynyl]-1,2-benzisothiazol-3(2H)-one 1,1-dioxide was prepared by reacting 4-(2-chlorophenyl)-2-iodo-9-methyl-6H-thieno[3,2-f][1,2,4]triazolo [4,3-a][1,4]diazepine with 2-(2-propynyl)-1,2-benzisothiazol-3(2H)-one 1,1-dioxide [ref. R. Granger and J. Giroux, French patent 1,273,867 Feb. 1962] as described in Example 25d. It was isolated by chromatography and crystallized from ethyl acetate to give colorless crystals with m.p. 232-234 °C.

Example 32

Coupling of 4-(2-chlorophenyl)-2-iodo-9-methyl-6H-thieno[3,2-f][1,2,4] triazolo[4,3-a][1,4]diazepine with 1-(3-pyridinyloxy)-2-propyne [ref. J. Bruhn J. Zsindely, H. Schmid and G. Frater, Helv. Chim. Acta 61, 2542 (1978)] under conditions described in Example 25d yielded after the usual chromatographic isolation resinous material containing 4-(2-chlorophenyl)-9-methyl-2-[3-(3-pyridinyloxy)-1-propynyl]-6H-thieno[3,2-f]-[1,2,4]triazolo[4,3-a][1,4]diazepine which did not crystallize and was therefore characterized spectroscopically only. NMR ($CDCl_3$): 2.72 (s,3,Me) 4.95 (s,4,$CH_2$;$C_6$-H), 6.8 (s,1,$C_3$-H), 7.2-7.6 (m,6,aromatic H), 8.26 (m,1,) and 8.36 (broad s,1) pyridine $C_2$ and $C_6$-H) ppm.

Example 33

4-(2-Chlorophenyl)-9-methyl-2-[3-(1H-indazol-1-yl)-1-propynyl]-6H-thieno[3,2-f][1,2,4]triazolo[4,3-a][1,4]-diazepine was prepared as described in Example 25d by reaction of 4-(2-chlorophenyl)-2-iodo-9-methyl-6H-thieno [3,2-f][1,2,4]triazolo[4,3-a] [1,4]diazepine with 1-(2-propynyl)-1H-indazole [ref. P. V. Tkachenko et al., Khim. Gertersikl. Soedin., 1542 (1975)]. The product was isolated and purified by chromatography and was crystallized from ethyl acetate/ ether to yield off-white crystals with m.p. 170-173 °C.

Example 34

Coupling of 6-(2-fluorophenyl)-8-iodo-1-methyl-4H-1,2,4]triazolo[4,3-a] [1,4]benzodiazepine with 1-(2-propynyl)-1H-indole [ref. A. J. Hubert and H. Reimlinger, J. Chem Soc. C. 606 (1968)] as described in Example 9 gave after chromatography and crystallization from ethyl acetate/ether off-white crystals of 6-(2-fluorophenyl)-8-[3-(1H-indol-1-yl)-1-propynyl]-1-methyl-4H-[1,2,4]triazolo[4,3-a][1,4]benzodiazepine with m.p. 167-169 °C.

Example 35

Reaction of 6-(2-fluorophenyl)-8-iodo-1-methyl-4H-[1,2,4]triazolo[4,3-a] [1,4]benzodiazepine with 3,7-dihydro-3,7-dimethyl-1-(2-propynyl)-1H-purine-2,6-dione [ref. J. W. Daly, W. L. Padgett and M. T. Shamim, J. Med. Chem. 29, 1305, (1986)] yielded after chromatography and crystallization from methylene chloride/ethanol off-white crystals of 1-[3-[6-(2-fluorophenyl)-1-methyl-4H-[1,2,4]triazolo[4,3-a][1,4]benzodiazepin-8-yl]-2-propynyl]-3,7-dihydro-3,7-dimethyl-1H-purine-2,6-dione with m.p. 290-292 °C. These crystals contained 0.75 molar amounts of water according to analytical data.

Example 36

2-[4-[6-(2-Fluorophenyl)-1-methyl-4H-[1,2,4]triazolo[4,3-a][1,4]benzodiazepin-8-yl]-3-butynyl]-1H-isoindole-1,3(2H)-dione was obtained by coupling of 6-(2-fluorophenyl)-8-iodo-1-methyl-4H-[1,2,4]triazolo-[4,3-a][1,4] benzodiazepine with 2-(3-butyn-1-yl)-1H-isoindole-1,3(2H)-dione [ref. K. J. Hoffman, P. Stenberg,

C. Ljunggren, U. Svensson, J. L. G. Nilsson, O. Erikson, A. Hartkoorn and R. Lunden, J. Med. Chem 18, 278 (1975)] as described in Example 1. The product was isolated by chromatography and crystallized from ethanol to give colorless crystals with m.p. 128-130 °C (with foaming). These crystals contained according to analytical and spectral data molar amounts of ethanol.

Example 37

4-[3-[4-(2-Chlorophenyl)-9-methyl-6H-thieno[3,2-f][1,2,4] triazolo[4,3-a][1,4]diazepin-2-yl]-2-propynyl]-2H-1,4-benzoxazin-3(4H)-one was prepared by reaction of 4-(2-chlorophenyl)-2-iodo-9-methyl-6H-thieno[3,2-f]-[1,2,4]triazolo [4,3-a][1,4]diazepine with 4-(2-propynyl)-2H-1,4-benzoxazin-3(4H)-one [ref. A. Lindquist et al., Acta Pharm. Suecica, 9, 99 (1972)] as described in Example 25d. After chromatographic isolation, the product was crystallized from ethyl acetate to give yellowish crystals with m.p. 190-192 °C. Treatment with ethanolic hydrogen chloride yielded a crystalline hydrochloride with m.p. 215-218 °C.

Example 38

1-[3-[4-(2-Chlorophenyl)-9-methyl-6H-thieno[3,2-f][1,2,4]triazolo[4,3-a][1,4]diazepin-2-yl]-2-propynyl]-3,7-dihydro-3,7-dimethyl-1H-purine-2,6-dione was obtained by coupling 4-(2-chlorophenyl)-2-iodo-9-methyl-6H-thieno[3,2-f][1,2,4]triazolo[4,3-a][1,4]diazepine with 3,7-dihydro-3,7-dimethyl-1-(2-propynyl)-1H-purine-2,6-dione [Ref. J. W. Daly, W. L. Padgett and M. T. Shamim, J. Med. Chem. 29, 1305 (1986)] as described in Example 25d. The product was isolated by chromatography and crystallized from ethyl acetate to yield yellow crystals with m.p. 277-280 °C.

Example 39

Reaction of 4-(2-chlorophenyl)-2-iodo-9-methyl-6H-thieno [3,2-f][1,2,4] triazolo[4,3-a][1,4]diazepine with 3,7-dihydro-1,3-dimethyl-7-(2-propynyl)-1H-purine-2,6-dione [ref. J. W. Daly, W. L. Padgett and M. T. Shamim, J. Med. Chem. 29, 1305 (1986)] followed by chromatographic isolation and crystallization from ethyl acetate/ethanol gave yellow crystals of 7-[3-[4-(2-chlorophenyl)-9-methyl-6H-thieno[3,2-f][1,2,4]triazolo-[4,3-a][1,4]diazepin-2-yl]-2-propynyl]-3,7-dihydro-1,3-dimethyl-1H-purine-2,6-dione with m.p. 229-232 °C. These crystals contained 0.125 molar amounts of ethyl acetate according to analytical and spectral data.

Example 40

Coupling of 4-(2-chlorophenyl)-2-iodo-9-methyl-6H-thieno[3,2-f][1,2,4] triazolo[4,3-a][1,4]diazepine with 2-(3-butyn-1-yl)-1H-benz[de]isoquinoline-1,3(2H)-dione as described in Example 25d yielded after chromatography and crystallization from ethyl acetate light yellow crystals of 2-[4-[4-(2-Chlorophenyl)-9-methyl-6H-thieno[3,2-f][1,2,4]triazolo[4,3-a][1,4]diazepin-2-yl]-3-butynyl]-1H-benz[de]isoquinoline-1,3(2H)-dione with m.p. 175-179 °C. A higher melting crystalline modification with m.p. 227-229 °C. was also observed.

The acetylenic reaction component was prepared as follows:

A mixture of 6g (0.03 mol) of 1H-benz[de]isoquinoline-1,3(2H)-dione, 4g (0.0355 mol) of potassium t-butoxide, 9g (0.04 mol of 4-tosyloxy-1-butyne [ref. G. Eglinton and M. C. Whiting, J. Chem. Soc. 3650 (1950)] and 150 ml of dimethylformamide was heated on the steam bath with stirring for 1.5 hours. The bulk of the solvent was then removed under reduced pressure and the remaining suspension was filtered. The filtrate was diluted with water and the precipitated product was collected by filtration and dissolved in methylene chloride. The solution was dried and passed over a plug of silica gel using methylene chloride for elution. The fractions containing clean product were combined and evaporated. Crystallization from methylene chloride/ethanol gave 2-(3-butyn-1-yl)-1H-benz[de]isoquinoline-1,3(2H)-dione as colorless needles with m.p. 191-193 °C.

Example 41

a) A mixture of 2,52 g of 6-(2-fluorophenyl)-8-iodo-1-methyl-4H-[1,2,4]triazolo[4,3-a][1,4]benzodiazepine, 270 mg of triphenylphosphine, 60 mg of cuprous iodide, 1,5 ml of triethylamine and 60 ml of dimethylformamide was stirred and degassed by a slow stream of argon for 10 minutes. Trimethylsilylacetylene, 1.2 ml, was then added and degassing was continued for 2 minutes. At this time 90 mg of palladium acetate was added and the mixture was stirred under argon for 4 hours at room temperature.

The reaction mixture was partitioned between methylene chloride and saturated sodium bicarbonate solution. The organic layer was washed with water, dried and evaporated, at the end azeotropically with xylene. The residue was chromatographed over 60g of silica gel (Merck, 70-230 mesh) using 5 % of ethanol in methylene chloride for elution. Crystallization of the combined clean fractions from ethyl acetate/hexane gave 2.05 g (86%) of colorless crystals of 6-(2-fluorophenyl)-1-methyl-8-[(trimethylsilyl)-ethynyl]-4H-[1,2,4]triazolo [4,3-a][1,4]benzodiazepine with m.p. 218-220 °C.

b) Sodium hydroxide, 1 ml 10N, was added to a solution of 2.3 g of 6-(2-fluorophenyl)-1-methyl-8-[-(trimethylsilyl)ethynyl]-4H-[1,2,4]triazolo [4,3-a][1,4]benzodiazepine in 50 ml of ethanol. The mixture was stirred under argon at room temperature for 1 hour and was then partitioned between methylene chloride and saturated aqueous sodium bicarbonate solution. The organic phase was separated, dried and evaporated. The residue was filtered over a pad of silica gel using 5% of ethanol in methylene chloride for elution. The filtrate was evaporated and the residue was crystallized from ethyl acetate/hexane to yield colorless crystals of 8-ethynyl-6-(2-fluorophenyl)-1-methyl-4H-[1,2,4]triazolo [4,3-a][1,4] benzodiazepine with m.p. 258-260 °C.

c) A mixture of 316 mg (1 mmol) of 8-ethynyl-6-(2-fluorophenyl)-1-methyl-4H-[1,2,4]triazolo[4,3-a][1,4]-benzodiazepine, 200 mg (1.25 mmol) of 5-bromopyrimidine, 45 mg of triphenylphosphine, 10 mg of cuprous iodide, 0.5 ml of triethylamine and 10 ml of dimethylformamide was stirred and degassed for 10 minutes by a slow stream of argon. Palladium acetate, 15 mg, was then added and stirring under argon was continued for 24 hours. The reaction mixture was partitioned between aqueous sodium bicarbonate solution and methylene chloride. The organic layer was washed with water, dried and evaporated, at the end azeotropically with xylene. The residue was chromatographed over 20g of silica gel (Merck 70-230 mesh) using 5% of ethanol in methylene chloride. The combined clean fractions were evaporated and the residue was crystallized from ethyl acetate to give off-white crystals of 6-(2-fluorophenyl)-1-methyl-8-(5-pyrimidinyl)ethynyl-4H-[1,2,4] triazolo[4,3-a][1,4]benzodiazepine with m.p. 143-146 °C.

## Example 42

a) 6-(2-Chlorophenyl)-1-methyl-8-[(trimethylsilyl)ethynyl]-4H-[1,2,4]triazolo[4,3-a][1,4]benzodiazepine was similarly prepared by coupling of 6-(2-chlorophenyl)-8-iodo-1-methyl-4H-[1,2,4]triazolo [4,3-a] [1,4]-benzodiazepine with trimethylsilylacetylene as described in Example 41a. The product was isolated by chromatography and was crystallized from ethyl acetate/ hexane to give colorless crystals with m.p. 243-245 °C.

b) 6-(2-Chlorophenyl)-8-ethynyl-1-methyl-4H-[1,2,4]triazolo [4,3-a][1,4]benzodiazepine was obtained as described in Example 41b by treatment of 6-(2-chlorophenyl)-1-methyl-8-[(trimethylsilyl)ethynyl]-4H-[1,2,4]triazolo[4,3-a][1,4]benzodiazepine with sodium hydroxide in ethanol. The product was crystallized from ethanol to give colorless crystals with m.p. 304-306 °C.

c) 6-(2-Chlorophenyl)-1-methyl-8-(2-thienylethynyl)-4H-[1,2,4]triazolo[4,3-a][1,4]benzodiazepine was prepared by coupling of 6-(2-chlorophenyl)-8-ethynyl-1-methyl-4H-[1,2,4]triazolo[4,3-a][1,4]benzodiazepine with 2-iodothiophene as described in Example 41c. The product was isolated and purified by chromatography and was crystallized from ethyl acetate to give crystals with m.p. 160-163 °C. These crystals contained according to analytical and spectral data 0.25 molar amounts of ethyl acetate and molar amounts of water.

## Example 43

a) Reaction of 4-(2-chlorophenyl)-2-iodo-9-methyl-6H-thieno[3,2-f][1,2,4] triazolo[4,3-a][1,4]diazepine with trimethylsilylacetylene as described in Example 41a yielded after chromatographic isolation and crystallization from ethyl acetate hexane off-white crystals of 4-(2-chlorophenyl)-9-methyl-2-[(trimethylsilyl)-ethynyl]-6H-thieno[3,2-f][1,2,4]triazolo[4,3-a][1,4]diazepine with m.p. 135-138 °C.

b) Treatment of 4-(2-chlorophenyl)-9-methyl-2-[(trimethylsilyl)ethynyl]-6H-thieno[3,2-f][1,2,4]triazolo [4,3-a][1,4]diazepine with sodium hydroxide in ethanol gave after chromatographic purification and crystallization from methanol/ethyl acetate colorless crystals of 4-(2-chlorophenyl)-2-ethynyl-9-methyl-6H-thieno-[3,2-f][1,2,4] triazolo [4,3-a][1,4]diazepine with m.p. 232-233 °C.

c) A mixture of 0.34g of 4-(2-chlorophenyl)-2-ethynyl-9-methyl-6H-thieno[3,2-f][1,2,4]triazolo[4,3-a][1,4]-diazepine, 45 mg of triphenylphosphine, 10 mg of cuprous iodide, 1 ml of triethylamine, 0.38 g of 1-iodonaphthalene and 10 ml of dimethylformamide was degased for 10 minutes by a slow stream of argon. After addition of 15 mg of palladium acetate the mixture was stirred under argon for 2 hours at room temperature. The reaction mixture was poured into saturated sodium bicarbonate solution and ice.

The precipitate was filtered off, washed with water and dissolved in methylene chloride. The solution was dried and evaporated and the residue was chromatographed over 15 g of silica gel using 25% (v/v) of hexane in tetrahydrofuran. The combined clean factions containing the product were evaporated and the residue was crystallized from ethyl acetate/hexane to give off-white crystals of 4-(2-chlorophenyl)-2-[(1-naphthyl)ethynyl]-9-methyl-6H-thieno[3,2-f][1,2,4]triazolo[4,3-a][1,4]diazepine with m.p. 197-199 °C.

Example 44

4-(2-Chlorophenyl)-2-iodo-9-methyl-6H-thieno[3,2-f][1,2,4] triazolo[4,3-a][1,4] diazepine was reacted with 1-(2-propynyl)- 2(1H)quinolinone [ref. A. Lindquist et al., Acta Pharm. Suecica, 9, 99 (1972)] as described in Example 25d. The product was isolated by chromatography over 50 g of silica gel using 5 % (v/v) of ethanol in methylene chloride and was further purified by rechromatography over 50 g of silica gel using tetrahydrofuran. Crystallization from ethyl acetate/methanol gave off-white crystals of 1-{3-[4-(2-chlorophenyl)-9-methyl-6H-thieno[3,2-f][1,2,4]triazolo[4,3-a][1,4]diazepin-2-yl]-2-propynyl}-2(1H)-quinolinone with m.p. 162-165 °C. These crystals contained according to analytical data 0.5 molar amounts of water.

Example 45

A mixture of 33 g (0.075 mol) of 4-(2-chlorophenyl)-2-iodo-9-methyl-6H-thieno[3,2,f][1,2,4]triazolo[4,3-a]-[1,4]diazepine, 21 g (0.113 mol) of 3,4-dihydro-1-(2-propynyl)-2(1H)-quinolinone [ref. A. Lindquist et al., Acta Pharm. Suecica, 9, 99 (1972)], 0.75 g of triphenylphosphine, 0.2 g of cuprous iodide, 60 ml of triethylamine and 600 ml of dimethylformamide was stirred and degassed by a stream of argon for 30 minutes. At this time 0.225 g of palladium acetate was added and the mixture was stirred at room temperature under argon for 3 days. The mixture was poured into 2.5 l of saturated aqueous sodium bicarbonate solution and ice. After stirring for 15 minutes, the precipitate was filtered off, washed with water and sucked dry. This material was dissolved in methylene chloride and the solution was washed with bicarbonate solution and dried over sodium sulfate. The solvent was removed under reduced pressure and the residue was dissolved by warming in ethyl acetate. After seeding and cooling, the crystallized product was collected and recrystallized from methanol/ethyl acetate to leave off-white crystals of 1-{3-[4-(2-chlorophenyl)-9-methyl-6H-thieno[3,2,f][1,2,4]triazolo[4,3-a][1,4]diazepin-2-yl]-2-propynyl}-3,4-dihydro-2(1H)-quinolinone with m.p. 180-182 °C.

Example 46

4-(2-Chlorophenyl)-2-iodo-9-methyl-6H-thieno[3,2,f][1,2,4]triazolo[4,3-a][1,4]diazepine was reacted with 2,3-dihydro-2-(2-propynyl)-1H-benz[de]isoquinolin-1-one under the conditions described in Example 25d. The product was isolated by chromatography over 40 fold amount of silica gel using tetrahydrofuran for elution. The fractions containing the title compound were combined and rechromatographed over the 30 fold amount of silica gel using 5 % (v/v) of ethanol in methylene chloride. The combined clean fractions crystallized from methanol/ethyl acetate to give yellowish crystals of 2-{3-[4-(2-chlorophenyl)-9-methyl-6H-thieno[3,2,f][1,2,4]triazolo[4,3-a][1,4]diazepin-2-yl]-2-propynyl}-2,3-dihydro-1H-benz[de]isoquinolin-1-one with m.p. 205-210 ° with dec. These crystals contained 0.66 molar amounts of water according to the analytical and spectral data.

The required propargyl derivative was prepared as follows:

A mixture of 2 g of 2-(2-propynyl)-1H-benz[de]isoquinoline-1,3(2H)-dione, 0.75 g of sodium borohydride, 50 ml of ethanol and 50 ml of tetrahydrofuran was warmed on the steam bath until solution was complete. An additional portion of 0.25 g of sodium borohydride was then added and the tetrahydrofuran was boiled off on the steam bath within 30 minutes. The remaining mixture was cooled, diluted with ice-water, buffered with acetic acid and diluted with aqueous sodium bicarbonate solution. The precipitated product was filtered off after stirring over ice, was sucked dry and dissolved in about 250 ml of methylene chloride. The solution was dried and evaporated and the residue was slurried with methylene chloride/hexane. The crystals were collected and washed with ether to leave 0.67 g of 2,3-dihydro-3-hydroxy-2-(2-propynyl)-1H-benz[de] isoquinolin-1-one which was reduced further as follows.

Sodium borohydride 0.3 g, was added in small portions to a stirred solution of 0.6 g of the above intermediate in 6 ml of trifluoroacetic acid. After 15 minutes of reaction time, the mixture was partitioned between ice, ammonium hydroxide and methylene chloride. The organic layer was separated, dried and evaporated to leave a crystalline residue which was chromatographed over 30g of silica gel using 10 % (v/v) of ethyl acetate in methylene chloride. Crystallization of the combined clean fractions from ethyl

acetate/hexane gave colorless crystals of 2,3-dihydro-2-(2-propynyl)-1H-benz[de]isoquinolin-1-one with m.p. 139-140°.

Example 47

4-(2-Chlorophenyl)-2-iodo-9-methyl-6H-thieno[3,2,f][1,2,4]triazolo[4,3-a][1,4]diazepine was reacted with 1,3-dihydro-1-methyl-3-(2-propynyl)-2H-benzimidazol-2-one under the conditions described in Example 25d. The product was isolated by chromatography over the 50 fold amount of silica gel using tetrahydrofuran. Crystallization of the combined homogenous fractions from ethanol gave light yellow crystals of 1-{3-[4-(2-chlorophenyl)-9-methyl-6H-thieno[3,2,f][1,2,4]triazolo[4,3-a][1,4]diazepin-2-yl]-2-propynyl}-1,3-dihydro-3-methyl-2H-benzimidazol-2-one with m.p. 188-191°.

The required acetylene was prepared as follows:

Potassium tert.-butoxide, 2.1 g (18.5 mmol), was added to a solution of 2.5 g (16.9 mmol) of 1,3-dihydro-1-methyl-2H-benzimidazol-2-one in 25 ml of dimethylformamide. After stirring under nitrogen for 15 minutes, 2.21 g (18.5 mmol) of propargyl bromide was added and the mixture was stirred at room temperature for 30 minutes. It was diluted with ice-water and the precipitate was filtered off, washed with water and sucked dry. The crude product was passed over silica gel using 10 % (v/v) of ethyl/acetate in methylene chloride for elution. Crystallization from ethyl acetate/hexane gave colorless crystals of 1,3-dihydro-1-methyl-3-(2-propynyl)-2H-benzimidazol-2-one with m.p. 110-112°.

Example 48

4-(2-Chlorophenyl)-2-iodo-9-methyl-6H-thieno[3,2,f][1,2,4] triazolo[4,3-a][1,4]diazepine was reacted with rac-2a,3,4,5-tetrahydro-2a-(2-propynyl)benz[cd]indol-2(1H)-one under the conditions described in Example 9. The product was isolated by chromatography over the 50-fold amount of silica gel using 5 % (v/v) of ethanol in methylene chloride for elution. The combined clean fractions were evaporated and the residue was crystallized from ethyl acetate/ether to give colorless crystals of rac-2a-{3-[4-(2-chlorophenyl)-9-methyl-6H-thieno[3,2,f][1,2,4]triazolo[4,3-a][1,4]diazepin-2-yl]-2-propynyl}-2a,3,4,5-tetrahydrobenz[cd]indol-2(1H)-one with m.p. 215-217°.

The required propargyl compound was prepared as follows:

Potassium tert.-butoxide, 4.94 g (0.044 mol), was added to a solution of 6.92 g (0.04 mol) of rac-2a,3,4,5-tetrahydrobenz[cd]indol-2(1H)-one in 50 ml of dimethylformamide. After stirring for 15 minutes under nitrogen, 5.23 g or 3.9 ml of propargyl bromide was added and stirring was continued for 30 minutes. The mixture was diluted with water and ice and the precipitate was collected by filtration, washed with water and sucked dry. The solids were dissolved in methylene chloride and the solution was dried and evaporated. Crystallization of the residue from ethyl acetate gave crude product of rac-2a,3,4,5-tetrahydro-2a-(2-propynyl)benz[cd]indol-2(1H)-one with m.p. 174-177° which was recrystallized from ethyl acetate to melt at 177-180°.

Example 49

4-(2-Chlorophenyl)-2-iodo-9-methyl-6H-thieno[3,2,f][1,2,4] triazolo[4,3-a][1,4]diazepine was reacted with 5-(2-propynyl)-5H-carbazole [ref. J. L. Dumont et al., Bull. Soc. Chim. Fr. 1197, (1967)] under the conditions described in Example 25d. The product was purified by chromatography over the 60-fold amount of silica gel using 5% (v/v) of ethanol in methylenechloride. The combined clean fractions did not crystallize and the viscous resin was converted to a crystalline dihydrochloride by treatment with excess ethanolic hydrogen chloride in ethanol/ethyl acetate. The yellow crystals of 2-[3-(9H-carbazol-9-yl)-1-propynyl]-4-(2-chlorophenyl)-9-methyl- 6H-thieno[3,2,f] [1,2,4]triazolo[4,3-a][1,4]diazepine dihydrochloride had m.p. 180-184°.

Example 50

A mixture of 44 g (0.1 mol) of 4-(2-chlorophenyl)-2-iodo-9-methyl-6H-thieno[3,2,f][1,2,4]triazolo[4,3-a]-[1,4]diazepine, 28 g (0.12 mol) of 5-(2-propynyl)-6(5H)-phenanthridinone, [ref. R. F. Cookson et al. J. Herterocyclic Chem., 9, 475 (1972)], 1 g of triphenylphosphine, 0.25 g of cuprous iodide, 80 ml of triethylamine and 800 ml of dimethylformamide was degassed by as slow stream of argon for 30 minutes. Palladium acetate, 0.3 g, was then added and the mixture was stirred under argon for 4 days at room temperature. The insoluble material was removed by filtration over celite and the filtrate was concentrated to

about 400 ml under reduced pressure. This solution was poured into 2 l of saturated aqueous sodium bicarbonate with stirring. The precipitate was collected by filtration after 10 minutes and was washed with water and sucked dry. The solids were partitioned between 2 l of methylene chloride containing 5 % (v/v) of ethanol and sodium bicarbonate solution. The organic layer was dried over sodium sulfate, filtered and evaporated partially. After dilution with 500 ml of ethyl acetate, the solution was concentrated on the steam bath with crystallization of the product. After cooling, the crystals were collected and washed with ethyl acetate and ether to leave 56.5 g of product. The analytical sample was recrystallized once from ethanol and then from tetrahydrofuran/ethyl acetate to give off-white crystals of 5-{3-[4-(2-chlorophenyl)-9-methyl-6H-thieno[3,2-f][1,2,4] triazolo[4,3-a][1,4]diazepin-2-yl]-2-propynyl}-phenanthridin-6(5H)-one with m.p. 247-249°.

Example 51

4-(2-Chlorophenyl)-2-iodo-9-methyl-6H-thieno[3,2-f][1,2,4]triazolo[4,3-a][1,4]diazepine as reacted with 6-(2-propynyl)-5H-dibenz[c,e]azepine-5,7(6H)-dione [ref. J. R. Grunder et al., J. Pharm. Sci., 62, 1204 (1973)] under the conditions used in Example 25d. The product was purified by chromatography over the 50-fold amount of silica gel using 5 % (v/v) of ethanol in methylene chloride. The combined clean fractions were evaporated and the residue was crystallized from ethanol/ethyl acetate to give off-white crystals of 6-{3-[4-(2-chlorophenyl)-9-methyl-6H-thieno[3,2-f][1,2,4]triazolo[4,3-a][1,4]diazepin-2-yl]-2-propynyl}-5H-dibenz[c,e]-azepine-5,7(6H)-dione with m.p. 220-223°. These crystals contained 0.166 molar amounts of ethyl acetate according to spectral and analytical data.

Example 52

4-(2-Chlorophenyl)-2-iodo-9-methyl-6H-thieno[3,2-f][1,2,4]triazolo[4,3-a][1,4]diazepine was reacted with rac-2a,3,4,5-tetrahydro-2a-methyl-1-(2-propynyl)-benz[c,d]indol-2(1H)-one under the conditions used in Example 25d. The product was purified by chromatography over the 50-fold amount of silica gel using 5 % (v/v) of ethanol in methylene chloride for elution. The combined clean fractions were evaporated to leave a resinous material which did not crystallize but yielded a crystalline dihydrochloride upon treatment with excess ethanolic hydrogen chloride and ethyl acetate. These crystals contained 0.33 molar amounts of ethanol according to spectral and analytical data. The pale yellow crystals of 1-{3-[4-(2-chlorophenyl)-9-methyl-6H-thieno[3,2-f][1,2,4]triazolo[4,3-a][1,4]diazepin-2-yl]-2-propynyl}-2a,3,4,5-tetrahydro-2a-methylbenz[c,d]indol-2-(1H)-one dihydrochloride had m.p. 175-178°.

The propargyl starting material wa synthesized as follows:

Potassium tert.-butoxide, 4.94 g (0.044 mol), was added to a solution of 6.92 g (0.04 mol) of rac-2a,3,4,5-tetrahydrobenz[cd]indol-2(1H)-one in 50 ml of dimethylformamide. After stirring for 15 minutes under nitrogen, 6.24 g or 2.75 ml (0.044 mol) of methyl iodide was added and stirring was continued for 30 minutes. After dilution with water and ice, it was extracted with methylene chloride. The extracts were dried over sodium sulfate and evaporated. The residue was chromatographed over silica gel using 10 % (v/v) of ethyl acetate in methylene chloride. The combined clean fractions were evaporated and the residue was crystallized from ether to give 3.2 g of colorless rac-2a,3,4,5-tetrahydro-2a-methylbenz[c,d]indol-2(1H)-one with m.p. 148-150°.

This material, 2.5 g (13.3 mmol), was dissolved in 20 ml of dimethylformamide. The solution was treated with 1.65 g (14.7 mmol) of potassium tert.-butoxide and was stirred under nitrogen for 15 minutes. Propargyl bromide, 1.75 g or 1.31 ml (14 mmol), was then added and stirring at room temperature was continued for 30 minutes. The reaction mixture was diluted with ice and saturated sodium bicarbonate solution. The precipitate was collected by filtration and the solids were washed with water and sucked dry. It was dissolved in methylene chloride and the dried solution was filtered over a plug of silica gel. The filtrate was evaporated and the residue was crystallized from ethyl acetate/hexane to give colorless crystals of rac-2a,3,4,5-tetrahydro-2a-methyl-1-(2-propynyl)benz[c,d] indol-2(1H)-one with m.p. 127-130°.

Example 53

4-(2-Chlorophenyl)-2-iodo-9-methyl-6H-thieno[3,2-f][1,2,4]triazolo[4,3-a][1,4]diazepine was reacted with 3-phenoxy-1-propyne under the conditions described in Example 25d. The product was purified by chromatography over the 50-fold amount of silica gel using 5 % (v/v) of ethanol in methylene chloride. The evaporated clean fractions left a viscous oil which did not crystallize but yielded a crystalline dihydrochloride upon treatment with excess ethanolic hydrogen chloride and ethyl acetate. The pale yellow crystals of

4-(2-chlorophenyl)-9-methyl-2-[3-(phenoxy)-1-propynyl]-6H-thieno[3,2-f][1,2,4]triazolo [4,3-a] [1,4]diazepine dihydrochloride melted with foaming at 148-151° and analyzed for a hydrate.

Example 54

A mixture of 0.88 g of 4-(2-chlorophenyl)-2-iodo-9-methyl-6H-thieno[3,2-f][1,2,4]triazolo[4,3-a] [1,4]-diazepine, 0.65 g of 3-methyl-1-(2-propynyl)-2,4(1H,3H)-quinazolinedione [ref. B. Danielsson et al., Acta Pharm. Suecica, 2, 167 (1965)], 90 mg of triphenylphosphine, 20 mg of cuprous iodide, 2 ml of triethylamine and 50 ml of dimethylformamide was degassed with argon for 10 minutes. Palladium acetate, 30 mg, was then added and the mixture was heated up to 90-95° within 30 minutes with stirring under argon. The temperature was maintained at 90-95° for 15 minutes and the bulk of the dimethylformamide was evaporated under reduced pressure. The residue was partitioned between methylene chloride and aqueous sodium bicarbonate solution. The organic layer was dried and evaporated, at the end azeotropically with xylene. The residue was chromatographed over 30 g of silica gel using tetrahydrofuran/hexane 4:1 for elution. The combined clean fractions were evaporated and the resin obtained was crystallized from ethanol to give colorless crystals of 1-{3-[4-(2-chlorophenyl)-9-methyl-6H-thieno[3,2-f][1,2,4]triazolo[4,3-a]-[1,4]diazepin-2-yl]-2-propynyl}-3-methyl-2,4(1H,3H)-quinazolinedione with m.p. 167-170°. These crystals contained 0.5 molar amounts of water according to analytical and spectral data.

Example 55

4-(2-Chlorophenyl)-2-iodo-9-methyl-6H-thieno[3,2-f][1,2,4] triazolo[4,3-a][1,4]diazepine was reacted with 5-(2-propynyl)-5H- dibenz[b,e]azepine-6,11-dione under the conditions described in Example 25d. The product was purified by chromatography over the 50-fold amount of silica gel using tetrahydrofuran/hexane 4 : 1 for elution. The resin left after evaporation of the combined clean fractions was crystallized from ethyl acetate to give colorless crystals of 5-{3-[4-(2-chlorophenyl)-9-methyl-6H-thieno[3,2-f][1,2,4]triazolo[4,3-a]-[1,4]diazepin-2-yl]-2-propynyl}-5H-dibenz[b,e]azepine-6,11-dione with m.p. 245-247°. These crystals contained 0.33 molar amounts of ethyl acetate according to analytical and spectral data.

The acetylenic starting material was prepared as follows:

Potassium tert.-butoxide, 2.5 g, was added to a suspension of 4.5 g of 5H-dibenz[b,e]azepine-6,11-dione in 50 ml of dimethylformamide. After stirring under nitrogen for 30 minutes, 2 ml of propargyl bromide was added and stirring was continued for 1 hour at room temperature. The reaction mixture was acidified with acetic acid and diluted with water. The precipitate was filtered off and sucked dry. The solids were dissolved in methylene chloride. The solution was dried and evaporated and the residue was chromatographed over 120 g of silica gel using methylene chloride. The combined clean fractions containing product were evaporated and the residue was crystallized from ether/hexane to give colorless crystals of 5-(2-propynyl)-5H-dibenz[b,e]azepine-6,11-dione with m.p. 117-118°.

Example 56

4-(2-Chlorophenyl)-2-iodo-9-methyl-6H-thieno [3,2-f][1,2,4]triazolo[4,3-a][1,4]diazepine was reacted with 6-chloro-4-(2-propynyl)-2H-1,4-benzoxazin-3(4H)-one [ref. P. Rao et al. Indian J. Chem. 24 B, 1120 (1985)] as described in Example 25d but the reaction time was extended to 72 hours. The product was isolated by chromatography over the 100-fold amount of silica gel (Merck 230-400 mesh) using 10 % (v/v) of ethanol in methylene chloride. Crystallization of the residue obtained after evaporation of the combined clean fractions from ethyl acetate gave colorless crystals of 6-chloro-4-{3-[4-(2-chlorophenyl)-9-methyl-6H-thieno[3,2-f]-[1,2,4]triazolo[4,3-a][1,4]diazepin-2-yl]-2-propynyl}-2H-1,4-benzoxazin-3(4H)-one with m.p. 202-205°.

Example 57

4-(2-Chlorophenyl)-2-iodo-9-methyl-6H-thieno [3,2-f][1,2,4]triazolo[4,3-a][1,4]diazepine was reacted with 1,4-dihydro-1-(2-propynyl)-3,1-benzoxazin-2-one under the conditions described in Example 25d. The product was isolated by chromatography over the 50-fold amount of silica gel using 5 % (v/v) of ethanol in methylene chloride and further purified by rechromatography over the 30-fold amount of silica gel using tetrahydrofuran/hexane 4 : 1 for elution. The clean fractions were evaporated and the residue was crystallized from methanol/ethyl acetate to give off-white crystals of 1-{3-[4-(2-chlorophenyl)-9-methyl-6H-thieno[3,2-f][1,2,4]triazolo[4,3-a][1,4]diazepin-2-yl]-2-propynyl}-2H-3,1-benzoxazin-2-one with m.p. 173-176°. These crystals contained 0.33 molar amounts of water, according to the analytical data.

The required acetylene was synthesized as follows:

Potassium tert.-butoxide, 3.9 g (34.6 mmol) was added to a solution of 4.7 g (31 mmol) of 1,4-dihydro-3,1-benzoxazin-2-one in 30 ml of dimethylformamide. After stirring for 15 minutes under nitrogen, 4.1 g or 3.1 ml of propargyl bromide was added and stirring at room temperature was continued for 30 minutes. The reaction mixture was diluted with saturated aqueous sodium bicarbonate solution and the precipitated product was filtered off, washed with water and sucked dry. The residue was dissolved in methylene chloride and the solution was passed over a plug of silica gel.

The filtrate was evaporated and the residue was crystallized from methanol to give colorless crystals of 1,4-dihydro-1-(2-propynyl)-3,1-benzoxazin-2-one with m.p. 123-125°.

Example 58

4-(2-Chlorophenyl)-2-iodo-9-methyl-6H-thieno[3,2-f][1,2,4]triazolo[4,3-a][1,4]diazepine was reacted with 2-ethynylpyridine under the conditions used in Example 25d. The product was isolated by chromatography over the 50-fold amount of silica gel using 5 % (v/v) of ethanol in methylene chloride. The fractions homogenous by TLC were combined and evaporated. Crystallization of the residue from ethyl acetate and recrystallization from tetrahydrofuran/methanol gave off-white crystals of 4-(2-chlorophenyl)-9-methyl-2-[2-(2-pyridinyl)ethynyl]-6H-thieno[3,2-f][1,2,4]triazolo[4,3-a][1,4]diazepine with m.p. 153-155°.

Example 59

4-(2-Chlorophenyl)-2-iodo-9-methyl-6H-thieno[3,2-f] [1,2,4]triazolo[4,3-a][1,4]diazepine was reacted with 2-(2-propynyl)-1(2H)-isoquinolinone under the conditions described in Example 25d. The product was isolated by chromatography over the 50-fold amount of silica gel using tetrahydrofuran/hexane 4 : 1. The fractions clean by TLC were combined and evaporated and the residue was crystallized from ethanol/ethyl acetate to give colorless crystals of 2-{3-[4-(2-chlorophenyl)-9-methyl-6H-thieno[3,2-f][1,2,4]triazolo[4,3-a]-[1,4]diazepin-2-yl]-2-propynyl}-1(2H)-isoquinolinone with m.p. 148-150°. These crystals contained according to analytical data 0.66 molar amounts of water.

The required acetylene was prepared as follows:

A solution of 1 g (7 mmol) of isocarbostyril in 40 ml of dimethylformamide was treated with 0.86 g (7.7 mmol) of potassium-tert.-butoxide. After stirring under nitrogen for 30 minutes, 0.7 ml of propargyl bromide was added and stirring at room temperature was continued for 1 hour. The reaction mixture was acidified with acetic acid and diluted with water. The precipitated product was filtered off, washed with water and sucked dry. The residue was purified by chromatography over 30 g of silica gel using 5 % (v/v) of ethanol in methylene chloride and crystallization from ether to give colorless crystals of 2-(2-propynyl)-1(2H)-isoquinolinone with m.p. 104-105°.

Example 60

4-(2-Chlorophenyl)-2-iodo-9-methyl-6H-thieno-[3,2-f][1,2,4]triazolo[4,3-a][1,4]diazepine was reacted with 1,3-dihydro-1-phenyl-3-(2-propynyl)-2H-benzimidazol-2-one under the conditions used in Example 25d. The product was isolated by chromatography over the 50-fold amount of silica gel using 5 % (v/v) of ethanol in methylene chloride. The clean fractions were combined and evaporated and the residue was crystallized from methanol/ethyl acetate and recrystallized from methanol to yield off-white crystals of 1-{3-[4-(2-chlorophenyl)-9-methyl-6H-thieno[3,2-f][1,2,4]triazolo[4,3-a][1,4]diazepin-2-yl]-2-propynyl}-1,3-dihydro-3-phenyl-2H-benzimidazol-2-one with m.p.176-179°. These crystals contained 0.66 mol of water according to analytical data.

The required propargyl derivative was synthesized as follows:

Potassium tert.-butoxide, 1.76 g (15.7 mmol) was added to a solution of 3 g (14.2 mmol) of 1,3-dihydro-1-phenyl-2H-benzimidazol-2-one in 30 ml of dimethylformamide and the mixture was stirred under nitrogen for 15 minutes. Propargyl bromide, 1.4 ml (15 mmol) was then added and stirring was continued for 30 minutes at room temperature and 15 minutes on the steam bath. The product was precipitated by dilution with saturated aqueous sodium bicarbonate solution and was filtered off, washed with water and sucked dry. It was dissolved in methylene chloride and the solution was passed over a plug of silica gel using methylene chloride. The filtrate was evaporated and the residue was crystallized from methylene chloride/ethyl acetate to give colorless crystals of 1,3-dihydro-1-phenyl-3-(2-propynyl)-2H-benzimidazol-2-one with m.p. 145-147°.

Example 61

4-(2-Chlorophenyl)-2-iodo-9-methyl-6H-thieno[3,2-f][1,2,4]triazolo[4,3-a][1,4]diazepine was reacted with 6,8-dichloro-3,4- dihydro-1-(2-propynyl)-2(1H)-quinolinone under the conditions used in Example 25d. The product was purified by chromatography over the 50-fold amount of silica gel using tetrahydrofuran/hexane 4 : 1. The fractions homogenous by TLC were combined and evaporated. The residue was crystallized from ethyl acetate to give colorless crystals of 1-{3-[4-(2-chlorophenyl)-9-methyl-6H-thieno[3,2-f][1,2,4]triazolo-[4,3-a][1,4]diazepin-2-yl]-2-propynyl}-6,8-dichloro-3,4-dihydro-2(1H)-quinolinon e with m.p. 163-165°.

The required propargyl derivative was prepared as follows:

A mixture of 1.5 g of 6,8-dichloro-3,4-dihydro-2(1H)-quinolinone, 1.3 g of barium oxide, 40 ml of dimethylformamide and 0.8 ml of propargyl bromide was heated on the steam bath for 45 minutes and was stirred for an additional hour without heating. The product was precipitated by addition of ice and water and was collected by filtration. The solids were dissolved in methylene chloride and the solution was dried and evaporated. The residue was crystallized from ether/hexane to give colorless crystals of 6,8-dichloro-3,4-dihydro-1-(2- propynyl)-2(1H)-quinolinone with m.p. 92-95°.

The starting material was obtained as follows:

A solution of 6 g of 3,4-dihydro-2(1H)-quinolinone in 50 ml of formic acid and 50 ml of conc. hydrochloric acid was added to 75 ml of 1,2-dichloroethane which had been saturated with chlorine. The mixture was stirred over ice for 30 minutes. Chlorine was introduced for 5 minutes and stirring in the cold was continued for 2 hours. It was then poured onto ice, was made basic with ammonium hydroxide and was extracted with methylene chloride. The extracts were dried and evaporated and the residue was chromatographed over 300 g of silica gel using 8 % (v/v) of ethyl acetate in methylene chloride. Crystallization of the combined clean fractions from ether gave colorless crystals of 6,8-dichloro-3,4-dihydro-2(1H)-quinolinone with m.p. 145-146°.

Example 62

4-(2-Chlorophenyl)-2-iodo-9-methyl-6H-thieno-[3,2-f][1,2,4]triazolo[4,3-a][1,4]diazepine was reacted with 3,4-dihydro-2-(2-propynyl)-1(2H)-isoquinolinone [ref. W. Schneider et al. Arch. Pharm., 291, 560 (1958)] under the conditions described in Example 25d. The product was isolated by chromatography over the 50-fold amount of solica gel using tetrhydrofuran/hexane 4 : 1 for elution. The clean fractions were combined and evaporated and the residue was crystallized from ethyl acetate to give colorless crystals of 2-{3-[4-(2-chlorophenyl)-9-methyl-6H-thieno[3,2-f][1,2,4]triazolo[4,3-a][1,4]diazepin-2-yl]-2-propynyl}-3,4-dihydro-1(2H)-isoquinolinone with m.p. 164-166°.

Example 63

4-(2-Chlorophenyl)-2-iodo-9-methyl-6H-thieno[3,2-f][1,2,4] triazolo[4,3-a][1,4]diazepine was reacted with 7-fluoro-4-(2-propynyl)-2H-1,4-benzoxazin-3(4H)-one under the conditions used in Example 25d. The product was isolated by chromatography over the 50 fold amount of silica gel using 4 % (v/v) of ethanol in methylene chloride. The good fractions were combined and evaporated and the residue was crystallized from ethyl acetate to give colorless crystals of 4-{3-[4-(2-chlorophenyl)-9-methyl-6H-thieno[3,2-f][1,2,4]-triazolo[4,3-a][1,4]diazepin-2-yl]-2-propynyl}-7-fluoro-2H-1,4-benzoxazin-3(4H)-one with m.p. 215-217°.

The required propargyl compound was prepared as follows:

A mixture of 1.7 g of 7-fluoro-2,4-dihydro-1,4-benzoxazin-3-one, 2 g of barium oxide, 1.3 ml of propargyl bromide and 40 ml of dimethyl formamide was heated on the steam bath for 45 minutes. The cooled reaction mixture was diluted with water and the precipitated product was collected by filtration. The precipitate was dissolved in methylene chloride and the solution was washed with water, dried and evaporated. The residue was crystallized from ether/hexane to give colorless crystals of 7-fluoro-4-(2-propynyl)-2H-1,4- benzoxazin-3(4H)-one with m.p. 98-100°.

Example 64

4-(2-Chlorophenyl)-2-iodo-9-methyl-6H-thieno-[3,2-f][1,2,4] triazolo[4,3-a][1,4]diazepine was reacted with 1,2,3,4-tetrahydro-9-(2-propynyl)-9H-carbazole under the conditions used in Example 25d. The product was isolated by chromatography over the 50-fold amount of silica gel using 8 % (v/v) of ethanol in methylene chloride for elution. The residue obtained after evaporation of the combined clean fractions containing product was crystallized from ethanol to give colorless crystals of 4-(2-chlorophenyl)-2-[3-(1,2,3,4-tetrahydro-9H-carbazol-9-yl)-1-propynyl]-9-methyl-6H-thieno-[3,2-f][1,2,4]triazolo[4,3-a][1,4] diazepine with m.p. 164-166°. These crystals contained 0.66 molar amounts of ethanol according to analytical and spectral data.

The required propargyl compound was synthesized as follows:

Potassium tert.-butoxide, 3.6 g (33 mmol), was added to a solution of 5 g (30 mmol) of 1,2,3,4-tetrahydro-9H-carbazole in 50 ml of dimethylformamide. After stirring for 45 minutes under nitrogen, 3.9 g or 2.9 ml (33 mmol) of propargyl bromide was added and stirring was continued for 30 minutes at room temperature followed by 45 minutes on the steam bath. The cooled reaction mixture was diluted with water and the product was extracted with ether. The extracts were washed with water, dried over sodium sulfate and evaporated. The residue was passed over a plug of silica gel using methylene chloride/hexane 1 : 1. The fractions containing the least polar product were combined and evaporated. The residue was crystallized from hexane to give colorless crystals of 1,2,3,4-tetrahydro-9-(2-propynyl)-9H-carbazole with m.p. 74-76°.

Example 65

A mixture of 0.88 g (2 mmol) of 4-(2-chlorophenyl)-2-iodo-9-methyl-6H-thieno[3,2-f][1,2,4]triazolo[4,3-a]-[1,4] diazepine, 0.6 g (2.6 mmol) of 10-(2-propynyl)-9(10H)-acridinone [ref.A. R. Katritzky et al. J. Org. Chem. 50, 852 (1985)], 80 mg of triphenylphosphine, 20 ml of cuprous iodide, 5.6 ml of triethylamine and 50 ml of dimethylformamide was degassed with argon for 10 minutes. Palladium acetate, 25 mg, was then added and the mixture was heated to 80-90° for 30 minutes. The product was precipitated by addition of saturated aqueous sodium bicarbonate solution and was collected by filtration. The solids were dissolved in methylene chloride and the solution was dried and evaporated. The residue was chromatographed over 50 g of silica gel using 5 % (v/v) of ethanol in methylene chloride for elution. The combined clean fractions were evaporated and the residue was crystallized from ethanol to yield yellow crystals of 10-{3-[4-(2-chlorophenyl)-9-methyl-6H-thieno[3,2-f][1,2,4]triazolo[4,3-a][1,4]diazepin-2-yl]-2-propynyl}-9(10H)-acridinone with m.p. 175-180° with foaming. These crystals contained molar equivalents of water.

Example 66

4-(2-Chlorophenyl)-2-iodo-9-methyl-6H-thieno[3,2-f] [1,2,4]triazolo[4,3-a][1,4]diazepine was reacted with 3,8-dichloro-5-(2-propynyl)-6(5H)-phenanthridinone under the conditions used in Example 25d, but at the end, the reaction mixture was heated for 5 minutes at 90-95°. The product was isolated by chromatography over the 50-fold amount of silica gel using tetrahydrofuran/hexane 4 : 1 for elution. Crystallization of the combined clean fractions from tetrahydrofuran/ethyl acetate gave colorless crystals of 3,8-dichloro-5-{3-[4-(2-chlorophenyl)-9-methyl-6H-thieno[3,2-f][1,2,4]triazolo[4,3-a][1,4]diazepin-2-yl]-2-propynyl}-6(5H)-phenanthridinone with m.p. 218-220°.

The propargyl derivative used for this experiment was prepared as follows:

A mixture of 5 g (19 mmol) of 3,8-dichloro-6(5H)-phenanthridinone, 3.2 g (21 mmol) of barium oxide and 1.9 ml (21 mmol) of propargyl bromide in 40 ml of dimethylformamide was heated on the steam bath for 1 hour. After cooling, the product was precipitated by addition of water, filtered off and washed with water. It has dissolved in methylene chloride and the solution was dried and evaporated. The residue was chromatographed over 150 g of silica gel using 10 % (v/v) of hexane in methylene chloride. The fractions containing the product were combined and evaporated. The solid residue was recrystallized from ethanol to give colorless crystals of 3,8-dichloro-5-(2-propynyl)-6(5H)-phenanthridinone with m.p. 248-250°.

Example 67

4-(2-Chlorophenyl)-2-iodo-9-methyl-6H-thieno[3,2-f][1,2,4] triazolo[4,3-a][1,4]diazepine was reacted with phenylacetylene under the conditions described in Example 25d. The product was isolated by chromatography over the 50-fold amount of silica gel using 5 % (v/v) of ethanol in methylene chloride for elution. The combined clean fractions were evaporated and the residue was crystallized from ethyl acetate to give colorless crystals of 4-(2-chlorophenyl)-9-methyl-2-(phenylethynyl)-6H-thieno[3,2-f][1,2,4]triazolo[4,3-a][1,4]-diazepine with m.p. 215-217°.

Example 68

A mixture of 0.84 g (2 mmol) of 6-(2-fluorophenyl)-8-iodo-1-methyl-4H-[1,2,4]triazolo[4,3-a] [1,4]-benzodiazepine, 0.48 g (2.6 mmol) of 3,4-dihydro-1-(2-propynyl)-2(1H)-quinolinone, 80 mg of triphenylphosphine, 20 mg of cuprous iodide, 1.5 ml of triethylamine and 50 ml of dimethylformamide was degassed with argon for 10 minutes. Palladium acetate, 25 mg, was then added and stirring was continued for 18 hours. The solvent was evaporated under reduced pressure and the residue was partitioned between methylene chloride and saturated aqueous sodium bicarbonate solution. The organic layer was separated, dried and evaporated and the residue was chromatographed over 50 g of silica gel using 5 % (v/v) of ethanol in methylene chloride for elution. Crystallization of the combined clean fractions from ethyl acetate gave colorless crystals of 1-{3-[6-(2-fluorophenyl)-1-methyl-4H-[1,2,4]triazolo[4,3-a][1,4]benzodiazepine-8-yl]-2-propynyl}-3,4-dihydro-2(1H)-quinolinone with m.p. 236-239°.

Example 69

6-(2-Fluorophenyl)-8-iodo-1-methyl-4H-[1,2,4]triazolo[4,3-a][1,4]benzodiazepine was reacted with 4-phenyl-1-butyne under the conditions used in Example 3b. The product was isolated by chromatography over the 40-fold amount of silica gel using 5 % (v/v) of ethanol in methylene chloride for elution. The combined clean fractions were evaporated and the residue was crystallized from ethyl acetate with dry-ice cooling and was recrystallized from ethyl acetate/ether/hexane to give colorless crystals of 6-(2-fluorophenyl)-1-methyl-8-(4-phenyl-1-butynyl)-4H-[1,2,4]triazolo[4,3-a][1,4]benzodiazepine with m.p. 125-128°.

Example 70

a) A mixture of 5 g of 5-(2-fluorophenyl)-2-hydrazino-7-iodo-3H-1,4-benzodiazepine, 20 ml of trifluoroacetic acid, 5 ml of trifluoroacetic anhydride and 100 ml of methylene chloride was heated on the steam bath under a stream of nitrogen to distill off the methylene chloride. Toluene, 100 ml, was then added and heating on the steam bath was continued for 30 minutes. The mixture was partitioned between methylene chloride and saturated aqueous sodium bicarbonate solution. The organic phase was separated, dried and evaporated. The residue was crystallized from ether and recrystallized from ethanol to yield colorless crystals of 6-(2-fluorophenyl)-1-(trifluoromethyl)-8-iodo-4H-[1,2,4]triazolo[4,3-a][1,4]-benzodiazepine with m.p. 202-204°.

b) A mixture of 0.94 g (2 mmol) of 6-(2-fluorophenyl)-8-iodo-1-(trifluoromethyl)-4H-[1,2,4]triazolo [4,3-a]-[1,4]benzodiazepine, 0.55 g (3 mmol) of N-propargylphthalimide, 80 mg of triphenylphosphine, 20 mg of cuprous iodide, 0.6 ml of triethylamine and 50 ml of dimethylformamide was degassed with argon for 10 minutes. Palladium acetate, 25 mg, was then added and the mixture was stirred for 3 days at room temperature. The product was precipitated by dilution with sodium bicarbonate solution and was collected by filtration. It was dissolved in methylene chloride and the solution was washed with bicarbonate solution, dried and evaporated. The residue was chromatographed over 40 g of silica gel using 5 % (v/v) of ethanol in methylene chloride. The combined good fractions were evaporated and the residue was crystallized from ethyl acetate to give colorless crystals of 2-{3-[6-(2-fluorophenyl)-1-(trifluoromethyl)-4H-[1,2,4]triazolo[4,3-a][1,4]benzodiazepin-8-yl]-2-propynyl}-1H-isoindole-1,3(2H)-dione with m.p. 204-206°.

Example 71

a) A solution of 2.9 g (13 mmol) of N-benzyloxycarbonyl-L-alanine in 15 ml of tetrahydrofuran was cooled to -40°. Phophorus pentachloride, 2.7 g (13 mmol), was added and the mixture was stirred for 30 minutes at -30°. A solution of 3.41 g (10 mmol) of 2-(2-fluorobenzoyl)-4-iodoaniline in 50 ml of methylene chloride was then added and stirring was continued for 15 minutes at 0-10°. After addition of 10% aqueous sodium carbonate solution, the two-phase mixture was stirred at this temperature for 30 minutes. It was then extracted with ether. The extracts were washed with sodium carbonate solution and water, were dried and evaporated. The residue was passed over a plug of silica gel with methylene chloride. The filtrate was evaporated and the residue was crystallized from ethanol to give colorless crystals of (S)-{2-[2-(2-fluorobenzoyl-4-iodophenyl]amino}-1-methyl-2-oxoethylcarbamic acid phenylmethyl ester with m.p. 159-161°; $[\alpha]_D$ = -10.35° (c = 0.985 in $CH_2Cl_2$).

b) A mixture of 11 g of (S)-{2-[2-(2-fluorobenzoyl)-4-iodophenyl]amino}-1-methyl-2-oxoethyl-carbamic acid phenylmethyl ester and 30 ml of acetic acid containing 30 % of hydrogen bromide was stirred at

room temperature for 3 hours. The reaction mixture was partitioned between water and ether. The aqueous phase was washed with ether and made alkaline by addition of ice and ammonia. The precipitated material was extracted with methylene chloride and the extracts were dried and evaporated. The residue was heated in 50 ml of ethanol containing 5 ml of acetic acid on the steam bath for 15 minutes. The solvent was evaporated under reduced pressure and the residue was partitioned between methylene chloride and 10 % aqueous sodium carbonate solution. The organic phase was dried and evaporated and the residue was crystallised from ethyl acetate and recrystallized from methylene chloride/ethyl acetate to yield colorless crystals of (S)-5-(2-fluorophenyl)-1,3-dihydro-7-iodo-3-methyl-2H-1,4-benzodiazepin-2-one with m.p. 223-225°; $[\alpha]_D$ = +100.79° (c = 0.9891 in $CH_2Cl_2$).

c) A solution of 2 g (5.07 mmol) of (S)-5-(2-fluorophenyl)-1,3-dihydro-7-iodo-3-methyl-2H-1,4-benzodiazepin-2-one in 60 ml of tetrahydrofuran was cooled to -30° and 0.57 g (5.7 mmol) of potassium tert.-butoxide was added. The mixture was stirred under nitrogen for 30 minutes while the temperature was allowed to climb to 5°. Diethyl chlorophosphate, 1.03 g (6.5 mmol), was added and stirring was continued for 30 minutes without cooling. After addition of 0.54 g (7.2 mmol) of acetyl hydrazide the mixture was stirred for another 30 minutes at room temperature. Butanol, 75 ml, was then added and the tetrahydrofuran was distilled out. A few drops of acetic acid were added and part of the butanol was distilled over as well. The reaction mixture was evaporated under reduced pressure and the residue was partitioned between methylene chloride and saturated aqueous sodium bicarbonate solution. The organic layer was separated, dried and evaporated and the residue was chromatographed over silica gel using 5 % (v/v) of ethanol in methylene chloride for elution. Crystallization of the material obtained from the combined clean fractions from ethyl acetate/hexane gave colorless crystals of (S)-6-(2-fluorophenyl)-8-iodo-1,4-dimethyl-4H-[1,2,4]triazolo[4,3-a][1,4]benzodiazepine with m.p. 142-145°; $[\alpha]_D$ = +50.38° (c = 0.9964 in $CH_2Cl_2$)

d) (S)-6-(2-Fluorophenyl)-8-iodo-1,4-dimethyl-4H-[1,2,4]triazolo[4,3,a][1,4]benzodiazepine was reacted with 3,4-dihydro-1-(2-propynyl)-2(1H)-quinolinone under the conditions used in Example 68. The product was isolated by chromatography over the 50-fold amount of silica gel using 5 % (v/v) of ethanol in methylene chloride for elution. The combined clean fractions were evaporated and the residue crystallized very slowly from ethanol/ether to give colorless crystals of (S)-1-{3-[6-(2-fluorophenyl)-1,4-dimethyl-4H-[1,2,4]triazolo[4,3-a][1,4]benzodiazepin-8-yl]-2-propynyl}-3,4-dihydro-2(1H)-quinolinone with m.p. 155-160° with foaming. These crystals contained both ethanol and water according to analytical and spectral data. $[\alpha]_D$ = +87.5° (c = 1.0091 in $CH_2Cl_2$).

Example 72

a) A solution of 29 g of N-benzyloxycarbonyl-L-alanine in 100 ml of tetrahydrofuran was cooled to -40°. Phosphorus pentachloride, 27 g, was added and stirring was continued for 30 minutes at -30°. A solution of 23.7 g (0.1 mol) of 2-amino-3-(2-chlorobenzoyl)thiophene in 400 ml of methylene chloride was added and the mixture was stirred for 15 minutes at 0-10°. It was layered with 300 ml of 10 % aqueous sodium carbonate solution and the two-phase mixture was stirred at 0-10°o for 30 minutes. After dilution with ether, the organic layer was separated, washed with sodium carbonate solution, dried and evaporated. The residue was crystallized from ethanol with seeding. Seeds were obtained by chromatographing a 2 g sample over 60 g of silica gel using 10 % (v/v) ethyl acetate in methylene chloride for elution. The clean fractions yielded crystals from ether/hexane which were recrystallized from ethanol to give colorless crystals of (S)-{2-[3-(2-chloro-benzoyl)-2-thienyl]amino}-1-methyl-2-oxoethylcarbamic acid phenylmethyl ester with m.p. 133-135°; $[\alpha]_D$ = -26.92° (c = 0.9546 in $CH_2Cl_2$).

b) A solution of 22 g of (S)-{2-[3-(2-chlorobenzoyl)-2-thienyl]amino}-1-methyl-2-oxoethylcarbamic acid phenylmethyl ester in75 ml of acetic acid containing 30 % of hydrogen bromide was allowed to sit at room temperature for 3 hours. The reaction mixture was partitioned between water and ether. The aqueous phase was washed with ether and was made basic by addition of ice and ammonium hydroxide. The precipitated material was extracted with methylene chloride. The extracts were dried and evaporated and the residue was dissolved in 500 ml of toluene. After addition of 66 g of silica gel, the mixture was stirred and heated to reflux for 3 hours with separation of the water formed. The silica gel was filtered off and washed well with methanol. The filtrate was evaporated and the residue was passed over 200 g of silica gel using ethyl acetate/methylene chloride 1 : 1 for elution. The product crystallized from methylene chloride/hexane and was recrystallized from ether for analysis to yield (S)-5-(2-chlorophenyl)-1,3-dihydro-3-methyl-2H-thieno[2,3-e] [1,4]diazepin-2-one with m.p. 200-203°; $[\alpha]_D$ = -0.4° (c = 1.0185 in $CH_2Cl_2$).

c) A mixture of 7.7 g (26.5 mmol) of (S)-5-(2-chlorophenyl)-1,3-dihydro-3-methyl-2H-thieno[2,3-e][1,4]-diazepin-2-one, 60 ml of methanol, 60 ml of acetic acid, 8.61 g (53 mmol) of iodine monochloride and 2.17 g (26.5 mmol) of sodium acetate was stirred at ambient temperature for 15 minutes. A solution of 9 g of sodium bisulfite in water was added to reduce the excess reagent. The reaction mixture was made alkaline by addition of ice and ammonium hydroxide. The precipitated material was collected, washed with water and sucked dry. It was recrystallized from methanol/ethyl acetate to give colorless crystals of (S)-5-(2-chlorophenyl)-1,3-dihydro-7-iodo-3-methyl-2H-thieno[2,3-e][1,4]diazepin-2-one with m.p. 235-237°.

d) A mixture of 2.08 g (5 mmol) of (S)-5-(2-chlorophenyl)-1,3-dihydro-7-iodo-3-methyl-2H-thieno-[2,3-e]-[1,4]diazepin-2-one, 1,25 g of phosphorus pentasulfide, 1.3 g of sodium bicarbonate and 40 ml of diglyme was stirred under nitrogen at 80-90° for 6 hours. Water and ice were added and the mixture was stirred for 15 minutes. The solids were filtered off, washed with water and sucked dry. The resulting thione was further dried under vacuum at 50° to leave 2.6 g of crude material which was further reacted as follows:

The crude thione was stirred with 1.3 ml of anhydrous hydrazine in 30 ml of tetrahydrofuran for 30 minutes at room temperature. The solvent was evaporated under reduced pressure and the residue was stirred with 15 ml of water and 15 ml of methylene chloride. The crystalline hydrazine derivative was collected by filtration, washed with water and ether and was added to 13 ml of ethyl acetate and 6.5 ml of triethylorthoacetate. This mixture was heated on the steam bath for 30 minutes and the crystals formed were filtered off after cooling. The product was recrystallized from methylene chloride/methanol to give colorless crystals of rac-4-(2-chlorophenyl)-2-iodo-6,9-dimethyl-6H-thieno[3,2-f][1,2,4]triazolo[4,3-a][1,4]-diazepine with m.p. 262-264°. These crystals had no rotation, indicating total racemization had occurred.

e) rac-4-(2-Chlorophenyl)-2-iodo-6,9-dimethyl-6H-thieno-[3,2-f][1,2,4]triazolo[4,3-a][1,4]diazepine was reacted with 5-(2-propynyl)-6(5H)-phenanthridinone under the conditions described in example 25d. The product was isolated by chromatography over the 50-fold amount of silica gel using 5% (v/v) of ethanol in methylene chloride. The product was crystallized from ethanol to give yellowish crystals of rac-5-{3-[4-(2-Chlorophenyl)-6,9-dimethyl-6H-thieno[3,2-f][1,2,4]triazolo[4,3-a][1,4]diazepin-2-yl]-2-propynyl}-6(5H)-phenanthridinone with m.p. 182-186° with foaming. These crystals contained according to analytical data molar amounts of water.

Example 73

A mixture of 1.1 g (2 mmol) of 5-{3-[4-(2-chlorophenyl)-9-methyl-6H-thieno-[3,2-f][1,2,4]triazolo[4,3-a]-[1,4]diazepin-2-yl]-2-propynyl}-6(5H)-phenanthridinone and 0.68 g of m-chloroperoxybenzoic acid in 100 ml of methylene chloride was allowed to sit at room temperature for 20 hours. The solution was washed with 10 % aqueous sodium carbonate solution, was dried and evaporated. The residue was crystallized from methanol/ethyl acetate to give off-whie crystals of 5-{3-[4-(2-chlorophenyl)-9-methyl-6H-thieno[3,2-f][1,2,4]-triazolo[4,3-a][1,4]diazepin-5-oxide-2-yl]-2-propynyl}-6(5H)-phenanthridinone with m.p. 260-270° with decomposition.

Example 74

A mixture of 0.7 g of 5-{3-[4-(2-chlorophenyl)-9-methyl-6H-thieno[3,2-f][1,2,4]triazolo[4,3-a][1,4]diazepin-5-oxide-2-yl]-2-propynyl}-6(5H)-phenanthridinone, 25 ml of acetic anhydride and 30 ml of pyridine was heated on the steam bath for 3 hours under argon. The reagents were removed under reduced pressure and the residue was chromatographed over 30 g of silica gel using 3 % (v/v) of ethanol in methylene chloride for elution. The clean fractions containing 5-{3-[6-acetyloxy-4-(2-chlorophenyl)-9-methyl-6H-thieno-[3,2-f][1,2,4]triazolo[4,3-a][1,4]diazepin-2-yl]-2-propynyl}-6(5H)-phenanthridinone were combined and evaporated. The residue did not crystallize and was characterized spectroscopically only. NMR ($CDCl_3$): 2.36 (s, 3, OAc), 2.68 (s, 3, 9-Me), 5.43 (s,2, $-CH_2-$), 6.78 (s, 2, C6-H and thienyl-H), 7.2-8.6 (m, 12, aromatic H) ppm.

Example 75

A solution of 0.25 g of 5{3-[6-acetyloxy-4-(2-chlorophenyl)-9-methyl-6H-thieno[3,2-f][1,2,4]triazolo[4,3-a][1,4]diazepin-2-yl]-2-propynyl}-6(5H)-phenanthridinone in 30 ml of warm methanol was treated with 2ml of 3N sodium hydroxide solution. After 15 minutes, the reaction mixture was acidified with acetic acid and was

partitioned between methylene chloride and aqueous sodium bicarbonate solution. The organic phase was separated, dried and evaporated. The residue was chromatographed over 25 g of silica gel (Merck 230-400 mesh) using 5 % (v/v) of ethanol in methylene chloride for elution. After elution of some rearranged rac-4-(2-chlorophenyl)-4,5-dihydro-9-methyl-2-[3-(5,6-dihydro-6-oxo-5-phenanthridinyl)-1-propynyl]-6H-thieno[3,2-f][1,2,4]triazolo[4,3-a][1,4]diazepin-6-one with m.p. 225-230° (from methanol/ethyl acetate), the fractions containing rac-5-{3-[4(2-chlorophenyl)-6-hydroxy-9-methyl-6H-thieno-[3,2,-f][1,2,4]triazolo[4,3-a][1,4]-diazepin-2-yl]-2-propynyl}-6(5H)-phenanthridinone were combined and evaporated. The residue was crystallized from methanol/ethyl acetate to give colorless crystals with m.p. 255-258°.

Example 76

6-(2-Fluorophenyl)-8-iodo-1-methyl-4H-[1,2,4]triazolo[4,3-a][1,4]benzodiazepine was reacted with rac-2-ethynyl-3,4-dihydro-2,5,7,8-tetramethyl-2H-1-benzopyran-6-ol [ref. H. Mayer et al., Helv. Chim. Acta, 67, 650 (1963)] under the conditions described in Example 25d. The product was isolated by chromatography over the 50-fold amount of silica gel using 5 % (v/v) of ethanol in methylene chloride. The clean fractions containing product were combined and evaporated. The residue was crystallized from ethanol/ethyl acetate to give colorless crystals of rac-2-{[6-(2-fluorophenyl)-1-methyl-4H-[1,2,4]triazolo[4,3-a][1,4]benzodiazepin-8-yl]ethynyl}-3,4-dihydro-2,5,7,8-tetramethyl-2H-1-benzopyran-6-ol with m.p. 265-267°.

Example 77

4-(2-Chlorophenyl)-2-iodo-9-methyl-6H-thieno-[3,2-f][1,2,4]triazolo[4,3-a][1,4]diazepine was reacted with rac-2-ethynyl-3,4-dihydro-2,5,7,8-tetramethyl-2H-1-benzopyran-6-ol under the conditions described in Example 25d. The product was isolated by chromatography over the 50-fold amount of silica gel using 5 % (v/v) of ethanol in methylene chloride. The combined clean fractions were evaporated and the residue was crystallized from ethyl acetate to give colorless crystals of rac-2-{[4-(2-chlorophenyl)-9-methyl-6H-thieno-[3,2-f][1,2,4]triazolo[4,3-a][1,4]diazepin-2-yl]ethynyl}-3,4-dihydro-2,5,7,8-tetramethyl-2H-1-benzopyran-6-ol with m.p. 155-160° with foaming. These crystals contained 1.5 molar amounts of water on the basis of the analytical and spectral data.

Example 78

a) A solution of 0.8 g of potassium tert.-butoxide in 20 ml of tetrahydrofuran and 15 ml of tert. butanol and 0.6 ml of triethylphosphite was cooled to -30° with stirring under argon. A solution of 0.8 g of 6-(2-fluorophenyl)-8-iodo-1-methyl-4H-[1,2,4]triazolo[4,3-a][1,4]benzodiazepine in 5 ml of dimethylformamide was added and stirring was continued for 1 hour at -20° to -10°. A stream of oxygen was introduced while the mixture was stirred for an additional hour at this temperature. The reaction mixture was acidified by addition of acetic acid and was partitioned between sodium carbonate solution and methylene chloride containing 10 % (v/v) of ethanol. The organic layer was dried and evaporated and the residue was crystallized from methylene chloride/ethyl acetate and recrystallized from ethanol to give colorless crystals of rac-6-(2-fluorophenyl)-4-hydroxy-8-iodo-1-methyl-4H-[1,2,4]triazolo[4,3-a][1,4]-benzodiazepine with m.p. 258-260°.

b) A mixture of 0.435 g (1 mmol) of rac-6-(2-fluorophenyl)-4-hydroxy-8-iodo-1-methyl-4H-[1,2,4]triazolo-[4,3-a][1,4]benzodiazepine, 3 ml of thionyl chloride and 20 ml of methylene chloride was stirred at room temperature for 2 hours. After evaporation under reduced pressure, the residue was dissolved in 20 ml of methanol and the solution was treated with 3 ml of triethylamine. After heating on the steam bath for 5 minutes, the mixture was evaporated to dryness and the residue was partitioned between methylene chloride and aqueous sodium bicarbonate solution. The organic layer was dried and evaporated and the residue was crystallized from ethyl acetate to yield colorless crystals of rac-6-(2-fluorophenyl)-8-iodo-4-methoxy-1-methyl-4H-[1,2,4]triazolo[4,3-a][1,4]benzodiazepine with m.p. 240-242°. The analytical sample was recrystallized from methanol/ethyl acetate and had m.p. 243-244°.

c) rac-6-(2-Fluorophenyl)-8-iodo-4-methoxy-1-methyl-4H-[1,2,4]triazolo[4,3-a][1,4]benzodiazepine was reacted with 3,4-dihydro-1-(2-propynyl)-2(1H)-quinolinone under the conditions used in Example 25d. The product was isolated by chromatography over the 50-fold amount of silica gel using 5 % (v/v) of ethanol in methylene chloride for elution. The combined clean fractions were evaporated and the residue was crystallized from ethyl acetate/ether to give colorless crystals of rac-1-{3-[6-(2-fluorophenyl)-4-methoxy-1-methyl-4H-[1,2,4]triazolo[4,3-a][1,4]benzodiazepin-8-yl]-2-propynyl}-3,4-dihydro-2(1H)-quinolinone with m.p. 155-160°. These crystals contained 0.33 molar equivalents of water.

Example 79

6-(2-Fluorophenyl)-1-(trifluoromethyl)-8-iodo-4H-[1,2,4]triazolo [4,3-a][1,4]benzodiazepine was reacted with 3,4-dihydro-1-(2-propynyl)-2(1H)-quinolinone under the conditions used in Example 25d. The product was purified by chromatography over the 50-fold amount of silica gel using 5 % (v/v) of ethanol in methylene chloride. The clean fractions were combined and evaporated and the residue was crystallized from ethyl acetate to yield colorless crystals of 1-{3-[6-(2-fluorophenyl)-1-(trifluoromethyl)-4H-[1,2,4]triazolo-[4,3-a][1,4]benzodiazepin-8-yl]-2-pro pynyl}- 3,4-dihydro-2(1H)-quinolinone with m.p. 193-196°.

Example 80

rac-6-(2-Fluorophenyl)-4-hydroxy-8-iodo-1-methyl-4H-[1,2,4] triazolo[4,3-a][1,4]benzodiazepine was reacted with 3,4-dihydro-1-(2-propynyl)-2(1H)-quinolinone under the conditions used in Example 25d. The product was isolated by chromatography over the 50-fold amount of silica gel using 5 % (v/v) of ethanol in methylene chloride. The combined good fractions were evaporated and the residue was crystallized from methanol/ethyl acetate to give colorless crystals of rac-1-{3-[6-(2-fluorophenyl)-4-hydroxy-1-methyl-4H-[1,2,4]-triazolo[4,3-a][1,4]benzodiazepin-8-yl]-2-propynyl}-3,4-dihydro-2(1H)-quinolinone with m.p. 253-255° with decomposition. These crystals contained molar equivalents of water.

Example 81

4-(2-Chlorophenyl)-2-iodo-9-methyl-6H-thieno[3,2-f][1,2,4] triazolo[4,3-a][1,4]diazepine was reacted with 1′-(2-propynyl)spiro [cyclopentane-1,3′-[3H]indol]-2′-(1′H)-one under the conditions described in Example 25d. The product was isolated in chromatography over the 50-fold amount of silica gelusing 5 % (v/v) of ethanol in methylene chloride for elution. The combined good fractions were evaporated and the residue was converted to a crystalline dihydrochloride by treatment with excess ethanolic hydrogen chloride in ethanol/ethyl acetate. The light yellow crystals of 1-{3-[4-(2-chlorophenyl)-9-methyl-6H-thieno[3,2-f][1,2,4]-triazolo[4,3-a][1,4]diazepin-2-yl]-2-propynyl}spiro[cyclopentane-1,3′-[3H]indol]-2(1′H)-one dihydrochloride had m.p. 173-176° with foaming and contained two molar equivalents of water and 0.66 molar equivalents of ethanol according to the analytical and spectral data.

The starting propargyl derivative used in this experiment was prepared as follows:

Potassium tert.-butoxide, 0.37 g (3.3 mmol), was added to a solution of 0.56 g (3 mmol) of spiro-[cyclopentane-1,3′-[3H]indol]-2′-(1′H)-one [ref. R. J. Owellen, J. Org. Chem. 39, 69 (1974)] in 10 ml of dimethylformamide. The mixture was stirred for 15 minutes and 0.3 ml of propargyl bromide was added and stirring was continued for 30 minutes. The reaction mixture was then partitioned between xylene and saturated sodium bicarbonate solution. The organic phase was dried and evaporated and the residue was chromatographed over 20 g of silica gel using methylene chloride for elution. The combined clean fractions were evaporated and the residue was crystallized from ether/hexane to give colorless crystals of 1′-(2-propynyl)spiro[cyclopentane-1,3′-[3H]indol]-2′-(1′H)-one with m.p. 109-111°.

Examle 82

A mixture of 0.5 g (1 mmol) of 1-{3-[4-(2-chlorophenyl)-9-methyl-6H-thieno[3,2-f][1,2,4]triazolo[4,3-a]-[1,4]diazepin-2-yl]-2-propynyl}-3,4-dihydro-2(1H)-quinolinone and 0.34 g (2 mmol) of m-chloroperoxybenzoic acid in 30 ml of methylene chloride was allowed to sit at room temperature over night. It was then washed with 10% aqueous sodium carbonate solution, dried and evaporated. The residue was purified by chromatography over 20 g of silica gel (230-400 mesh) using 5% (v/v) of ethanol in methylene chloride. The combined clean fractions were evaporated and the residue was crystallized from ethyl acetate and recrystallized from methanol/ethyl acetate to give colorless crystals of 1-{3-[4-(2-chlorophenyl)-9-methyl-6H-thieno[3,2-f][1,2,4]triazolo[4,3-a][1,4]diazepin-5-oxide-2-yl]2-propynyl}-3,4-dihydro-2(1H)-quinolinone with m.p. 225-230° with decomposition. These crystals contained according to spectral and analytical data 0.166 molar amounts of ethyl acetate.

Example 83

a) Meta-chloroperoxybenzoic acid, 3.4g, was added to a solution of 4.4g of 4-(2-chlorophenyl)-2-iodo-9-methyl-6H-thieno[3,2-f][1,2,4]triazolo[4,3-a][1,4]diazepine in 200 ml of methylene chloride. After sitting at room temperature in the dark for 18 hours, it was washed with 10% aqueous sodium carbonate solution.

The organic phase was dried over sodium sulfate, filtered and evaporated. The residue was crystallized from tetrahydrofuran/methanol/ethyl acetate to give 3.3g of crude product. It was purified by passing over a plug of silica gel using 10% (v/v) of methanol in methylene chloride. The eluate was evaporated and the residue was crystallized from tetrahydrofuran/methanol to give off-white crystals of 4-(2-chlorophenyl)-2-iodo-9-methyl-6H-thieno[3,2-f][1,2,4]triazolo[4,3-a][1,4]diazepine-5-oxide with m.p. 280-283° with decomposition.

b) The product of example 73 was prepared by coupling 4-(2-chlorophenyl)-2-iodo-9-methyl-6H-thieno-[3,2-f][1,2,4]triazolo[4,3-a][1,4]diazepine-5-oxide with 5-(2-propynyl)-6(5H)-phenanthridinone using the conditions described in Example 25d.

Example 84

a) A mixture of 1g of 4-(2-chlorophenyl)-2-iodo-9-methyl-6H-thieno[3,2-f][1,2,4]triazolo[4,3-a][1,4]-diazepine-5-oxide, 25 ml of pyridine and 15 ml of acetic anhydride was heated on the steam bath for 4 hours. The mixture was evaporated under reduced pressure, at the end azeotropically with xylene. The residue was chromatographed over 25g of silica gel using 5% (v/v) of ethanol in methylene chloride for elution. The combined clean fractions were evaporated and the product was crystallized from ethyl acetate and recrystallized from tetrahydrofuran/methanol/ethyl acetate to give colorless crystals of rac-6-acetyloxy-4-(2-chlorophenyl)-2-iodo-9-methyl-6H-thieno[3,2-f][1,2,4]triazolo[4,3-a][1,4]diazepine with m.p. 248-250°.

b) 1 ml of 3N-Sodium hydroxide and 10 ml of water was added to a solution of 0.3 g of rac-6-acetyloxy-4-(2-chlorophenyl)-2-iodo-9-methyl-6H-thieno[3,2-f][1,2,4]triazolo[4,3-a][1,4]diazepine in 30 ml of methanol. After standing at room temperature for 30 minutes, the solution was acidified with acetic acid and partitioned between methylene chloride and aqueous sodium bicarbonate solution. The organic phase was separated, dried and evaporated. The residue was crystallized from methanol/ ethyl acetate to give colorless crystals of rac-4-(2-chlorophenyl)-2-iodo-9-methyl-6H-thieno[3,2-f][1,2,4]traizolo[4,3-a][1,4]-diazepine-6-ol with m.p. 240-243° dec.

c) The product of example 75 was prepared by reacting 4-(2-chlorophenyl)-2-iodo-9-methyl-6H-thieno-[3,2-f][1,2,4]-triazolo[4,3-a][1,4]diazepine-6-ol with 5-(2-propynyl)-6(5H)-phenanthridinone under the conditions described in Example 25d.

Example 85

a) A solution of 23 g of (2-aminophenyl)(4-chlorophenyl)methanone in 500 ml of methylene chloride was cooled to -50°. Iodine monochloride, 21 g or 15 ml, was added and the mixture was stirred in the cold for 4 hours. It was then allowed to warm to 0° and was quenched with a solution of sodium bisulfite in water. After stirring for 10 minutes, the organic layer was separated, dried and evaporated. The product was crystallized from toluene/hexane to give yellow crystals of (2-amino-5-iodophenyl)(4-chlorophenyl)-methanone with m.p. 137-139°.

b) Bromoacetyl bromide, 5 ml, was added to a solution of 18g of (2-amino-5-iodophenyl)(4-chlorophenyl)-methanone in 250 ml of methylene chloride and stirred with crushed ice for 15 minutes. The organic layer was separated, washed with sodium bicarbonate solution, dried and evaporated. The residue was crystallized from methylene chloride/ether to give 18g of bromoacetyl derivative. This material was dissolved in 200 ml of methylene chloride and added to 300 ml of liquid ammonia. The ammonia was allowed to gradually evaporate over night and the remaining methylene chloride was washed with water, dried and evaporated. The residue was dissolved in 300 ml of ethanol and heated to reflux for 30 minutes after the addition of 10 ml of acetic acid. The solvent was partially evaporated and the product was crystallized by cooling. Recrystallization from methylene chloride/ethanol gave colorless crystals of 5-(4-chlorophenyl)-1,3-dihydro-7-iodo-2H-1,4-benzodiazepin-2-one with m.p. 246-248°.

c) A mixture of 12g of 5-(4-chlorophenyl)-1,3-dihydro-7-iodo-2H-1,4-benzodiazepin-2-one, 8 g of phosphorus pantasulfide, 8g of sodium bicarbonate and 100 ml of diglyme was stirred and heated to 80-85° for 3 hours. Water and crushed ice was added after cooling and stirring was continued for 10 minutes. The precipitated product was collected by filtration and washed with water, 2-propanol and ether. For analysis it was recrystallized from tetrahydrofuran/ethanol to yield the 5-(4-chlorophenyl)-1,3-dihydro-7-iodo-2H-1,4-benzodiazepine-2-thione which melted at 260-262°.

d) A mixture of 4g of 5-(4-chlorophenyl)-1,3-dihydro-7-iodo-2H-1,4-benzodiazepine-2-thione, 50 ml of tetrahydrofuran, 20 ml of 2-propanol and 1.5 ml of hydrazine was stirred at room temperature for 30 minutes. This mixture was filtered over a plug of 10 g of silica gel using tetrahydrofuran for elution. The

filtrate was evaporated and the residue was crystallized from ether. Recrystallization of this material from tetrahydrofuran/ethanol gave colorless crystals of 5-(4-chlorophenyl)-2-hydrazino-7-iodo-3H-1,4-benzodiazepine with m.p. 250-252°.

e) A mixture of 2.8 g of 5-(4-chlorophenyl)-2-hydrazino-7-iodo-3H-1,4-benzodiazepine, 40 ml of xylene and 10 ml of triethyl orthoacetate was heated to reflux for 1.5 hours. The crystals which separated from the cooled reaction mixture were filtered off and recrystallized from tetrahydrofuran/ethanol to give colorless crystals of 6-(4-chlorophenyl)-8-iodo-1-methyl-4H-[1,2,4]triazolo[4,3-a][1,4]benzodiazepine with m.p. 358-360°.

f) 6-(4-Chlorophenyl)-8-iodo-1-methyl-4H-[1,2,4]triazolo[4,3-a][1,4]benzodiazepine was reacted with 3,4-dihydro-1-(2-propynyl)-2(1H)-quinolinone under the conditions used in Example 25d. The product was isolated by chromatography over the 50-fold amount of silica gel using 20% (v/v) of hexane in tetrahydrofuran for elution. Crystallization of the combined clean fractions from ethyl acetate and recrystallization from the same solvent gave colorless crystals of 1-{3-[6-(4-chlorophenyl)-1-methyl-4H-[1,2,4]triazolo[4,3-a][1,4]benzodiazepin-8-yl]-2-propynyl}-3,4-dihydro-2(1H)-quinolinone with m.p. 215-217°. These crystals contained 0.33 molar amounts of water according to analytical data.

Example 86

a) A mixture of 3.4g of 5-(4-chlorophenyl)-2-hydrazino-7-iodo-3H,1,4-benzodiazepine, 75 ml of methylene chloride, 5 ml of trifluoroacetic anhydride and 15 ml of trifluoroacetic acid was heated under nitrogen on the steam bath to remove the methylene chloride within ca 30 minutes. Toluene, 100 ml, was then added and heating on the steam bath was continued for 30 minutes. The cooled mixture was washed with saturated sodium bicarbonate solution, was dried and evaporated. The residue was chromatographed over 200 g of silica gel using 10% (v/v) of ethyl acetate in methylene chloride for elution. The fractions containing the product were combined and evaporated. Crystallization from ethyl acetate gave colorless crystals of 6-(4-chlorophenyl)-1-trifluoromethyl-8-iodo-4H-[1,2,4]triazolo[4,3-a][1,4]benzodiazepine with m.p. 243-245°.

b) 6-(4-Chlorophenyl)-1-trifluoromethyl-8-iodo-4H-[1,2,4]triazolo[4,3-a][1,4]benzodiazepine was reacted with 3,4-dihydro-1-(2-propynyl)-2(1H)-quinolinone under the conditions used in Example 25d, but extending the reaction time to 48 hours. The product was isolated by chromatography over the 50-fold amount of silica gel using methylene chloride: ethyl acetate 1:1. The clean fractions containing the product were combined and evaporated and the residue was crystallized from ethyl acetate/hexane to give off-white crystals of 1-{3-[6-(4-chlorophenyl)-1-trifluoromethyl-4H-[1,2,4]triazolo-[4,3-a][1,4]benzodiazepin-8-yl]-2-propynyl}-3,4-dihydro-2(1H)-quinolinone with m.p. 220-222°. These crystals contained 0.5 molar amounts of water according to analytical data.

Example A

| Suspension (oral) | |
|---|---|
| 2-[3-[4-(2-chlorophenyl)-9-methyl 6H-thieno[3,2-f][1,2,4]-triazolo[4,3-a][1,4]diazepin-2-yl]-2-propynyl]-1H-benz[de]isoquinoline-1,3(2H)-dione (microfine) (Compound A) | 5.0 gm |
| Hydroxypropylmethyl cellulose | 8.0 gm |
| Polysorbate 80 | 0.5 gm |
| Distilled Water q.s. and | 100.0 ml |

Procedure

1. Add Compound A to a solution of polysorbate 80 and disperse.
2. In distilled water make a solution of hydroxypropylmethyl cellulose.
3. Mix the materials from step 1 and 2, then add distilled water to bring to 100 ml.

Example B

| Capsule Formulation | |
|---|---|
| | mg/capsule |
| 2-[3-[4-(2-chlorophenyl)-9-methyl 6H-thieno[3,2-f][1,2,4]-triazolo[4,3-a][1,4]diazepin-2-yl]-2-propynyl]-1H-benz[de]isoquinoline-1,3(2H)-dione (microfine) (Compound A) | 50.00 |
| Polyvinylpyrrolidone K-90 | 0.50 |
| Polysorbate 80 | 0.25 |
| Microcrystalline Cellulose | 99.00 |
| Distilled Water | q.s. |
| Magnesium Stearate | 0.25 |
| Total Weight | 150.00mg |

Procedure

1. To Compound A add a sufficient amount of an aqueous solution of Polyvinylpyrrolidone K-90 and Polysorbate 80.
2. Granulate to consistency, dry and screen through a 40 mesh sieve.
3. Add the microcrystalline cellulose and Magnesium Stearate, then blend and fill into capsules.

Example C

| Tablet Formulation | |
|---|---|
| | mg/tablet |
| 2-[3-[4-(2-chlorophenyl)-9-methyl 6H-thieno[3,2-f][1,2,4]-triazolo[4,3-a][1,4]diazepin-2-yl]-2-propynyl]-1H-benz[de]isoquinoline-1,3(2H)-dione (microfine) (Compound A) | 50.00 |
| Polyvinylpyrrolidone (fine) | 14.25 |
| Polyvinylpyrrolidone K-90 | 0.50 |
| Microcrystalline Cellulose | 35.00 |
| Sodium Starch Glycolate | 10.00 |
| Distilled Water | q.s. |
| Magnesium Stearate | 0.25 |
| Total Weight | 110.00 |

Procedure

1. Prepare a blend of Compound A, sodium starch glycolate, and polyvinylpyrrolidone, then add a sufficient amount of an aqueous solution of polyvinlypyrrolidone K-90.
2. Granulate to consistency, dry and screen trough a 40 mesh sieve.
3. Add microcrystalline cellulose and magnesium stearate, then blend and compress.

Example D

| Aerosol Suspension, 0.5 mg/actuation | |
|---|---|
| 2-[3-[4-(2-chlorophenyl)-9-methyl 6H-thieno[3,2-f][1,2,4]-triazolo[4,3-a][1,4]diazepin-2-yl]-2-propynyl]-1H-benz[de]isoquinoline-1,3(2H)-dione (microfine) (Compound A) | 120.00mg |
| Sorbitan trioleate | 40.0mg |
| Trichloromonofluoro methane | 1.80ml |
| Dichlorodifluoro methane | 10.20ml |

Procedure

1. To Compound A add a solution of sorbitan trioleate and trichloromonofluoro methane.
2. Homogenize and add the suspension to an aluminum container.
3. Crimp a 50 microliter metering valve to the container and pressure fill the dichlorodifluoro methane.

Example E

| Topical Solution 1% | |
|---|---|
| 2-[3-[4-(2-chlorophenyl)-9-methyl 6H-thieno[3,2-f][1,2,4]-triazolo [4,3-a][1,4]diazepin-2-yl]-2-propynyl]-1H-benz[de]isoquinoline-1,3(2H)-dione (microfine)(Compound A) | 1.0gm |
| Polyethylene glycol 400 | 99.0gm |

Procedure

1. Add compound A to the polyethyleneglycol 400 and mix well until dissolved.

**Claims**

**Claims for the following Contracting States : AT, BE, CH, LI, DE, FR, GB, IT, LU, NL, SE**

**1.** Compounds of the general formula

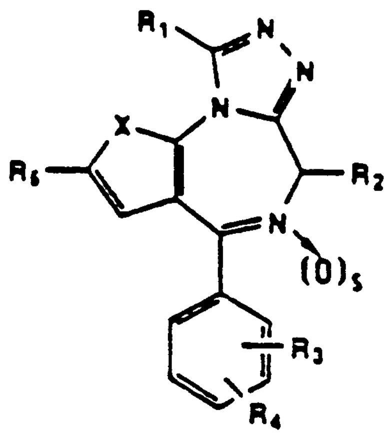

I

wherein X is -CH=CH- or S;
$R_1$ is lower alkyl, lower alkoxy or trifluoromethyl;
$R_2$ is hydrogen, lower alkyl, lower alkoxy, hydroxy or lower alkanoyloxy;
$R_3$ and $R_4$, independently, are hydrogen, chlorine, fluorine, lower alkyl or lower alkoxy;

$R_5$ is a radical of the formula $R_6$-$(CH_2)_n$C≡C-or $R_7$-O-$(CH_2)_m$-C≡C-,

$R_6$ and $R_7$ are aryl or a heterocyclic radical; n is an integer from 0 to 2; m is an integer from 1 to 2 and s is an integer from 0 to 1; wherein the term "lower alkyl", denotes a straight or branched chain saturated hydrocarbon group containing from 1 to 7 carbon atoms, the term "lower alkoxy" denotes an alkyl ether group in which the alkyl group is as described above and the term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted by lower alkyl, lower alkoxy, oxo, hydroxy, chlorine or fluorine; with the proviso that, when s is 1, $R_2$ cannot be hydroxy, lower alkoxy or lower alkanoyloxy; that, when n is 0, $R_6$ must be attached through a carbon to carbon bond, and that $R_7$ is always attached through a carbon to oxygen bond,
and, when at least one asymmetric centre is present, their enantiomers and racemates, and pharmaceutically acceptable acid addition salts thereof.

**2.** Compounds in accordance with claim 1, wherein X is -CH = CH- or S; $R_1$ is lower alkyl or lower alkoxy; $R_2$ is hydrogen, lower alkyl or lower alkoxy; $R_3$ and $R_4$, independently, are hydrogen, chlorine, fluorine, lower alkyl or lower alkoxy; $R_5$ is a radical of the formula $R_6$-$(CH_2)_n$-C≡C- or $R_7$-O-$(CH_2)_m$-C≡C-, $R_6$ and $R_7$ are aryl or a heterocyclic radical, n is an integer from 0 to 2; m is an integer from 1 to 2 and s is the integer 0; wherein the term "lower alkyl", denotes a straight or branched chain saturated hydrocarbon group containing from 1 to 7 carbon atoms, the term "lower alkoxy" denotes an alkyl ether group in which the alkyl group is as described above and the term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted by lower alkyl, lower alkoxy, oxo, hydroxy, chlorine or fluorine; with the proviso that, when n is 0, $R_6$ must be attached through a carbon to carbon bond and that $R_7$ is always attached through a carbon to oxygen bond.

**3.** Compounds in accordance with claim 1 or 2, wherein $R_1$, is methyl or ethyl, $R_2$ is hydrogen, $R_3$ is fluorine or chlorine, $R_4$ is hydrogen, s is 0 and n is 1 or 2.

**4.** Compounds in accordance with claim 1 or 2, wherein $R_1$ is methyl, $R_2$ is hydrogen, $R_3$ is fluorine or chlorine at the 2-position of the phenyl moiety, $R_4$ is hydrogen, s is 0, m and n are 1, $R_6$ is a bi- or tricyclic heterocyclic radical and $R_7$ is aryl.

**5.** Compounds in accordance with claim 1 or 2, wherein X is S, $R_1$ is methyl, $R_2$ is hydrogen, $R_3$ is chlorine and at the 2-position of the phenyl moiety, $R_4$ is hydrogen, s is O, $R_5$ is $R_6$-$(CH_2)_n$-C≡C-, n is 1 and $R_6$ is

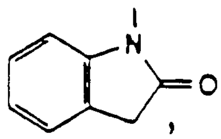,
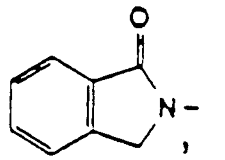,
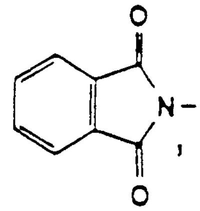,

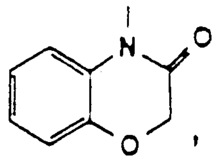,
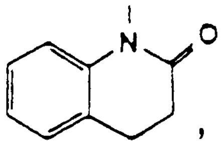,
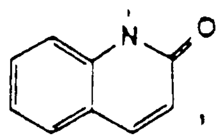,

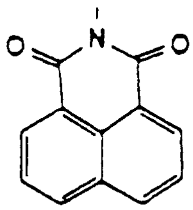,
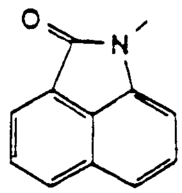,
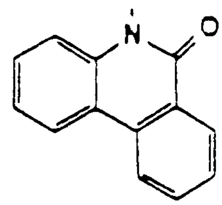 and

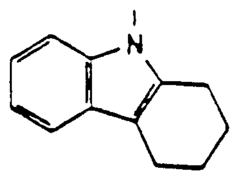

**6.** 5-{3-[4-(2-Chlorophenyl)-9-methyl-6H-thieno[3,2-f][1,2,4]triazolo[4,3-a][1,4]diazepin-2-yl]-2-propynyl}-phenanthridin-6(5H)-one.

**7.** 4-(2-Chlorophenyl)-2-[3-(1,2,3,4-tetrahydro-9H-carbazol-9-yl)-1-propynyl]-9-methyl-6H-thieno[3,2,f]-[1,2,4]triazolo[4,3-a] [1,4]diazepine.

**8.** 1-{3-[4-(2-Chlorophenyl)-9-methyl-6H-thieno[3,2-f][1,2,4] triazolo[4,3-a][1,4]diazepin-2-yl]-2-propynyl}-3,4-dinhydro-2(1H)-quinolinone.

**9.** 2-[3-[4-(2-Chlorophenyl)-9-methyl-6H-thieno[3,2-f][1,2,4]triazolo[4,3-a][1,4]diazepin-2-yl]-2-propynyl]-1H-benz[de]isoquinoline-1,3(2H)-dione.

**10.** 1-[3-[4-(2-Chlorophenyl)-9-methyl-6H-thieno[3,2-f][1,2,4] triazolo[4,3-a] [1,4]diazepin-2-yl]-2-propynyl]-benz[cd]indol-2(1H)-one.

**11.** 4-[3-[4-(2-Chlorophenyl)-9-methyl-6H-thieno[3,2-f][1,2,4] triazolo[4,3-a] [1,4]diazepin-2-yl]-2-propynyl]-2H-1,4-benzoxazin-3(4H)-one.

**12.** 1-[3-[4-(2-Chlorophenyl)-9-methyl-6H-thieno[3,2-f][1,2,4] triazolo[4,3-a] [1,4]diazepin-2-yl]-2-propynyl]-1H-indole-2,3-dione;
1-[3-[4-(2-chlorophenyl)-9-methyl-6H-thieno[3,2-f][1,2,4] triazolo[4,3-a] [1,4]diazepin-2-yl]-2-propynyl]-1,3-dinhydro-2H-indol-2-one;
2-[3-[4-(2-chlorophenyl)-9-methyl-6H-thieno[3,2-f][1,2,4] triazolo[4,3-a] [1,4]diazepin-2-yl]-2-propynyl]-1,2,4-triazolo[4,3-a]pyridin-3(2H)-one;
2-[3-[4-(2-chlorophenyl)-9-methyl-6H-thieno[3,2-f][1,2,4] triazolo[4,3-a] [1,4]diazepin-2-yl]-2-propynyl]-1,2-benzisothiazol-3(2H)-one 1,1-dioxide;
4-(2-chlorophenyl)-2-[3-(1H-indazol-1-yl)-1-propynyl]-9-methyl-6H-thieno[3,2-f][1,2,4]triazolo[4,3-a][1,4]-diazepine;
2-[3-(1H-benzimidazol-1-yl)-1-propynyl]-4-(2-chlorophenyl)-9-methyl-6H-thieno[3,2-f][1,2,4]triazolo[4,3-a][1,4]diazepine;
2-[3-[6-(2-fluorophenyl)-1-methyl-4H-[1,2,4]triazolo [4,3-a][1,4]benzodiazepin-8-yl]-2-propynyl]-1H-isoindole-1,3(2H)-dione and

4-[3-[6-(2-fluorophenyl)-1-methyl-4H-[1,2,4]triazolo [4,3-a][1,4]benzodiazepin-8-yl]-2-propynyl]-2H-1,4-benzoxazin-3(4H)-one.

**13.** Compounds of the general formulae

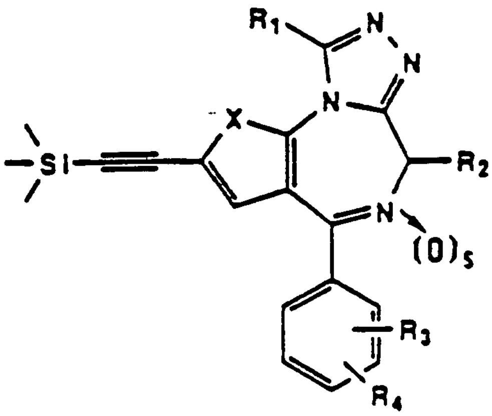

IV

and

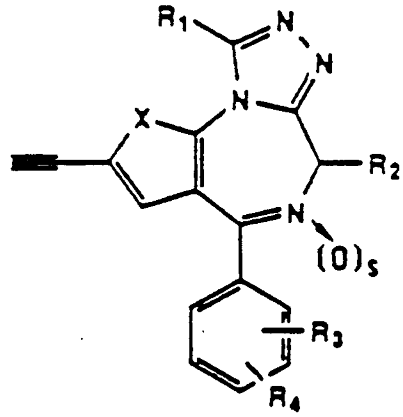

V

wherein X, $R_1$, $R_2$, $R_3$, $R_4$ and s are as defined in claim 1.

**14.** Compounds in accordance with any one of claims 1-12 for use as therapeutically active substances.

**15.** Compounds in accordance with any one of claims 1-12 for use as platelet activating factor (PAF) antagonists.

**16.** A process for the manufacture of compounds in accordance with any one of claims 1-12 and of pharmaceutically acceptable acid addition salts thereof, which process comprises

a) reacting a compound of the general formula

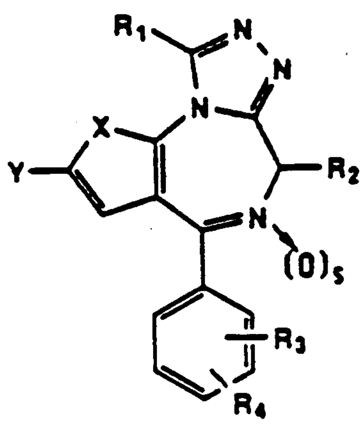

II

wherein X, $R_1$, $R_2$, $R_3$, $R_4$ and s are as defined in claim 1, and y is bromo or iodo, with a compound of the general formula

$R_6$-$(CH_2)_n$-C≡C-H (IIIa) or $R_7$-O-$(CH_2)_n$-C≡C-H (IIIb)

wherein $R_6$, $R_7$, n and m are as defined in claim 1,

or

b) reacting a compound of the general formula

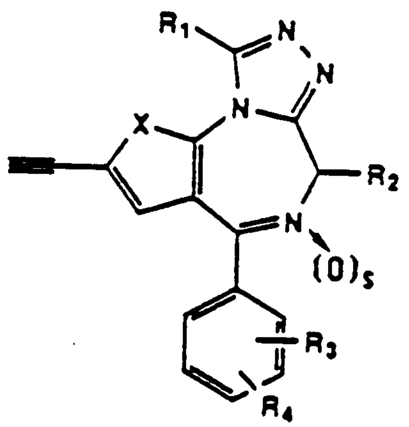

V

wherein X, $R_1$, $R_2$, $R_3$, $R_4$ and s are as defined in claim 1, with a compound of the general formula

$R_6$-Y

wherein $R_6$ is as defined in claim 1 and Y is as defined above, or

c) for the manufacture of a compound of formula I, wherein s is 1 and $R_5$ is other than a moiety containing a basic nitrogen atom, reacting a compound of formula I, wherein s is 0 and $R_5$ is other than a moiety containing a basic nitrogen atom, with a peroxy acid, or

d) for the manufacture of a compound of formula I, wherein s is 0 and $R_2$ is lower alkanoyloxy, reacting a compound of formula I, wherein s is 1 and $R_2$ is hydrogen, with a lower alkanoic acid anhydride, or

e) for the manufacture of a compound of formula I, wherein $R_2$ is hydroxy, hydrolyzing a compound of formula I, wherein $R_2$ is lower alkanoyloxy, and

f) if desired, converting a compound of the general formula I obtained into a pharmaceutically acceptable acid addition salt.

**17.** A pharmaceutical composition comprising a compound in accordance with any one of claims 1-12 and a therapeutically inert carrier.

**18.** A pharmaceutical composition which exhibits activity as a platelet activating factor antagonist comprising a compound in accordance with any one of claims 1-12 and a therapeutically inert carrier.

**19.** The use of a compound in accordance with any one of claims 1-12 for the manufacture of a medicament for the control or prevention of illnesses.

**20.** The use of a compound in accordance with any one of claims 1-12 for the manufacture of a medicament for the control or prevention of disease states characterized by excess platelet activating factor or of cardiovascular diseases, pulmonary diseases, immunological disorders, inflammatory diseases, dermatological disorders, shocks or transplant rejections.

**21.** The use of a compound in accordance with any one of claims 1-12 for the manufacture of a medicament which exhibits activity as platelet activating factor antagonist.

**Claims for the following Contracting State : ES**

**1.** A process for the manufacture of compounds of the general formula

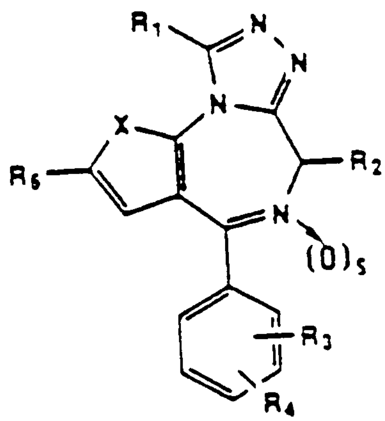

I

wherein X is -CH = CH- or S;
$R_1$ is lower alkyl, lower alkoxy or trifluoromethyl;
$R_2$ is hydrogen, lower alkyl, lower alkoxy, hydroxy or lower alkanoyloxy;
$R_3$ and $R_4$, independently, are hydrogen, chlorine, fluorine, lower alkyl or lower alkoxy;
$R_5$ is a radical of the formula $R_6$-$(CH_2)_n$-C≡C-or $R_7$-O-$(CH_2)_m$-C≡C-,
$R_6$ and $R_7$ are aryl or a heterocyclic radical; n is an integer from 0 to 2; m is an integer from 1 to 2 and s is an integer from 0 to 1; wherein the term "lower alkyl", denotes a straight or branched chain saturated hydrocarbon group containing from 1 to 7 carbon atoms, the term "lower alkoxy" denotes an alkyl ether group in which the alkyl group is as described above and the term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted by lower alkyl, lower alkoxy, oxo, hydroxy, chlorine or fluorine; with the proviso that, when s is 1, $R_2$ cannot be hydroxy, lower alkoxy or lower alkanoyloxy; that, when n is 0, $R_6$ must be attached through a carbon to carbon bond, and that $R_7$ is always attached through a carbon to oxygen bond,
and, when at least one asymmetric centre is present, of their enantiomers and racemates and of pharmaceutically acceptable acid addition salts thereof, which process comprises
a) reacting a compound of the general formula

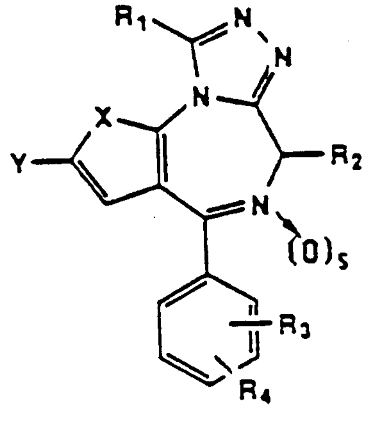

II

wherein X, $R_1$, $R_2$, $R_3$, $R_4$ and s are as defined above, and y is bromo or iodo,
with a compound of the general formula

$R_6$-$(CH_2)_n$-C≡C-H (IIIa) or $R_7$-O-$(CH_2)_n$-C≡C-H (IIIb)

wherein $R_6$, $R_7$, n and m are as defined above, or
b) reacting a compound of the general formula

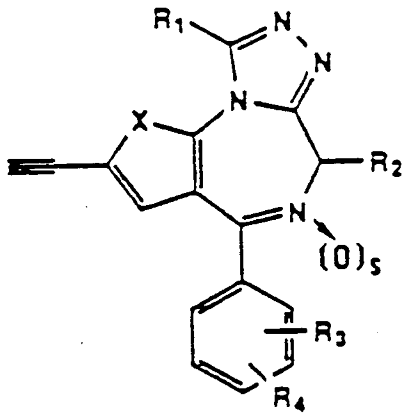

V

wherein X, $R_1$, $R_2$, $R_3$, $R_4$ and s are as defined above,
with a compound of the general formula

$R_6$-Y

wherein $R_6$ and Y are as defined above,
or
c) for the manufacture of a compound of formula I, wherein s is 1 and $R_5$ is other than a moiety containing a basic nitrogen atom, reacting a compound of formula I, wherein s is 0 and $R_5$ is other than a moiety containing a basic nitrogen atom, with a peroxy acid, or
d) for the manufacture of a compound of formula I, wherein s is 0 and $R_2$ is lower alkanoyloxy, reacting a compound of formula I, wherein s is 1 and $R_2$ is hydrogen, with a lower alkanoic acid anhydride, or
e) for the manufacture of a compound of formula I, wherein $R_2$ is hydroxy, hydrolyzing a compound of formula I, wherein $R_2$ is lower alkanoyloxy, and
f) if desired, converting a compound of the general formula I obtained into a pharmaceutically acceptable acid addition salt.

**2.** A process in accordance with claim 1, which comprises manufacturing a compound of formula I defined in claim 1, wherein X is -CH=CH- or S; $R_1$ is lower alkyl or lower alkoxy; $R_2$ is hydrogen, lower alkyl or lower alkoxy; $R_3$ and $R_4$, independently, are hydrogen, chlorine, fluorine, lower alkyl or lower alkoxy; $R_5$ is a radical of the formula $R_6$-$(CH_2)_n$-C≡C- or $R_7$-O-$(CH_2)_m$-C≡C-, $R_6$ and $R_7$ are aryl or a heterocyclic radical, n is an integer from 0 to 2; m is an integer from 1 to 2 and s is the integer 0; with the proviso that, when n is 0, $R_6$ must be attached through a carbon to carbon bond and that $R_7$ is always attached through a carbon to oxygen bond; wherein the term "lower alkyl", denotes a straight or branched chain saturated hydrocarbon group containing from 1 to 7 carbon atoms, the term "lower alkoxy" denotes an alkyl ether group in which the alkyl group is as described above and the term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted by lower alkyl, lower alkoxy, oxo, hydroxy, chlorine or fluorine, and, when at least one asymmetric carbon is present, their enantiomers and racemates and pharmaceutically acceptable acid addition salts thereof in accordance with steps a), b) or f).

**3.** A process in accordance with claim 1 or 2, wherein $R_1$, is methyl or ethyl, $R_2$ is hydrogen, $R_3$ is fluorine or chlorine, $R_4$ is hydrogen, s is 0 and n is 1 or 2.

**4.** A process in accordance with claim 1 or 2, wherein $R_1$ is methyl, $R_2$ is hydrogen, $R_3$ is fluorine or chlorine at the 2-position of the phenyl moiety, $R_4$ is hydrogen, s is 0, m and n are 1, $R_6$ is a bi- or tricyclic heterocyclic radical and $R_7$ is aryl.

**5.** A process in accordance with claim 1 or 2, wherein X is S, $R_1$ is methyl, $R_2$ is hydrogen, $R_3$ is chlorine and at the 2-position of the phenyl moiety, $R_4$ is hydrogen, s is 0, $R_5$ is $R_6$-$(CH_2)_n$-C≡C-, n is 1 and $R_6$ is

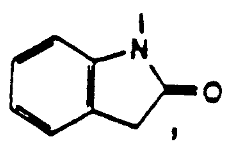,
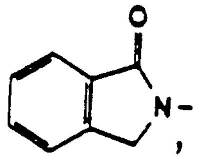,
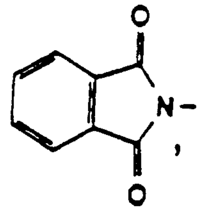,
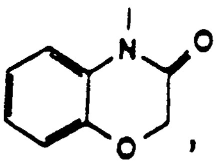,
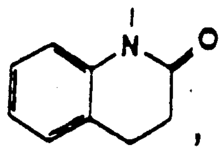,
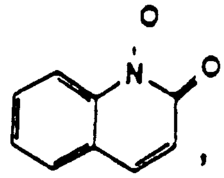,
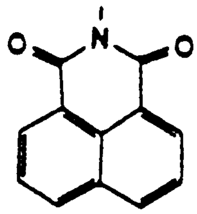,
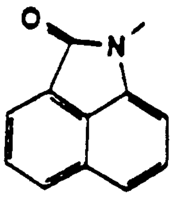,
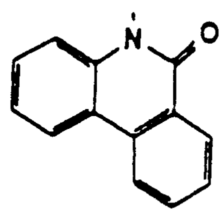 and
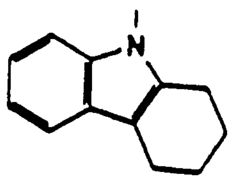

**6.** A process in accordance with claim 1, wherein 5-{3-[4-(2-chlorophenyl)-9-methyl-6H-thieno[3,2-f]-[1,2,4]triazolo[4,3-a][1,4]diazepin-2-yl]-2-propynyl}-phenanthridin-6(5H)-one is manufactured.

**7.** A process in accordance with claim 1, wherein 4-(2-chlorophenyl)-2-[3-(1,2,3,4-tetrahydro-9H-carbazol-9-yl)-1-propynyl]-9-methyl-6H-thieno[3,2-f][1,2,4]triazolo[4,3-a] [1,4]diazepine is manufactured.

**8.** A process in accordance with claim 1, wherein 1-{3-[4-(2-chlorophenyl)-9-methyl-6H-thieno[3,2-f][1,2,4] triazolo[4,3-a][1,4]diazepin-2-yl]-2-propynyl}-3,4-dihydro-2(1H)-quinolinone is manufactured.

**9.** A process in accordance with claim 1, wherein 2-[3-[4-(2-chlorophenyl)-9-methyl-6H-thieno[3,2-f][1,2,4]-triazolo[4,3-a][1,4]diazepin-2-yl]-2-propynyl]-1H-benz[de]isoquinoline-1,3(2H)-dione is manufactured.

**10.** A process in accordance with claim 1, wherein 1-[3-[4-(2-chlorophenyl)-9-methyl-6H-thieno[3,2-f][1,2,4] triazolo[4,3-a] [1,4]diazepin-2-yl]-2-propynyl]-benz[cd]-indol-2(1H)-one is manufactured.

**11.** A process in accordance with claim 1, wherein 4-[3-[4-(2-chlorophenyl)-9-methyl-6H-thieno[3,2-f][1,2,4] triazolo[4,3-a] [1,4]diazepin-2-yl]-2-propynyl]-2H-1,4-benzoxazin-3(4H)-one is manufactured.

**12.** A process for the manufacture of a pharmaceutical composition, particularly to be used in the control or prevention of disease states characterized by excess platelet activating factor or of cardiovascular diseases, plumanory diseases, immunological disorders, inflammatory diseases, dermatological disorders, shocks or transplant rejections, which comprises bringing a compound of formula I, as defined in claim 1 or a pharmaceutically acceptable acid addition salt thereof, and, if desired, one or more other therapeutically active substances together with a therapeutcally inert carrier into a galenical administration form.

**Claims for the following Contracting State : GR**

**1.** A process for the manufacture of compounds of the general formula

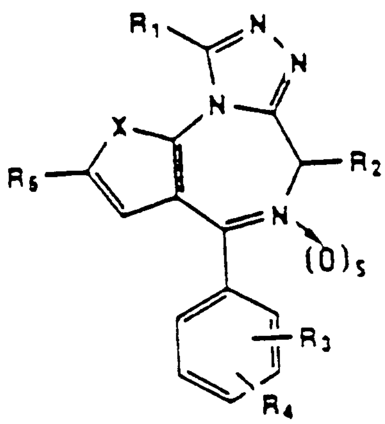

I

wherein X is -CH = CH- or S;
$R_1$ is lower alkyl, lower alkoxy or trifluoromethyl;
$R_2$ is hydrogen, lower alkyl, lower alkoxy, hydroxy or lower alkanoyloxy;
$R_3$ and $R_4$, independently, are hydrogen, chlorine, fluorine, lower alkyl or lower alkoxy;
$R_5$ is a radical of the formula $R_6$-$(CH_2)_n$-C≡C-or $R_7$-O-$(CH_2)_m$-C≡C-,
$R_6$ and $R_7$ are aryl or a heterocyclic radical; n is an integer from 0 to 2; m is an integer from 1 to 2 and s is an integer from 0 to 1; wherein the term "lower alkyl", denotes a straight or branched chain saturated hydrocarbon group containing from 1 to 7 carbon atoms, the term "lower alkoxy" denotes an alkyl ether group in which the alkyl group is as described above and the term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted by lower alkyl, lower alkoxy, oxo, hydroxy, chlorine or fluorine; with the proviso that, when s is 1, $R_2$ cannot be hydroxy, lower alkoxy or lower alkanoyloxy; that, when n is 0, $R_6$ must be attached through a carbon to carbon bond, and that $R_7$ is always attached through a carbon to oxygen bond, and, when at least one asymmetric centre is present, of their enantiomers and racemates and of pharmaceutically acceptable acid addition salts thereof, which process comprises

a) reacting a compound of the general formula

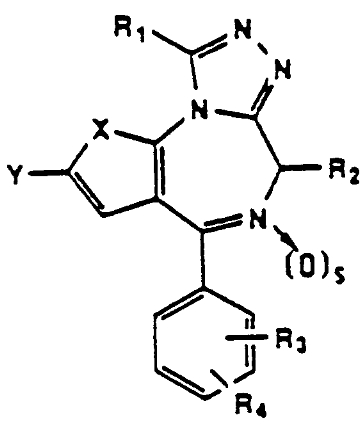

II

wherein X, $R_1$, $R_2$, $R_3$, $R_4$ and s are as defined above, and y is bromo or iodo,
with a compound of the general formula

$R_6$-$(CH_2)_n$-C≡C-H (IIIa) or $R_7$-O-$(CH_2)_n$-C≡C-H (IIIb)

wherein $R_6$, $R_7$, n and m are as defined above,
or
b) reacting a compound of the general formula

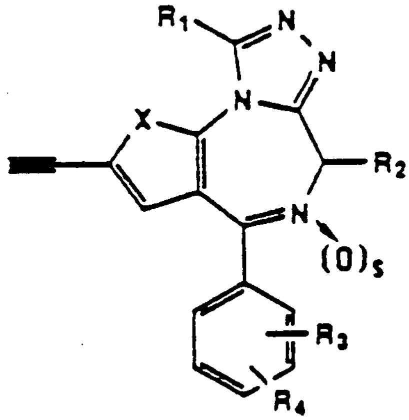

V

wherein X, $R_1$, $R_2$, $R_3$, $R_4$ and s are as defined above,
with a compound of the general formula

$R_6$-Y

wherein $R_6$ and Y are as defined above,
or
c) for the manufacture of a compound of formula I, wherein s is 1 and $R_5$ is other than a moiety containing a basic nitrogen atom, reacting a compound of formula I, wherein s is 0 and $R_5$ is other than a moiety containing a basic nitrogen atom, with a peroxy acid, or
d) for the manufacture of a compound of formula I, wherein s is 0 and $R_2$ is lower alkanoyloxy, reacting a compound of formula I, wherein s is 1 and $R_2$ is hydrogen, with a lower alkanoic acid anhydride, or
e) for the manufacture of a compound of formula I, wherein $R_2$ is hydroxy, hydrolyzing a compound of formula I, wherein $R_2$ is lower alkanoyloxy, and
f) if desired, converting a compound of the general formula I obtained into a pharmaceutically acceptable acid addition salt.

**2.** A process in accordance with claim 1, which comprises manufacturing a compound of formula I defined in claim 1, wherein X is -CH=CH- or S; $R_1$ is lower alkyl or lower alkoxy; $R_2$ is hydrogen, lower alkyl or lower alkoxy; $R_3$ and $R_4$, independently, are hydrogen, chlorine, fluorine, lower alkyl or lower alkoxy; $R_5$ is a radical of the formula $R_6$-$(CH_2)_n$-C≡C- or $R_7$-O-$(CH_2)_m$-C≡C-, $R_6$ and $R_7$ are aryl or a heterocyclic radical, n is an integer from 0 to 2; m is an integer from 1 to 2 and s is the integer 0; with the proviso that, when n is 0, $R_6$ must be attached through a carbon to carbon bond and that $R_7$ is always attached through a carbon to oxygen bond; wherein the term "lower alkyl", denotes a straight or branched chain saturated hydrocarbon group containing from 1 to 7 carbon atoms, the term "lower alkoxy" denotes an alkyl ether group in which the alkyl group is as described above and the term "heterocyclic radical" denotes a monocyclic 5-, 6- or 7- membered heterocyclic or a bi- or tricyclic heterocyclic radical containing one or more hetero atoms, selected from nitrogen, oxygen and sulfur, which radical may be substituted by lower alkyl, lower alkoxy, oxo, hydroxy, chlorine or fluorine; and, when at least one asymmetric carbon is present, their enantiomers and racemates and pharmaceutically acceptable acid addition salts thereof in accordance with steps a), b) or f).

**3.** A process in accordance with claim 1 or 2, wherein $R_1$, is methyl or ethyl, $R_2$ is hydrogen, $R_3$ is fluorine or chlorine, $R_4$ is hydrogen, s is 0 and n is 1 or 2.

**4.** A process in accordance with claim 1 or 2, wherein $R_1$ is methyl, $R_2$ is hydrogen, $R_3$ is fluorine or chlorine at the 2-position of the phenyl moiety, $R_4$ is hydrogen, s is 0, m and n are 1, $R_6$ is a bi- or tricyclic heterocyclic radical and $R_7$ is aryl.

**5.** A process in accordance with claim 1 or 2, wherein X is S, $R_1$ is methyl, $R_2$ is hydrogen, $R_3$ is chlorine and at the 2-position of the phenyl moiety, $R_4$ is hydrogen, s is 0, $R_5$ is $R_6$-$(CH_2)_n$-C≡C-, n is 1 and $R_6$ is

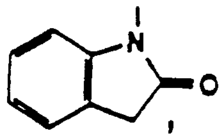 , 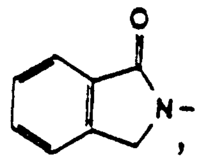 , 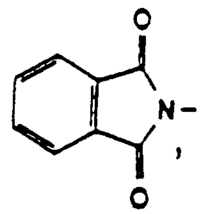 ,

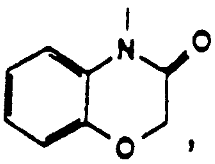 , 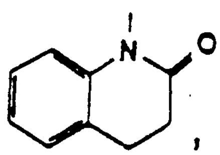 , 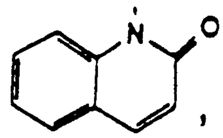 ,

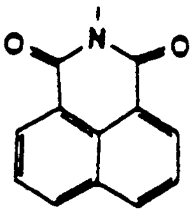 , 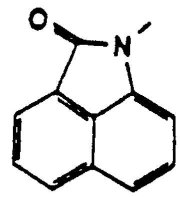 , 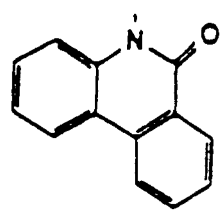 and

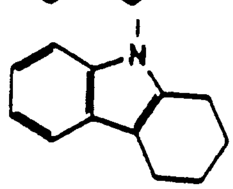

**6.** A process in accordance with claim 1, wherein 5-{3-[4-(2-chlorophenyl)-9-methyl-6H-thieno[3,2-f]-[1,2,4]triazolo[4,3-a][1,4]diazepin-2-yl]-2-propynyl}-phenanthridin-6(5H)-one is manufactured.

**7.** A process in accordance with claim 1, wherein 4-(2-chlorophenyl)-2-[3-(1,2,3,4-tetrahydro-9H-carbazol-9-yl)-1-propynyl]-9-methyl-6H-thieno[3,2-f][1,2,4]triazolo[4,3-a] [1,4]diazepine is manufactured.

**8.** A process in accordance with claim 1, wherein 1-{3-[4-(2-chlorophenyl)-9-methyl-6H-thieno[3,2-f][1,2,4] triazolo[4,3-a][1,4]diazepin-2-yl]-2-propynyl}-3,4-dihydro-2(1H)-quinolinone is manufactured.

**9.** A process in accordance with claim 1, wherein 2-[3-[4-(2-chlorophenyl)-9-methyl-6H-thieno[3,2-f][1,2,4]-triazolo[4,3-a][1,4]diazepin-2-yl]-2-propynyl]-1H-benz[de]isoquinoline-1,3(2H)-dione is manufactured.

**10.** A process in accordance with claim 1, wherein 1-[3-[4-(2-chlorophenyl)-9-methyl-6H-thieno[3,2-f][1,2,4] triazolo[4,3-a] [1,4]diazepin-2-yl]-2-propynyl]-benz[cd]indol-2(1H)-one is manufactured.

**11.** A process in accordance with claim 1, wherein 4-[3-[4-(2-chlorophenyl)-9-methyl-6H-thieno[3,2-f][1,2,4] triazolo[4,3-a] [1,4]diazepin-2-yl]-2-propynyl]-2H-1,4-benzoxazin-3(4H)-one is manufactured.

**12.** A process for the manufacture of a pharmaceutical composition, particularly to be used in the control or prevention of disease states characterized by excess platelet activating factor or of cardiovascular diseases, pulmanory diseases, immunological disorders, inflammatory diseases, dermatological disorders, shocks or transplant rejections, which comprises bringing a compound of formula I, as defined in claim 1 or a pharmaceutically acceptable acid addition salt thereof, and, if desired, one or more other

therapeutically active substances together with a therapeutically inert carrier into a galenical administration form.

**13.** Compounds of the general formulae

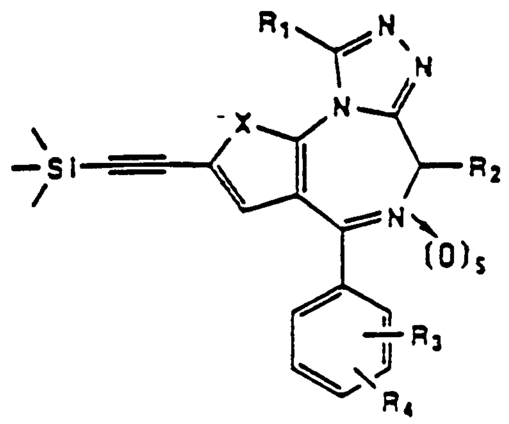

IV

and

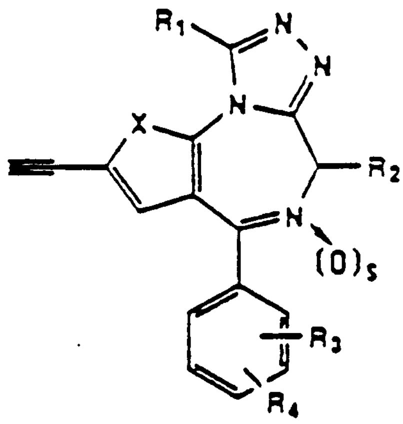

V

wherein X, $R_1$, $R_2$, $R_3$, $R_4$ and s are as defined in claim 1.

**Patentansprüche**
**Patentansprüche für folgende Vertragsstaaten : AT, BE, CH, LI, DE, FR, GB, IT, LU, NL, SE**

**1.** Verbindungen der allgemeinen Formel

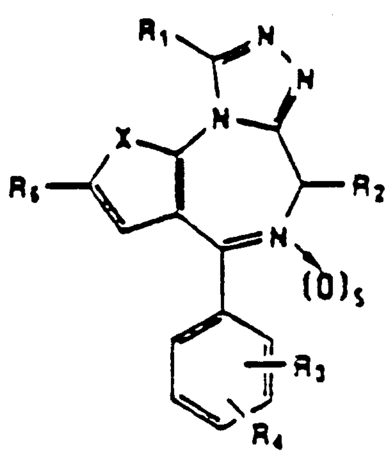

I

worin X -CH = CH- oder S ist;
$R_1$ ein Niedrigalkyl-, Niedrigalkoxy- oder Trifluormethylrest ist;
$R_2$ Wasserstoff, ein Niedrigalkyl-, Niedrigalkoxy-, Hydroxy- oder Niedrigalkanoyloxyrest ist;
$R_3$ und $R_4$ unabhängig Wasserstoff, Chlor, Fluor, Niedrigalkyl- oder Niedrigalkoxyreste sind;
$R_5$ ein Rest der Formel $R_6$-$(CH_2)_n$-C≡C- oder
$R_7$-O-$(CH_2)_m$-C≡C- ist,
$R_6$ und $R_7$ Arylreste oder heterocyclische Reste sind;
n eine ganze Zahl von 0 bis 2 ist; m eine ganze Zahl von 1 bis 2 ist und s eine ganze Zahl von 0 bis 1 ist; wobei der Ausdruck "Niedrigalkylrest" eine geradkettige oder verzweigte gesättigte Kohlenwasserstoffgruppe mit 1 bis 7 Kohlenstoffatomen bedeutet, der Ausdruck "Niedrigalkoxyrest" eine Alkylethergruppe bedeutet, worin die Alkylgruppe wie oben beschrieben ist und der Ausdruck "heterocyclischer

Rest" einen monocyclischen 5-, 6- oder 7-gliedrigen heterocyclischen oder bi- oder tricyclischen heterocyclischen Rest mit ein oder mehreren Heteroatomen, ausgewählt aus Stickstoff, Sauerstoff und Schwefel bedeutet, wobei der Rest mit Niedrigalkyl-, Niedrigalkoxy-, Oxo-, Hydroxy-, Chlor- oder Fluorresten substituiert sein kann; mit dem Vorbehalt, daß dann, wenn s 1 ist, $R_2$ kein Hydroxy-, Niedrigalkoxy- oder Niedrigalkanoyloxyrest sein kann; daß dann, wenn n 0 ist, $R_6$ über eine Kohlenstoff-Kohlenstoff-Bindung gebunden sein muß und daß $R_7$ immer über eine Kohlenstoff-Sauerstoff-Bindung gebunden sein muß,
und wenn mindestens ein asymmetrisches Zentrum vorhanden ist, deren Enantiomere und Razemate, und pharmazeutisch annehmbare Säure-Additionssalze davon.

**2.** Verbindungen nach Anspruch 1, worin X -CH=CH- oder S ist; $R_1$ ein Niedrigalkyl- oder Niedrigalkoxyrest ist; $R_2$ Wasserstoff, ein Niedrigalkyl- oder Niedrigalkoxyrest ist; $R_3$ und $R_4$ unabhängig Wasserstoff, Chlor, Fluor, Niedrigalkyl- oder Niedrigalkoxyreste sind; $R_5$ ein Rest der Formel $R_6$-$(CH_2)_n$-C≡C- oder $R_7$-O-$(CH_2)_m$-C≡C- ist, $R_6$ und $R_7$ Arylreste oder heterocyclische Reste sind, n eine ganze Zahl von 0 bis 2 ist; m eine ganze Zahl von 1 bis 2 ist und s die ganze Zahl 0 ist; wobei der Ausdruck "Niedrigalkylrest" eine geradkettige oder verzweigte gesättigte Kohlenwasserstoffgruppe mit 1 bis 7 Kohlenstoffatomen bedeutet, der Ausdruck "Niedrigalkoxyrest" eine Alkylethergruppe bedeutet, worin die Alkylgruppe wie oben beschrieben ist und der Ausdruck "heterocyclischer Rest" einen monocyclischen 5-, 6- oder 7-gliedrigen heterocyclischen oder bi- oder tricyclischen heterocyclischen Rest mit ein oder mehreren Heteroatomen, ausgewählt aus Stickstoff, Sauerstoff und Schwefel bedeutet, wobei der Rest mit Niedrigalkyl-, Niedrigalkoxy-, Oxo-, Hydroxy-, Chlor- oder Fluorresten substituiert sein kann; mit dem Vorbehalt, daß dann, wenn n 0 ist, $R_6$ über eine Kohlenstoff-Kohlenstoff-Bindung gebunden sein muß und daß $R_7$ immer über eine Kohlenstoff-Sauerstoff-Bindung gebunden ist.

**3.** Verbindungen nach Anspruch 1 oder 2, worin $R_1$ ein Methyl- oder Ethylrest ist, $R_2$ Wasserstoff ist, $R_3$ Fluor oder Chlor ist, $R_4$ Wasserstoff ist, s 0 ist und n 1 oder 2 ist.

**4.** Verbindungen nach Anspruch 1 oder 2, worin $R_1$ ein Methylrest ist, $R_2$ Wasserstoff ist, $R_3$ Fluor oder Chlor an Position 2 des Phenylrestes ist, $R_4$ Wasserstoff ist, s 0 ist, m und n 1 sind, $R_6$ ein bi- oder tricyclischer heterocyclischer Rest ist und $R_7$ ein Arylrest ist.

**5.** Verbindungen nach Anspruch 1 oder 2, worin X S ist, $R_1$ ein Methylrest ist, $R_2$ Wasserstoff ist, $R_3$ Chlor ist und an Position 2 des Phenylrestes ist, $R_4$ Wasserstoff ist, s 0 ist, $R_5$ $R_6$-$(CH_2)_n$-C≡C- ist, n 1 ist und $R_6$

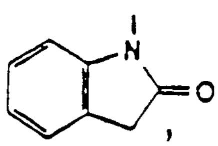 , 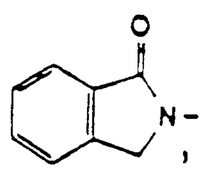 , 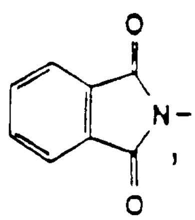 ,

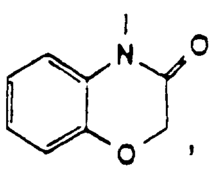 , 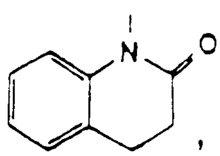 , 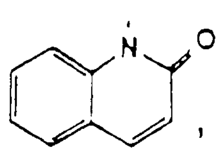 ,

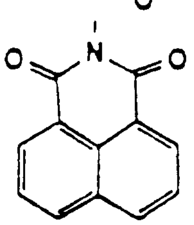 , 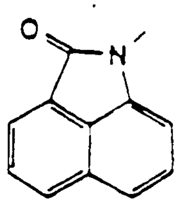 , 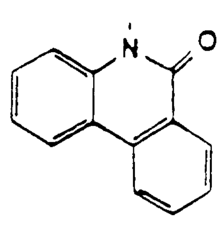 und 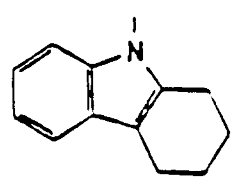

ist.

6. 5-{3-[4-(2-Chlorphenyl)-9-methyl-6H-thieno[3,2-f][1,2,4]triazolo[4,3-a][1,4]diazepin-2-yl]-2-propinyl}-phenanthridin-6(5H)-on.

7. 4-(2-Chlorphenyl)-2-[3-(1,2,3,4-tetrahydro-9H-carbazol-9-yl)-1-propinyl]-9-methyl-6H-thieno[3,2-f][1,2,4]-triazolo[4,3-a][1,4]diazepin.

8. 1-{3-[4-(2-Chlorphenyl)-9-methyl-6H-thieno[3,2-f][1,2,4]triazolo[4,3-a][1,4]diazepin-2-yl]-2-propinyl}-3,4-dihydro-2(1H)-chinolinon.

9. 2-[3-[4-(2-Chlorphenyl)-9-methyl-6H-thieno[3,2-f][1,2,4]triazolo[4,3-a][1,4]diazepin-2-yl]-2-propinyl]-1H-benz[de]isochinolin-1,3(2H)-dion.

10. 1-[3-[4-(2-Chlorphenyl)-9-methyl-6H-thieno[3,2-f][1,2,4]triazolo[4,3-a][1,4]diazepin-2-yl]-2-propinyl]-benz-[cd]-indol-2(1H)-on.

11. 4-[3-[4-(2-Chlorphenyl)-9-methyl-6H-thieno[3,2-f][1,2,4]triazolo[4,3-a][1,4]diazepin-2-yl]-2-propinyl]-2H-1,4-benzoxazin-3(4H)-on.

12. 1-[3-[4-(2-Chlorphenyl)-9-methyl-6H-thieno[3,2-f][1,2,4]triazolo[4,3-a][1,4]diazepin-2-yl]-2-propinyl]-1H-indol-2,3-dion;
1-[3-[4-(2-Chlorphenyl)-9-methyl-6H-thieno[3,2-f][1,2,4]triazolo[4,3-a][1,4]diazepin-2-yl]-2-propinyl]-1,3-dihydro-2H-indol-2-on;
2-[3-[4-(2-Chlorphenyl)-9-methyl-6H-thieno[3,2-f][1,2,4]triazolo[4,3-a][1,4]diazepin-2-yl]-2-propinyl]-[1,2,4]-triazolo[4,3-a]pyridin-3(2H)-on;
2-[3-[4-(2-Chlorphenyl)-9-methyl-6H-thieno[3,2-f][1,2,4]triazolo[4,3-a][1,4]diazepin-2-yl]-2-propinyl]-1,2-benzisothiazol-3(2H)-on-1,1-dioxid;
4-(2-Chlorphenyl)-2-[3-(1H-indazol-1-yl)-1-propinyl]-9-methyl-6H-thieno[3,2-f][1,2,4]triazolo[4,3-a][1,4]-diazepin;
2-[3-(1H-Benzimidazol-1-yl)-1-propinyl]-4-(2-chlorphenyl)-9-methyl-6H-thieno[3,2-f][1,2,4]triazolo[4,3-a]-[1,4]diazepin;
2-[3-[6-(2-Fluorphenyl)-1-methyl-4H-[1,2,4]triazolo[4,3-a][1,4]benzodiazepin-8-yl]-2-propinyl]-1H-isoindol-1,3(2H)-dion und
4-[3-[6-(2-Fluorphenyl)-1-methyl-4H-[1,2,4]triazolo[4,3-a][1,4]benzodiazepin-8-yl]-2-propinyl]-2H-1,4-benzoxazin-3(4H)-on.

13. Verbindungen der allgemeinen Formeln

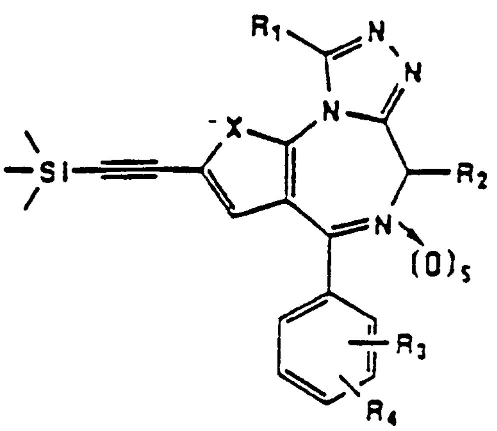

IV

und

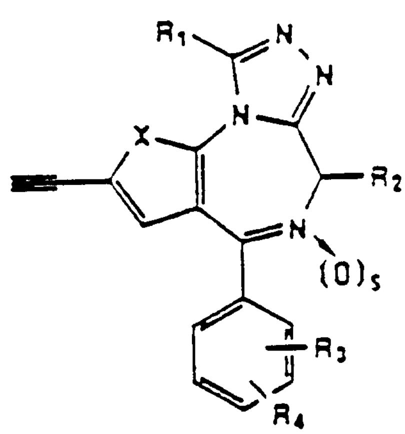

V

worin X, $R_1$, $R_2$, $R_3$, $R_4$ und s wie in Anspruch 1 definiert sind.

**14.** Verbindungen nach einem der Ansprüche 1 bis 12 zur Verwendung als therapeutisch aktive Substanzen.

**15.** Verbindungen nach einem der Ansprüche 1 bis 12 zur Verwendung als Antagonisten des Plättchenaktivierungsfaktors (PAF).

**16.** Verfahren zur Herstellung von Verbindungen nach einem der Ansprüche 1 bis 12 und von pharmazeutisch annehmbaren Säureadditonssalzen davon, wobei das Verfahren umfaßt, daß man
a) eine Verbindung der allgemeinen Formel

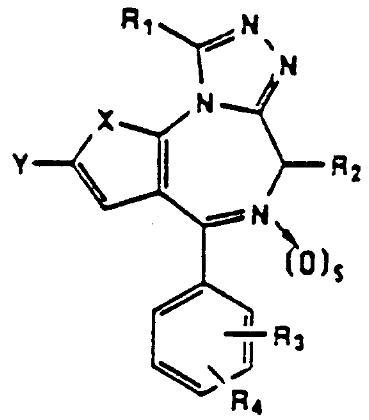

II

worin X, $R_1$, $R_2$, $R_3$, $R_4$ und s wie in Anspruch 1 definiert sind, und y Brom oder Iod ist mit einer Verbindung der allgemeinen Formel

$R_6$-$(CH_2)_n$-C≡C-H (IIIa) oder $R_7$-O-$(CH_2)_n$-C≡C-H (IIIb)

worin $R_6$, $R_7$, n und m wie in Anspruch 1 definiert sind, umsetzt oder
b) eine Verbindung der allgemeinen Formel

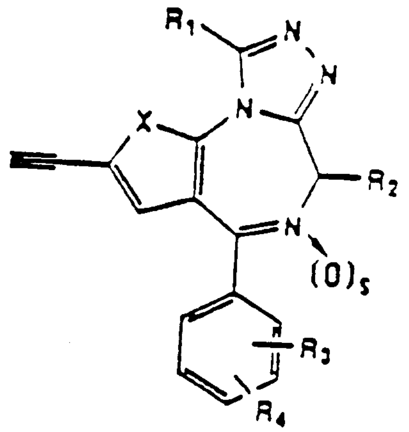

V

worin X, $R_1$, $R_2$, $R_3$, $R_4$ und s wie in Anspruch 1 definiert sind, mit einer Verbindung der allgemeinen Formel

$R_6$-Y

worin $R_6$ wie in Anspruch 1 definiert ist und Y wie oben definiert ist, umsetzt oder
c) zur Herstellung einer Verbindung der Formel I, worin s 1 ist und $R_5$ etwas anderes als ein ein basisches Stickstoffatom enthaltender Rest ist, eine Verbindung der Formel I, worin s 0 ist und $R_5$ etwas anderes als ein ein basisches Stickstoffatom enthaltender Rest ist, mit einer Peroxysäure

umsetzt oder

d) zur Herstellung einer Verbindung der Formel I, worin s 0 ist und $R_2$ ein Niedrigalkanoyloxyrest ist, eine Verbindung der Formel I, worin s 1 ist und $R_2$ Wasserstoff ist, mit einem Niedrigalkansäureanhydrid umsetzt oder

e) zur Herstellung einer Verbindung der Formel I, worin $R_2$ ein Hydroxyrest ist, eine Verbindung der Formel I, worin $R_2$ ein Niedrigalkanoyloxyrest ist, hydrolysiert und

f) falls erwünscht, eine Verbindung der allgemeinen Formel I, in ein pharmazeutisch annehmbares Säureadditionssalz umwandelt.

**17.** Pharmazeutische Zusammensetzung, umfassend eine Verbindung nach einem der Ansprüche 1 bis 12 und einen therapeutisch inerten Träger.

**18.** Pharmazeutische Zusammensetzung, die Aktivität als Antagonist des Plättchenaktivierungsfaktors aufweist, umfassend eine Verbindung nach einem der Ansprüche 1 bis 12 und einen therapeutisch inerten Träger.

**19.** Verwendung einer Verbindung nach einem der Ansprüche 1 bis 12 zur Herstellung eines Arzneimittels zur Kontrolle oder Verhütung von Krankheiten.

**20.** Verwendung einer Verbindung nach einem der Ansprüche 1 bis 12 zur Herstellung eines Arzneimittels zur Kontrolle oder Verhütung von Krankheitszuständen, die durch einen Überschuß des Plättchenaktivierungsfaktors gekennzeichnet sind oder von kardiovaskulären Erkrankungen, Lungenerkrankungen, immunologischen Störungen, entzündlichen Erkrankungen, dermatologischen Störungen, Schocks oder Transplantatabstoßungen.

**21.** Verwendung einer Verbindung nach einem der Ansprüche 1 bis 12 zur Herstellung eines Arzneimittels, das eine Aktivität als Antagonist des Plättchenaktivierungsfaktors zeigt.

**Patentansprüche für folgenden Vertragsstaat : ES**

**1.** Verfahren zur Herstellung von Verbindungen der allgemeinen Formel

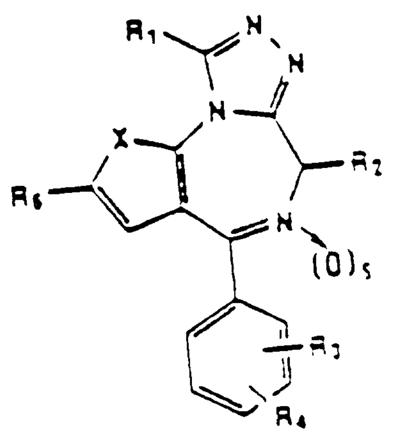

I

worin X -CH = CH- oder S ist;
$R_1$ ein Niedrigalkyl-, Niedrigalkoxy- oder Trifluormethylrest ist;
$R_2$ Wasserstoff, ein Niedrigalkyl-, Niedrigalkoxy-, Hydroxy- oder Niedrigalkanoyloxyrest ist;
$R_3$ und $R_4$ unabhängig Wasserstoff, Chlor, Fluor, Niedrigalkyl- oder Niedrigalkoxyreste sind;
$R_5$ ein Rest der Formel $R_6$-$(CH_2)_n$-C≡C- oder $R_7$-O-$(CH_2)_m$-C≡C- ist,
$R_6$ und $R_7$ Arylreste oder heterocyclische Reste sind;
n eine ganze Zahl von 0 bis 2 ist; m eine ganze Zahl von 1 bis 2 ist und s eine ganze Zahl von 0 bis 1 ist; wobei der Ausdruck "Niedrigalkylrest" eine geradkettige oder verzweigte gesättigte Kohlenwasserstoffgruppe mit 1 bis 7 Kohlenstoffatomen bedeutet, der Ausdruck "Niedrigalkoxyrest" eine Alkylethergruppe bedeutet, worin die Alkylgruppe wie oben beschrieben ist, und der Ausdruck "heterocyclischer

Rest" einen monocyclischen 5-, 6- oder 7-gliedrigen heterocyclischen oder bi- oder tricyclischen heterocyclischen Rest mit ein oder mehreren Heteroatomen, ausgewählt aus Stickstoff, Sauerstoff und Schwefel, bedeutet, wobei der Rest mit Niedrigalkyl-, Niedrigalkoxy-, Oxo-, Hydroxy-, Chlor- oder Fluorresten substituiert sein kann; mit dem Vorbehalt, daß dann, wenn s 1 ist, $R_2$ kein Hydroxy-, Niedrigalkoxy- oder Niedrigalkanoyloxyrest sein kann; daß dann, wenn n 0 ist, $R_6$ über eine Kohlenstoff-Kohlenstoff-Bindung gebunden sein muß und daß $R_7$ immer über eine Kohlenstoff-Sauerstoff-Bindung gebunden ist,

und, wenn mindestens ein asymmetrisches Zentrum vorhanden ist, von deren Enantiomeren und Razematen, und pharmazeutisch annehmbaren Säureadditionssalzen davon, wobei das Verfahren umfaßt, daß man

a) eine Verbindung der allgemeinen Formel

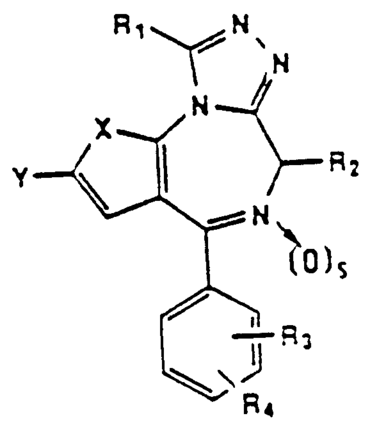

II

worin X, $R_1$, $R_2$, $R_3$, $R_4$ und s wie oben definiert sind, und y Brom oder Iod ist, mit einer Verbindung der allgemeinen Formel

$R_6$-$(CH_2)_n$-C≡C-H (IIIa) oder $R_7$-O-$(CH_2)_n$-C≡C-H (IIIb)

worin $R_6$, $R_7$, n und m wie oben definiert sind, umsetzt oder

b) eine Verbindung der allgemeinen Formel

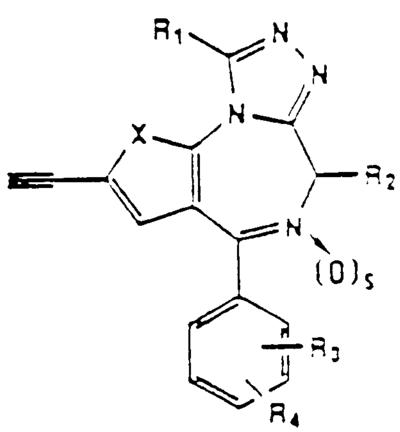

V

worin X, $R_1$, $R_2$, $R_3$, $R_4$ und s wie oben definiert sind, mit einer Verbindung der allgemeinen Formel

$R_6$-Y

worin $R_6$ und Y wie oben definiert sind, umsetzt oder

c) zur Herstellung einer Verbindung der Formel I, worin s 1 ist und $R_5$ etwas anderes als ein ein basisches Stickstoffatom enthaltender Rest ist, eine Verbindung der Formel I, worin s 0 ist und $R_5$ etwas anderes als ein ein basisches Stickstoffatom enthaltender Rest ist, mit einer Peroxysäure umsetzt oder

d) zur Herstellung einer Verbindung der Formel I, worin s 0 ist und $R_2$ ein Niedrigalkanoyloxyrest ist, eine Verbindung der Formel I, worin s 1 ist und $R_2$ Wasserstoff ist, mit einem Niedrigalkansäureanhydrid umsetzt oder

e) zur Herstellung einer Verbindung der Formel I, worin $R_2$ ein Hydroxyrest ist, eine Verbindung der Formel I, worin $R_2$ ein Niedrigalkanoyloxyrest ist, hydrolysiert und

f) falls erwünscht, eine Verbindung der allgemeinen Formel I, in ein pharmazeutisch annehmbares Säureadditionssalz umwandelt.

**2.** Verfahren nach Anspruch 1, umfassend, daß man eine Verbindung der Formel I, wie in Anspruch 1 definiert, herstellt, worin X -CH=CH- oder S ist; $R_1$ ein Niedrigalkyl- oder Niedrigalkoxyrest ist; $R_2$ Wasserstoff, ein Niedrigalkyl- oder Niedrigalkoxyrest ist; $R_3$ und $R_4$ unabhängig Wasserstoff, Chlor, Fluor, Niedrigalkyl- oder Niedrigalkoxyreste sind; $R_5$ ein Rest der Formel $R_6$-$(CH_2)_n$-C≡C- oder $R_7$-O-$(CH_2)_m$-C≡C-ist, $R_6$ und $R_7$ Arylreste oder heterocyclische Reste sind, n eine ganze Zahl von 0 bis 2 ist; m eine ganze Zahl von 1 bis 2 ist und s die ganze Zahl 0 ist;
mit dem Vorbehalt, daß dann, wenn n 0 ist, $R_6$ über eine Kohlenstoff-Kohlenstoff-Bindung gebunden sein muß und daß $R_7$ immer über eine Kohlenstoff-Sauerstoff-Bindung gebunden ist, wobei der Ausdruck "Niedrigalkylrest" eine geradkettige oder verzweigte gesättigte Kohlenwasserstoffgruppe mit 1 bis 7 Kohlenstoffatomen bedeutet, der Ausdruck "Niedrigalkoxyrest" eine Alkylethergruppe bedeutet, worin die Alkylgruppe wie oben beschrieben ist und der Ausdruck "heterocyclischer Rest" einen monocyclischen 5-, 6- oder 7-gliedrigen heterocyclischen oder bi- oder tricyclischen heterocyclischen Rest mit ein oder mehreren Heteroatomen, ausgewählt aus Stickstoff, Sauerstoff und Schwefel bedeutet, wobei der Rest mit Niedrigalkyl-, Niedrigalkoxy-, Oxo-, Hydroxy-, Chlor- oder Fluorresten substituiert sein kann; und, falls mindestens ein asymmetrisches Kohlenstoffatom vorhanden ist, deren Enantiomere und Racemate und pharmazeutisch annehmbaren Säureadditionssalze gemäß den Stufen a), b) oder f).

**3.** Verfahren nach Anspruch 1 oder 2, worin $R_1$ ein Methyl- oder Ethylrest ist, $R_2$ Wasserstoff ist, $R_3$ Fluor oder Chlor ist, $R_4$ Wasserstoff ist, s 0 ist und n 1 oder 2 ist.

**4.** Verfahren nach Anspruch 1 oder 2, worin $R_1$ ein Methylrest ist, $R_2$ Wasserstoff ist, $R_3$ Fluor oder Chlor ist und an Position 2 des Phenylrestes ist, $R_4$ Wasserstoff ist, s 0 ist, m und n 1 sind, $R_6$ ein bi- oder tricyclischer heterocyclischer Rest ist und $R_7$ ein Arylrest ist.

**5.** Verfahren nach Anspruch 1 oder 2, worin X S ist, $R_1$ ein Methylrest ist, $R_2$ Wasserstoff ist, $R_3$ Chlor ist und an Position 2 des Phenylrestes ist, $R_4$ Wasserstoff ist, s 0 ist, $R_5$ $R_6$-$(CH_2)_n$-C≡C- ist, n 1 ist und $R_6$

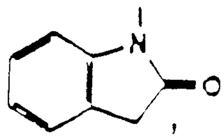, 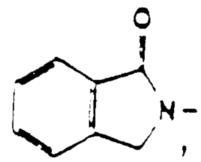, 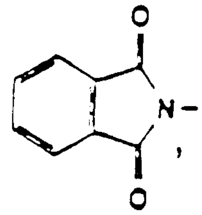, 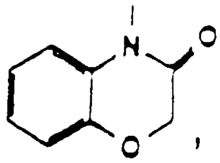, 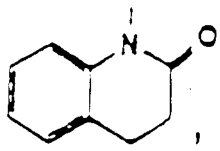, 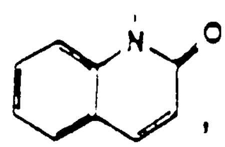, 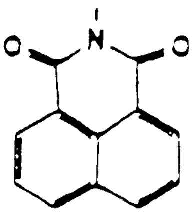, 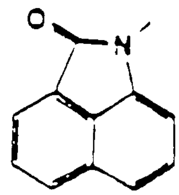, 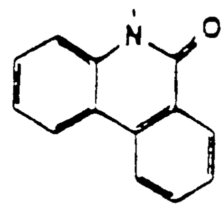 und 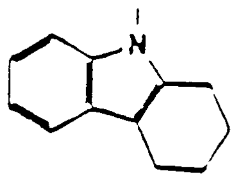

ist.

6. Verfahren nach Anspruch 1, worin 5-{3-[4-(2-Chlorphenyl)-9-methyl-6H-thieno[3,2-f][1,2,4]triazolo[4,3-a]-[1,4]diazepin-2-yl]-2-propinyl}-phenanthridin-6(5H)-on hergestellt wird.

7. Verfahren nach Anspruch 1, worin 4-(2-Chlorphenyl)-2-[3-(1,2,3,4-tetrahydro-9H-carbazol-9-yl)-1-propinyl]-9-methyl-6H-thieno[3,2-f][1,2,4]triazolo[4,3-a][1,4]-diazepin hergestellt wird.

8. Verfahren nach Anspruch 1, worin 1-{3-[4-(2-Chlorphenyl)-9-methyl-6H-thieno[3,2-f][1,2,4]triazolo[4,3-a]-[1,4]diazepin-2-yl]-2-propinyl}-3,4-dihydro-2(1H)-chinolinon hergestellt wird.

9. Verfahren nach Anspruch 1, worin 2-[3-[4-(2-Chlorphenyl)-9-methyl-6H-thieno[3,2-f][1,2,4]triazolo[4,3-a]-[1,4]diazepin-2-yl]-2-propinyl]-1H-benz[de]isochinolin-1,3(2H)-dion hergestellt wird.

10. Verfahren nach Anspruch 1, worin 1-[3-[4-(2-Chlorphenyl)-9-methyl-6H-thieno[3,2-f][1,2,4]triazolo[4,3-a]-[1,4]diazepin-2-yl]-2-propinyl]-benz[cd]-indol-2(1H)-on hergestellt wird.

11. Verfahren nach Anspruch 1, worin 4-[3-[4-(2-Chlorphenyl)-9-methyl-6H-thieno[3,2-f][1,2,4]triazolo[4,3-a]-[1,4]diazepin-2-yl]-2-propinyl]-2H-1,4-benzoxazin-3(4H)-on hergestellt wird.

12. Verfahren zur Herstellung einer pharmazeutischen Zusammensetzung, die insbesondere verwendet wird zur Kontrolle oder Verhütung von Krankheitszuständen, die durch einen Überschuß an Plättchenaktivierungsfaktor gekennzeichnet sind oder von kardiovaskulären Erkrankungen, Lungenerkrankungen, immunologischen Störungen, entzündlichen Erkrankungen, dermatologischen Störungen, Schocks oder Transplantatabstoßungen, umfassend, daß man eine Verbindung der Formel I, wie in Anspruch 1 definiert, oder ein pharmazeutisch annehmbares Säureadditionssalz davon und, falls erwünscht, ein oder mehrere andere therapeutisch aktive Substanzen, zusammen mit einem therapeutisch inerten Träger in eine galenische Verabreichungsform bringt.

**Patentansprüche für folgenden Vertragsstaat : GR**

1. Verfahren zur Herstellung von Verbindungen der allgemeinen Formel

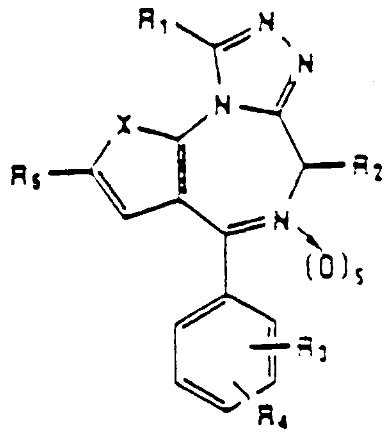

I

worin X -CH = CH- oder S ist;
$R_1$ ein Niedrigalkyl-, Niedrigalkoxy- oder Trifluormethylrest ist;
$R_2$ Wasserstoff, ein Niedrigalkyl-, Niedrigalkoxy-, Hydroxy- oder Niedrigalkanoyloxyrest ist;
$R_3$ und $R_4$ unabhängig Wasserstoff, Chlor, Fluor, Niedrigalkyl- oder Niedrigalkoxyreste sind;
$R_5$ ein Rest der Formel $R_6$-$(CH_2)_n$-C≡C- oder
$R_7$-O-$(CH_2)_m$-C≡C- ist,
$R_6$ und $R_7$ Arylreste oder heterocyclische Reste sind;
n eine ganze Zahl von 0 bis 2 ist; m eine ganze Zahl von 1 bis 2 ist und s eine ganze Zahl von 0 bis 1 ist; wobei der Ausdruck "Niedrigalkylrest" eine geradkettige oder verzweigte gesättigte Kohlenwasserstoffgruppe mit 1 bis 7 Kohlenstoffatomen bedeutet, der Ausdruck "Niedrigalkoxyrest" eine Alkylethergruppe bedeutet, worin die Alkylgruppe wie oben beschrieben ist, und der Ausdruck "heterocyclischer Rest" einen monocyclischen 5-, 6- oder 7-gliedrigen heterocyclischen oder bi- oder tricyclischen heterocyclischen Rest mit ein oder mehreren Heteroatomen, ausgewählt aus Stickstoff, Sauerstoff und Schwefel, bedeutet, wobei der Rest mit Niedrigalkyl-, Niedrigalkoxy-, Oxo-, Hydroxy-, Chlor- oder Fluorresten substituiert sein kann; mit dem Vorbehalt, daß dann, wenn s 1 ist, $R_2$ kein Hydroxy-, Niedrigalkoxy- oder Niedrigalkanoyloxyrest sein kann; daß dann, wenn n 0 ist, $R_6$ über eine Kohlenstoff-Kohlenstoff-Bindung gebunden sein muß und daß $R_7$ immer über eine Kohlenstoff-Sauerstoff-Bindung gebunden ist,
und, wenn mindestens ein asymmetrisches Zentrum vorhanden ist, von deren Enantiomeren und Razematen, und pharmazeutisch annehmbaren Säureadditionssalzen davon, wobei das Verfahren umfaßt, daß man

a) eine Verbindung der allgemeinen Formel

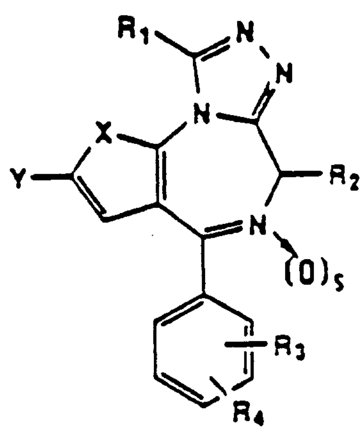

II

worin X, $R_1$, $R_2$, $R_3$, $R_4$ und s wie oben definiert sind, und y Brom oder Iod ist, mit einer Verbindung der allgemeinen Formel

$R_6$-$(CH_2)_n$-C≡C-H (IIIa) oder $R_7$-O-$(CH_2)_n$-C≡C-H (IIIb)

worin $R_6$, $R_7$, n und m wie oben definiert sind, umsetzt oder

b) eine Verbindung der allgemeinen Formel

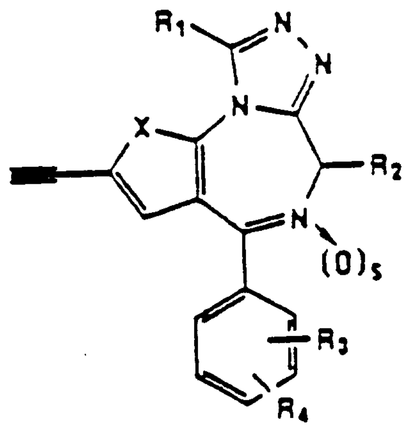

V

worin X, $R_1$, $R_2$, $R_3$, $R_4$ und s wie oben definiert sind, mit einer Verbindung der allgemeinen Formel

$R_6$-Y

worin $R_6$ und Y wie oben definiert sind, umsetzt oder

c) zur Herstellung einer Verbindung der Formel I, worin s 1 ist und $R_5$ etwas anderes als ein ein basisches Stickstoffatom enthaltender Rest ist, eine Verbindung der Formel I, worin s 0 ist und $R_5$ etwas anderes als ein ein basisches Stickstoffatom enthaltender Rest ist, mit einer Peroxysäure umsetzt oder

d) zur Herstellung einer Verbindung der Formel I, worin s 0 ist und $R_2$ ein Niedrigalkanoyloxyrest ist, eine Verbindung der Formel I, worin s 1 ist und $R_2$ Wasserstoff ist, mit einem Niedrigalkansäureanhydrid umsetzt oder

e) zur Herstellung einer Verbindung der Formel I, worin $R_2$ ein Hydroxyrest ist, eine Verbindung der Formel I, worin $R_2$ ein Niedrigalkanoyloxyrest ist, hydrolysiert und

f) falls erwünscht, eine Verbindung der allgemeinen Formel I, in ein pharmazeutisch annehmbares Säureadditionssalz umwandelt.

**2.** Verfahren nach Anspruch 1, umfassend, daß man eine Verbindung der Formel I, wie in Anspruch 1 definiert, herstellt, worin X -CH=CH- oder S ist; $R_1$ ein Niedrigalkyl- oder Niedrigalkoxyrest ist; $R_2$ Wasserstoff, ein Niedrigalkyl- oder Niedrigalkoxyrest ist; $R_3$ und $R_4$ unabhängig Wasserstoff, Chlor, Fluor, Niedrigalkyl- oder Niedrigalkoxyreste sind; $R_5$ ein Rest der Formel $R_6$-$(CH_2)_n$-C≡C- oder $R_7$-O-$(CH_2)_m$-C≡C-ist, $R_6$ und $R_7$ Arylreste oder heterocyclische Reste sind, n eine ganze Zahl von 0 bis 2 ist; m eine ganze Zahl von 1 bis 2 ist und s die ganze Zahl 0 ist; mit dem Vorbehalt, daß dann, wenn n 0 ist, $R_6$ über eine Kohlenstoff-Kohlenstoff-Bindung gebunden sein muß und daß $R_7$ immer über eine Kohlenstoff-Sauerstoff-Bindung gebunden ist, wobei der Ausdruck "Niedrigalkylrest" eine geradkettige oder verzweigte gesättigte Kohlenwasserstoffgruppe mit 1 bis 7 Kohlenstoffatomen bedeutet, der Ausdruck "Niedrigalkoxyrest" eine Alkylethergruppe bedeutet, worin die Alkylgruppe wie oben beschrieben ist und der Ausdruck "heterocyclischer Rest" einen monocyclischen 5-, 6- oder 7-gliedrigen heterocyclischen oder bi- oder tricyclischen heterocyclischen Rest mit ein oder mehreren Heteroatomen, ausgewählt aus Stickstoff, Sauerstoff und Schwefel bedeutet, wobei der Rest mit Niedrigalkyl-, Niedrigalkoxy-, Oxo-, Hydroxy-, Chlor- oder Fluorresten substituiert sein kann; und, falls mindestens ein asymmetrisches Kohlenstoffatom vorhanden ist, deren Enantiomere und Racemate und pharmazeutisch annehmbaren Säureadditionssalze gemäß den Stufen a), b) oder f).

3. Verfahren nach Anspruch 1 oder 2, worin $R_1$ ein Methyl- oder Ethylrest ist, $R_2$ Wasserstoff ist, $R_3$ Fluor oder Chlor ist, $R_4$ Wasserstoff ist, s 0 ist und n 1 oder 2 ist.

4. Verfahren nach Anspruch 1 oder 2, worin $R_1$ ein Methylrest ist, $R_2$ Wasserstoff ist, $R_3$ Fluor oder Chlor ist und an Position 2 des Phenylrestes ist, $R_4$ Wasserstoff ist, s 0 ist, m und n 1 sind, $R_6$ ein bi- oder tricyclischer heterocyclischer Rest ist und $R_7$ ein Arylrest ist.

5. Verfahren nach Anspruch 1 oder 2, worin X S ist, $R_1$ ein Methylrest ist, $R_2$ Wasserstoff ist, $R_3$ Chlor ist und an Position 2 des Phenylrestes ist, $R_4$ Wasserstoff ist, s 0 ist, $R_5$ $R_6$-$(CH_2)_n$-C≡C- ist, n 1 ist und $R_6$

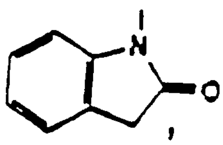
,
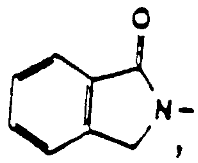
,
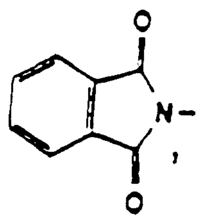
,
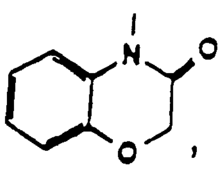
,
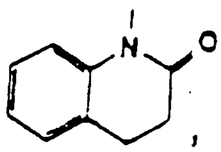
,
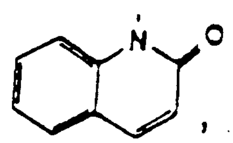
,
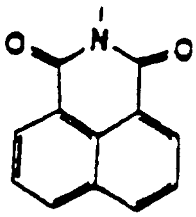
,
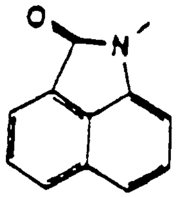
,
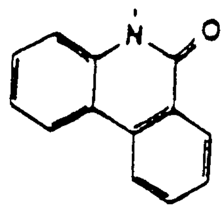
und
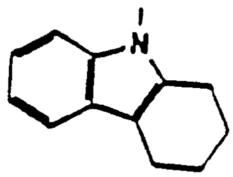

ist.

6. Verfahren nach Anspruch 1, worin 5-{3-[4-(2-Chlorphenyl)-9-methyl-6H-thieno[3,2-f][1,2,4]triazolo[4,3-a]-[1,4]diazepin-2-yl]-2-propinyl}-phenanthridin-6(5H)-on hergestellt wird.

7. Verfahren nach Anspruch 1, worin 4-(2-Chlorphenyl)-2-[3-(1,2,3,4-tetrahydro-9H-carbazol-9-yl)-1-propinyl]-9-methyl-6H-thieno[3,2-f][1,2,4]triazolo[4,3-a][1,4]diazepin hergestellt wird.

8. Verfahren nach Anspruch 1, worin 1-{3-[4-(2-Chlorphenyl)-9-methyl-6H-thieno[3,2-f][1,2,4]triazolo[4,3-a]-[1,4]diazepin-2-yl]-2-propinyl}-3,4-dihydro-2(1H)-chinolinon hergestellt wird.

9. Verfahren nach Anspruch 1, worin 2-[3-[4-(2-Chlorphenyl)-9-methyl-6H-thieno[3,2-f][1,2,4]triazolo[4,3-a]-[1,4]diazepin-2-yl]-2-propinyl]-1H-benz[de]isochinolin-1,3(2H)-dion hergestellt wird.

10. Verfahren nach Anspruch 1, worin 1-[3-[4-(2-Chlorphenyl)-9-methyl-6H-thieno[3,2-f][1,2,4]triazolo[4,3-a]-[1,4]diazepin-2-yl]-2-propinyl]-benz[cd]-indol-2(1H)-on hergestellt wird.

11. Verfahren nach Anspruch 1, worin 4-[3-[4-(2-Chlorphenyl)-9-methyl-6H-thieno[3,2-f][1,2,4]triazolo[4,3-a]-[1,4]diazepin-2-yl]-2-propinyl]-2H-1,4-benzoxazin-3(4H)-on hergestellt wird.

**12.** Verfahren zur Herstellung einer pharmazeutischen Zusammensetzung, die insbesondere verwendet wird zur Kontrolle oder Verhütung von Krankheitszuständen, die durch einen Überschuß an Plättchenaktivierungsfaktor gekennzeichnet sind oder von kardiovaskulären Erkrankungen, Lungenerkrankungen, immunologischen Störungen, entzündlichen Erkrankungen, dermatologischen Störungen, Schocks oder Transplantatabstoßungen, umfassend, daß man eine Verbindung der Formel I, wie in Anspruch 1 definiert, oder ein pharmazeutisch annehmbares Säureadditionssalz davon und, falls erwünscht, ein oder mehrere andere therapeutisch aktive Substanzen, zusammen mit einem therapeutisch inerten Träger in eine galenische Verabreichungsform bringt.

**13.** Verbindung mit einer der allgemeinen Formeln

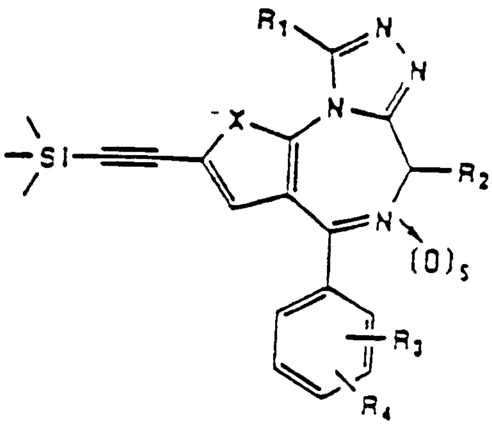

IV

und

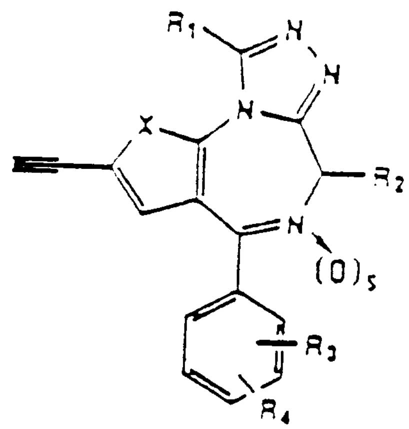

V

worin X, $R_1$, $R_2$, $R_3$, $R_4$ und s wie in Anspruch 1 definiert sind.

**Revendications**
**Revendications pour les Etats contractants suivants : AT, BE, CH, LI, DE, FR, GB, IT, LU, NL, SE**

**1.** Composés de formule générale

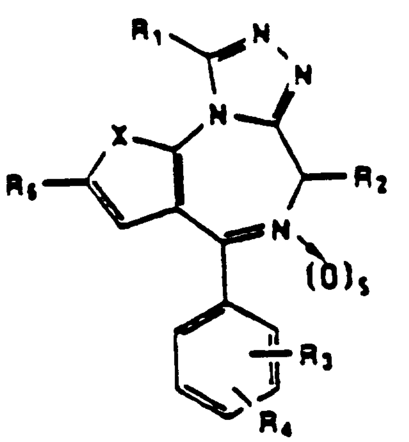

I

dans laquelle X représente -CH = CH- ou S ;
$R_1$ représente un groupe alkyle inférieur, alcoxy inférieur ou trifluorométhyle ;
$R_2$ représente un atome d'hydrogène ou un groupe alkyle inférieur, alcoxy inférieur, hydroxyle ou alcanoyloxy inférieur ;
$R_3$ et $R_4$ représentent, de manière indépendante, un atome d'hydrogène, de chlore, de fluor ou un groupe alkyle inférieur ou alcoxy inférieur ;

$R_5$ représente un radical de formule $R_6$-$(CH_2)_n$-C≡C- ou $R_7$-O-$(CH_2)_m$-C≡C- ;
$R_6$ et $R_7$ représentent un groupe aryle ou un radical hétérocyclique;
n est un nombre entier de 0 à 2; m est un nombre entier de 1 à 2 et s est un nombre entier de 0 à 1 ;
où le terme "alkyle inférieur" désigne un groupe hydrocarboné saturé, à chaîne linéaire ou ramifiée, contenant de 1 à 7 atomes de carbone , le terme "alcoxy inférieur" désigne un groupe éther alkylique dans lequel le groupe alkyle est tel que décrit ci-dessus, et le terme "radical hétérocyclique" désigne un radical hétérocyclique monocyclique à 5-, 6-ou 7 chaînons ou un radical hétérocyclique bi- ou tri-cyclique contenant un ou plusieurs hétéroatomes, choisis parmi l'azote, l'oxygène et le soufre, ce radical pouvant être substitué par des groupes alkyle inférieur, alcoxy inférieur, oxo, hydroxyle, l'atome de chlore ou de fluor ; à la condition que, lorsque s vaut 1, $R_2$ ne peut pas être un groupe hydroxyle, alcoxy inférieur ou alcanoyloxy inférieur ; lorsque n vaut 0, $R_6$ doit être fixé par l'intermédiaire d'une liaison carbone à carbone; et $R_7$ est toujours fixé par l'intermédiaire d'une liaison carbone à oxygène, et, lorsqu'au moins un centre asymétrique est présent, leurs énantiomères et racémates, et leurs sels d'addition d'acides pharmaceutiquement acceptables.

**2.** Composés selon la revendication 1, dans lesquels X représente -CH=CH- ou S ; $R_1$ représente un groupe alkyle inférieur ou alcoxy inférieur; $R_2$ représente un atome d'hydrogène ou un groupe alkyle inférieur ou alcoxy inférieur ; $R_3$ et $R_4$ représentent, indépendamment, un atome d'hydrogène, de chlore, de fluor ou un groupe alkyle inférieur ou alcoxy inférieur ; $R_5$ représente un radical de formule $R_6$-$(CH_2)_n$-C≡C- ou $R_7$-O-$(CH_2)_m$-C≡C-, $R_6$ et $R_7$ représentent un groupe aryle ou un radical hétérocyclique, n est un nombre entier de 0 à 2 ; m est un nombre entier de 1 à 2, et s représente le nombre entier 0 ; où le terme "alkyle inférieur" désigne un groupe hydrocarboné saturé, à chaîne linéaire ou ramifiée, contenant de 1 à 7 atomes de carbone , le terme "alcoxy inférieur" désigne un groupe éther alkylique dans lequel le groupe alkyle est tel que décrit ci-dessus, et le terme "radical hétéro-cyclique" désigne un radical hétérocyclique monocyclique à 5-, 6- ou 7 chaînons ou un radical hétérocyclique bi- ou tri-cyclique contenant un ou plusieurs hétéroatomes, choisis parmi l'azote, l'oxygène et le soufre, ce radical pouvant être substitué par des groupes alkyle inférieur, alcoxy inférieur, oxo, hydroxyle, l'atome de chlore ou de fluor ; à la condition que, lorsque n vaut 0, $R_6$ doit être fixé par l'intermédiaire d'une liaison carbone à carbone et que $R_7$ est toujours fixé par l'intermédiaire d'une liaison carbone à oxygène.

**3.** Composés selon la revendication 1 ou 2, dans lesquels $R_1$ représente un groupe méthyle ou éthyle, $R_2$ représente un atome d'hydrogène, $R_3$ représente un atome de fluor ou de chlore, $R_4$ représente un atome d'hydrogène, s vaut 0 et n vaut 1 ou 2.

**4.** Composés selon la revendication 1 ou 2, dans lesquels $R_1$ représente un groupe méthyle, $R_2$ représente un atome d'hydrogène, $R_3$ représente un atome de fluor ou de chlore en position 2 du fragment phényle, $R_4$ représente un atome d'hydrogène, s vaut 0, m et n valent 1, $R_6$ représente un radical hétérocyclique bi- ou tricyclique et $R_7$ représente un groupe aryle.

**5.** Composés selon la revendication 1 ou 2, dans lesquels X représente S, $R_1$ représente un groupe méthyle, $R_2$ représente un atome d'hydrogène, $R_3$ représente un atome de chlore en position 2 du fragment phényle, $R_4$ représente un atome d'hydrogène, s vaut 0, $R_5$ représente $R_6$-$(CH_2)_n$-C≡C-, n vaut 1 et $R_6$ représente

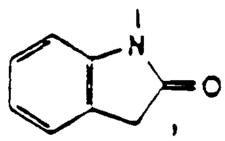 , 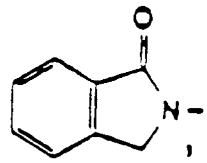 , 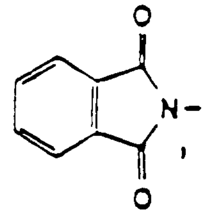 ,

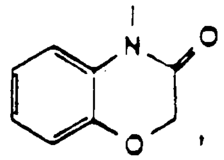 , 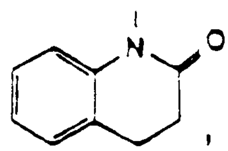 , 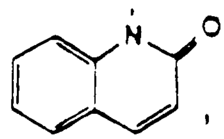 ,

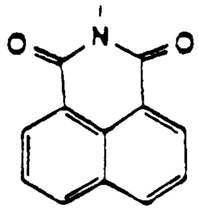 , 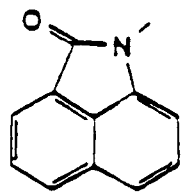 , 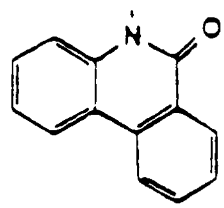 et

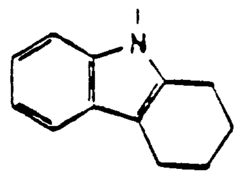

**6.** 5-(3-(4-(2-chlorophényl)-9-méthyl-6H-thiéno(3,2-f)-(1,2,4)triazolo(4,3-a)(1,4)diazépin-2-yl)-2-propynyl)-phénanthridin-6(5H)-one.

**7.** 4-(2-chlorophényl)-2-(3-(1,2,3,4-tétrahydro-9H-carbazol-9-yl)-1-propynyl)-9-méthyl-6H-thiéno(3,2-f)-(1,2,4)triazolo(4,3-a)(1,4)diazépine.

**8.** 1-(3-(4-(2-chlorophényl)-9-méthyl-6H-thiéno(3,2-f)(1,2,4)triazolo(4,3-a)(1,4)diazépin-2-yl)-2-propynyl)-3,4-dihydro-2(1H)-quinolinone.

**9.** 2-(3-(4-(2-chlorophényl)-9-méthyl-6H-thiéno(3,2-f)(1,2,4)-triazolo(4,3-a)(1,4)diazépin-2-yl)-2-propynyl)-1H-benz(de)isoquinoléine-1,3(2H)-dione.

**10.** 1-(3-(4-(2-chlorophényl)-9-méthyl-6H-thiéno(3,2-f)(1,2,4)triazolo(4,3-a)(1,4)diazépin-2-yl)-2-propynyl)-benz(cd)indol-2(1H)-one.

**11.** 4-(3-(4-(2-chlorophényl)-9-méthyl-6H-thiéno(3,2-f)(1,2,4)triazolo(4,3-a)(1,4)diazépin-2-yl)-2-propynyl)-2H-1,4-benzoxazin-3(4H)-one.

**12.** 1-(3-(4-(2-chlorophényl)-9-méthyl-6H-thiéno(3,2-f)(1,2,4)triazolo(4,3-a)(1,4)diazépin-2-yl)-2-propynyl)-1H-indole-2,3-dione ;
1-(3-(4-(2-chlorophényl)-9-méthyl-6H-thiéno(3,2f)(1,2,4)triazolo (4,3-a)(1,4)diazépin-2-yl)-2-propynyl)-1,3-dihydro-2H-indol-2-one; ;
2-(3-(4-(2-chlorophényl)-9-méthyl-6H-thiéno(3,2f)(1,2,4)triazolo (4,3-a)(1,4)diazépin-2-yl)-2-propynyl)-1,2,4-triazolo(4,3-a)pyridin-3(2H)-one ;
2-(3-(4-(2-chlorophényl)-9-méthyl-6H-thiéno(3,2f)(1,2,4)triazolo (4,3-a)(1,4)diazépin-2-yl)-2-propynyl)-1,2-benzisothiazol-3(2H)-one 1,1-dioxyde ;
4-(2-chlorophényl)-2-(3-(1H-indazol-1-yl)-1-propynyl)-9-méthyl-6H-thiéno(3,2-f)(1,2,4)triazolo(4,3-a)(1,4)-diazépine ;
2-(3-(1H-benzimidazol-1-yl)-1-propynyl)4-(2-chlorophényl)-9-méthyl-6H-thiéno(3,2-f)(1,2,4)triazolo(4,3-a)-(1,4)diazépine;

2-(3-(6-(2-fluorophényl)-1-méthyl-4H-(1,2,4)triazolo(4,3-a)(1,4)benzodiazépin-8-yl)-2-propynyl)-1H-isoindole-1,3(2H)-dione ; et
4-(3-6-(2-fluorophényl)-1-méthyl-4H-(1,2,4)triazolo(4,3-a)(1,4)benzodiazépin-8-yl)-2-propynyl)-2H-1,4-benzoxazin-3(4H)-one.

**13.** Composés de formules générales

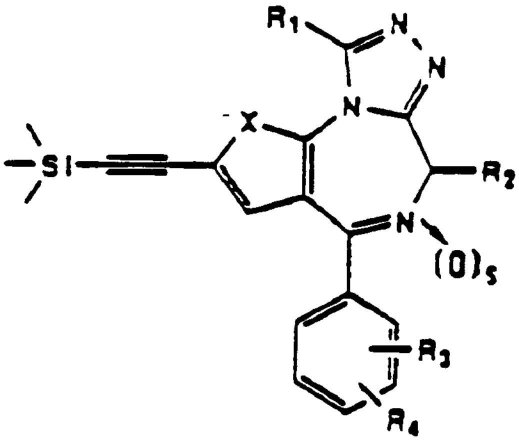

IV

et

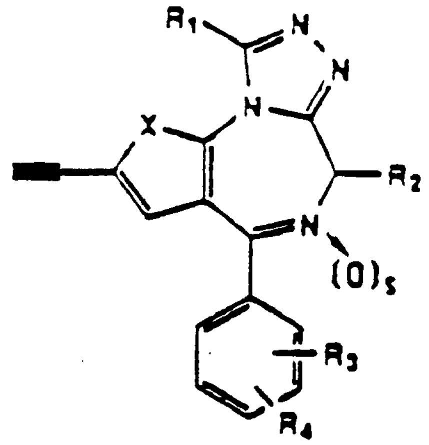

V

dans lesquels X, $R_1$, $R_2$, $R_3$, $R_4$ et s sont tels que définis dans la revendication 1.

**14.** Composés selon l'une quelconque des revendications 1 à 12 pour leur utilisation comme substances thérapeutiquement actives.

**15.** Composés selon l'une quelconque des revendications 1 12 pour leur utilisation comme antagonistes du facteur d'activation des plaquettes PAF.

**16.** Procédé de préparation des composés selon l'une quelconque des revendications 1 - 12 et de leurs sels d'addition d'acides pharmaceutiquement acceptables ce procédé comprenant

a) la réaction d'un composé de formule générale

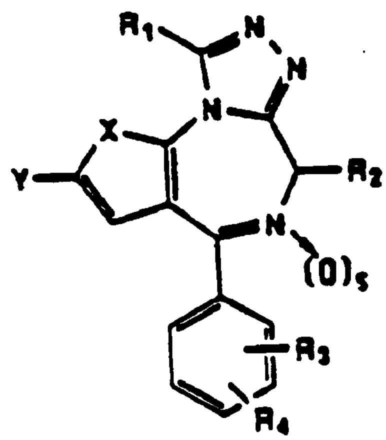

II

dans laquelle X, $R_1$, $R_2$, $R_3$, $R_4$ et s sont tels que définis dans la revendication 1, et y représente un atome de brome ou d'iode,
avec un composé de formule générale

$R_6$-$(CH_2)_n$-C≡C-H (IIIa) ou $R_7$-O-$(CH_2)_n$-C≡C-H (IIIb)

dans laquelle $R_6$, $R_7$, n et m sont tels que définis dans la revendication 1, ou
b) la réaction d un composé de formule générale

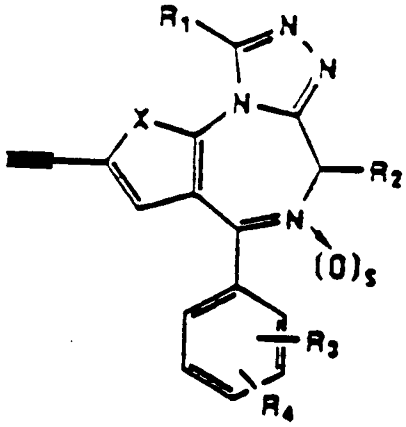

V

dans laquelle X, $R_1$, $R_2$,$R_3$,$R_4$ et s sont tels que définis dans la revendication 1, avec un composé de formule générale

$R_6$-Y

dans laquelle $R_6$ est tel que défini dans la revendication 1 et Y est tel que défini ci-dessus, ou
c) pour la préparation d'un composé de formule I, dans laquelle s vaut 1 et $R_5$ est différent d'un fragment contenant un atome d'azote basique, la réaction d'un composé de formule I, dans laquelle s vaut 0 et $R_5$ est différent d'un fragment contenant un atome d'azote basique, avec un peroxy acide, ou
d) pour la préparation d un composé de formule I, dans laquelle s vaut 0 et $R_2$ représente un groupe alcanoyloxy inférieur, la réaction d'un composé de formule I, dans laquelle s vaut 1 et $R_2$ représente un atome d'hydrogène, avec un anhydride d'acide alcanoïque inférieur, ou
e) pour la préparation d'un composé de formule I, dans laquelle $R_2$ représente un groupe hydroxyle, l'hydrolyse d'un composé de formule I, dans laquelle $R_2$ représente un groupe alcanoyloxy inférieur, et
f) si on le souhaite, la conversion d'un composé de formule générale I obtenu en un sel d'addition d'acide pharmaceutiquement acceptable.

**17.** Composition pharmaceutique comprenant un composé selon l'une quelconque des revendications 1 - 12 et un véhicule thérapeutiquement inerte.

**18.** Composition pharmaceutique qui manifeste une activité comme antagoniste du facteur d'activation des plaquettes, comprenant un composé selon l'une quelconque des revendications 1 - 12, et un véhicule thérapeutiquement inerte.

**19.** Utilisation d'un composé selon l'une quelconque des revendications 1 - 12 pour la préparation d'un médicament pour lutter contre les maladies ou prévenir les maladies.

**20.** Utilisation d'un composé selon l'une quelconque des revendications 1 - 12 pour la préparation d'un médicament pour prévenir ou lutter contre les états maladifs caractérisés par un excès de facteur d'activation des plaquettes ou les maladies cardiovasculaires, les maladies pulmonaires, les troubles immunologiques, les maladies inflammatoires, les troubles dermatologiques, les états de choc, ou les rejets du greffon.

**21.** Utilisation d'un composé selon l'une quelconque des revendications 1 - 12, pour la préparation d'un médicament qui manifeste une activité comme antagoniste du facteur d'activation des plaquettes.

**Revendications pour l'Etat contractant suivant : ES**

**1.** Procédé pour la préparation de composés de formule générale

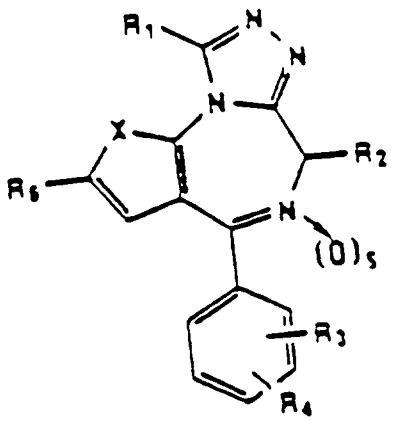

I

dans laquelle X représente -CH = CH- ou S ;
$R_1$ représente un groupe alkyle inférieur, alcoxy inférieur ou trifluorométhyle ;
$R_2$ représente un atome d'hydrogène ou un groupe alkyle inférieur, alcoxy inférieur, hydroxyle ou alcanoyloxy inférieur ;
$R_3$ et $R_4$ représentent, de manière indépendante, un atome d'hydrogène, de chlore, de fluor ou un groupe alkyle inférieur ou alcoxy inférieur ;
$R_5$ représente un radical de formule $R_6$-$(CH_2)_n$-C≡C- ou $R_7$-O-$(CH_2)_m$-C≡C- ;
$R_6$ et $R_7$ représentent un groupe aryle ou un radical hétérocyclique;
n est un nombre entier de 0 à 2; m est un nombre entier de 1 à 2 et s est un nombre entier de 0 à 1 ; où le terme "alkyle inférieur" désigne un groupe hydrocarboné saturé, à chaîne linéaire ou ramifiée, contenant de 1 à 7 atomes de carbone , le terme "alcoxy inférieur" désigne un groupe éther alkylique dans lequel le groupe alkyle est tel que décrit ci-dessus, et le terme "radical hétérocyclique" désigne un radical hétérocyclique monocyclique à 5-, 6-ou 7 chaînons ou un radical hétérocyclique bi- ou tri-cyclique contenant un ou plusieurs hétéroatomes, choisis parmi l'azote, l'oxygène et le soufre, ce radical pouvant être substitué par des groupes alkyle inférieur, alcoxy inférieur, oxo, hydroxyle, l'atome de chlore ou de fluor ; à la condition que, lorsque s vaut 1, $R_2$ ne peut pas être un groupe hydroxyle, alcoxy inférieur ou alcanoyloxy inférieur ; lorsque n vaut 0, $R_6$ doit être fixé par l'intermédiaire d'une liaison carbone à carbone; et $R_7$ est toujours fixé par l'intermédiaire d'une liaison carbone à oxygène,
et, lorsqu'au moins un centre asymétrique est présent, de leurs énantiomères et racémates, et de leurs sels d'addition d'acides pharmaceutiquement acceptables,
ce procédé comprenant

a) la réaction d'un composé de formule générale

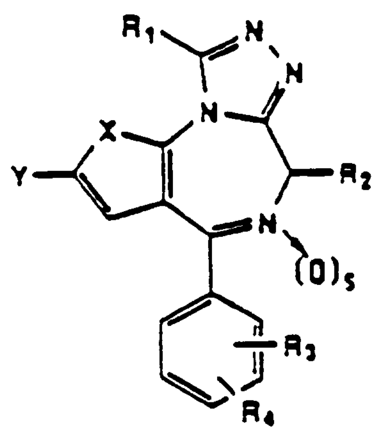

II

dans laquelle X, $R_1$, $R_2$, $R_3$, $R_4$ et s sont tels que définis ci-dessus, et y représente un atome de brome ou d'iode,
avec un composé de formule générale

$R_6$-$(CH_2)_n$-C≡C-H (IIIa) ou $R_7$-O-$(CH_2)_n$-C≡C-H (III-b)

dans laquelle $R_6$, $R_7$, n et m sont tels que définis ci-dessus, ou
b) la réaction d'un composé de formule générale

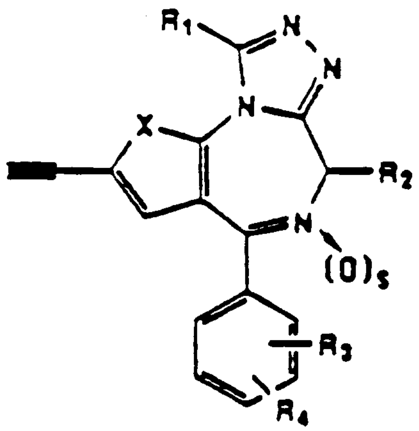

V

dans laquelle X, $R_1$, $R_2$, $R_3$,$R_4$ et s sont tels que définis ci-dessus,
avec un composé de formule générale

$R_6$-Y

dans laquelle $R_6$ et Y sont tels que définis ci-dessus, ou
c) pour la préparation d'un composé de formule I, dans laquelle s vaut 1 et $R_5$ est différent d'un fragment contenant un atome d'azote basique, la réaction d'un composé de formule I, dans laquelle s vaut 0 et $R_5$ est différent d'un fragment contenant un atome d'azote basique, avec un peroxy acide, ou
d) pour la préparation d'un composé de formule I, dans laquelle s vaut 0 et $R_2$ représente un groupe alcanoyloxy inférieur, la réaction d'un composé de formule I, dans laquelle s vaut 1 et $R_2$ représente un atome d'hydrogène, avec un anhydride d'acide alcanoïque inférieur, ou
e) pour la préparation d'un composé de formule I, dans laquelle $R_2$ représente un groupe hydroxyle, l'hydrolyse d'un composé de formule I, dans laquelle $R_2$ représente un groupe alcanoyloxy inférieur, et
f) si on le souhaite, la conversion d'un composé de formule générale I obtenu en un sel d'addition d'acides pharmaceutiquement acceptable.

**2.** Procédé selon la revendication 1, qui comprend la préparation d'un composé de formule I défini dans la revendication 1, dans lequel X représente -CH=CH- ou S ; $R_1$ représente un groupe alkyle inférieur ou alcoxy inférieur; $R_2$ représente un atome d'hydrogène ou un groupe alkyle inférieur ou alcoxy inférieur ; $R_3$ et $R_4$ représentent, indépendamment, un atome d'hydrogène, de chlore, de fluor ou un groupe alkyle inférieur ou alcoxy inférieur ; $R_5$ représente un radical de formule $R_6$-$(CH_2)_n$-C≡C- ou $R_7$-O-$(CH_2)_m$-C≡C-, $R_6$ et $R_7$ représentent un groupe aryle ou un radical hétérocyclique, n est un nombre entier de 0 à 2 ; m est un nombre entier de 1 à 2, et s représente le nombre entier 0 ; à la condition que, lorsque n vaut 0, $R_6$ doit être fixé par l'intermédiaire d'une liaison carbone à carbone et que $R_7$ est toujours fixé par l'intermédiaire d'une liaison carbone à oxygène ; où le terme "alkyle inférieur" désigne un groupe hydrocarboné saturé, à chaîne linéaire ou ramifiée, contenant de 1 à 7 atomes de carbone , le terme "alcoxy inférieur" désigne un groupe éther alkylique dans lequel le groupe alkyle est tel que décrit ci-dessus, et le terme "radical hétéro-cyclique" désigne un radical hétérocyclique monocyclique à 5-, 6- ou 7 chaînons ou un radical hétérocyclique bi- ou tri-cyclique contenant un ou plusieurs hétéroatomes, choisis parmi l'azote, l'oxygène et le soufre, ce radical pouvant être substitué

par des groupes alkyle inférieur, alcoxy inférieur, oxo, hydroxyle, l'atome de chlore ou de fluor ; et, lorsqu'au moins un atome de carbone asymétrique est présent, de leurs énantiomères et racémates et de leurs sels d'addition d'acides pharmaceutiquement acceptables conformément aux étapes a), b) ou f).

**3.** Procédé selon la revendication 1 ou 2, dans lequel $R_1$ représente un groupe méthyle ou éthyle, $R_2$ représente un atome d'hydrogène, $R_3$ représente un atome de fluor ou de chlore, $R_4$ représente un atome d'hydrogène, s vaut 0 et n vaut 1 ou 2.

**4.** Procédé selon la revendication 1 ou 2, dans lequel $R_1$ représente un groupe méthyle, $R_2$ représente un atome d'hydrogène, $R_3$ représente un atome de fluor ou de chlore en position 2 du fragment phényle, $R_4$ représente un atome d'hydrogène, s vaut 0, m et n valent 1, $R_6$ représente un radical hétérocyclique bi- ou tricycllque et $R_7$ représente un groupe aryle.

**5.** Procédé selon la revendication 1 ou 2, dans lequel X représente S, $R_1$ représente un groupe méthyle, $R_2$ représente un atome d'hydrogène, $R_3$ représente un atome de chlore en position 2 du fragment phényle, $R_4$ représente un atome d'hydrogène, s vaut 0, $R_5$ représente $R_6$-$(CH_2)_n$-C≡C-, n vaut 1 et $R_6$ représente

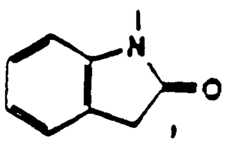 , 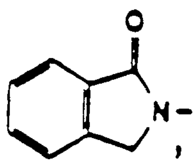 , 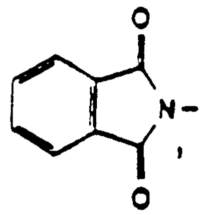 ,

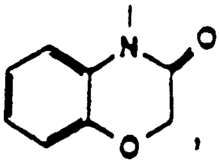 , 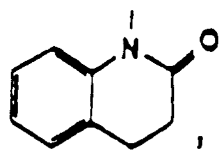 , 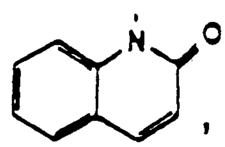 ,

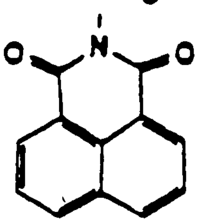 , 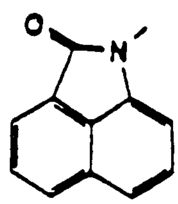 , 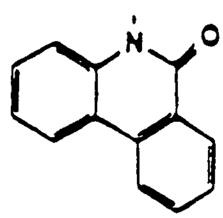 et

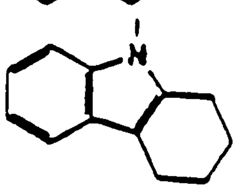

**6.** Procédé selon la revendication 1, dans lequel on prépare la 5-(3-(4-(2-chlorophényl)-9-méthyl-6H-thiéno(3,2-f)(1,2,4)-triazolo(4,3-a)(1,4)diazépin-2-yl)-2-propynyl)-phénanthridin-6(5H)-one.

**7.** Procédé selon la revendication 1, dans lequel on prépare la 4-(2-chlorophényl)-2-(3-(1,2,3,4-tétrahydro-9H-carbazol-9-yl)-1-propynyl)-9-méthyl-6H-thiéno(3,2-f)(1,2,4)triazolo (4,3-a)(1,4)diazépine.

**8.** Procédé selon la revendication 1, dans lequel on prépare la 1-(3-(4-(2-chlorophényl)-9-méthyl-6H-thiéno(3,2-f)(1,2,4)triazolo(4,3-a)(1,4)diazépin-2-yl)-2-propynyl)-3,4-dihydro -2(1H)-quinolinone.

**9.** Procédé selon la revendication 1, dans lequel on prépare la 2-(3-(4-(2-chlorophényl)-9-méthyl-6H-thiéno(3,2-f)(1,2,4)-triazolo(4,3-a)(1,4)diazépin-2-yl)-2-propynyl)-1H-benz(de)isoquinoléine-1,3(2H)-dione.

10. Procédé selon la revendication 1, dans lequel on prépare la 1-(3-(4-(2-chlorophényl)-9-méthyl-6H-thiéno(3,2-f)(1,2,4)-triazolo(4,3-a)(1,4)diazépin-2-yl)-2-propynyl)-benz(cd)-indol-2(1H)-one.

11. Procédé selon la revendication 1, dans lequel on prépare la 4-(3-(4-(2-chlorophényl)-9-méthyl-6H-thiéno(3,2-f)(1,2,4)triazolo(4,3-a)(1,4)diazépin-2-yl)-2-propynyl)-2H-1,4-benzoxazin-3(4H)-one.

12. Procédé pour la préparation d'une composition pharmaceutique, à utiliser en particulier pour maîtriser ou prévenir les états maladifs caractérisés par un excès de facteur d'activation des plaquettes ou les maladies cardiovasculaires, les maladies pulmonaires, les troubles immunologiques, les maladies inflammatoires, les troubles dermatologiques, les états de choc ou les rejets de greffon, qui consiste à amener un composé de formule I, tel que défini dans la revendication 1, ou un de ses sels d'addition d'acides pharmaceutiquement acceptable, et, si on le souhaite, une ou plusieurs autres substances thérapeutiquement actives conjointement avec un véhicule thérapeutiquement inerte, sous une forme d'administration galénique.

**Revendications pour l'Etat contractant suivant : GR**

1. Procédé pour la préparation de composés de formule générale

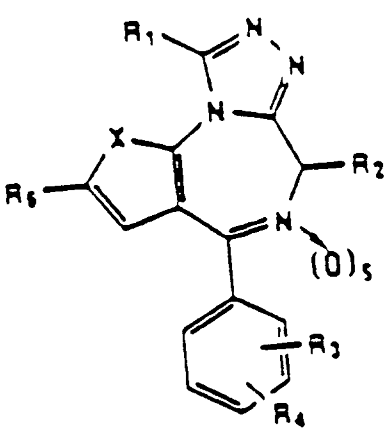

I

dans laquelle X représente -CH = CH- ou S ;
$R_1$ représente un groupe alkyle inférieur, alcoxy inférieur ou trifluorométhyle ;
$R_2$ représente un atome d'hydrogène ou un groupe alkyle inférieur, alcoxy inférieur, hydroxyle ou alcanoyloxy inférieur ;
$R_3$ et $R_4$ représentent, de manière indépendante, un atome d'hydrogène, de chlore, de fluor ou un groupe alkyle inférieur ou alcoxy inférieur ;
$R_5$ représente un radical de formule $R_6$-$(CH_2)_n$-C≡C- ou $R_7$-O-$(CH_2)_m$-C≡C- ;
$R_6$ et $R_7$ représentent un groupe aryle ou un radical hétérocyclique;
n est un nombre entier de 0 à 2; m est un nombre entier de 1 à 2 et s est un nombre entier de 0 à 1 ; où le terme "alkyle inférieur" désigne un groupe hydrocarboné saturé, à chaîne linéaire ou ramifiée, contenant de 1 à 7 atomes de carbone , le terme "alcoxy inférieur" désigne un groupe éther alkylique dans lequel le groupe alkyle est tel que décrit ci-dessus, et le terme "radical hétérocyclique" désigne un radical hétérocyclique monocyclique à 5-, 6- ou 7 chaînons ou un radical hétérocyclique bi- ou tri-cyclique contenant un ou plusieurs hétéroatomes, choisis parmi l'azote, l'oxygène et le soufre, ce radical pouvant être substitué par des groupes alkyle inférieur, alcoxy inférieur, oxo, hydroxyle, l'atome de chlore ou de fluor ; à la condition que, lorsque s vaut 1, $R_2$ ne peut pas être un groupe hydroxyle, alcoxy inférieur ou alcanoyloxy inférieur ; lorsque n vaut 0, $R_6$ doit être fixé par l'intermédiaire d'une liaison carbone à carbone; et $R_7$ est toujours fixé par l'intermédiaire d'une liaison carbone à oxygène,
et, lorsqu'au moins un centre asymétrique est présent, de leurs énantiomères et racémates, et de leurs sels d'addition d'acides pharmaceutiquement acceptables,
ce procédé comprenant

a) la réaction d un composé de formule générale

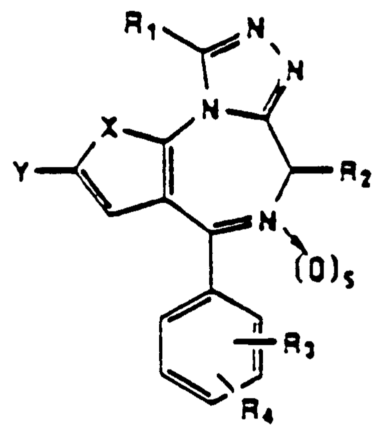

II

dans laquelle X, $R_1$, $R_2$, $R_3$, $R_4$ et s sont tels que définis ci-dessus, et y représente un atome de brome ou d'iode,
avec un composé de formule générale

$R_6$-$(CH_2)_n$-C≡C-H (IIIa) ou $R_7$-O-$(CH_2)_n$-C≡C-H (III-b)

dans laquelle $R_6$, $R_7$, n et m sont tels que définis ci-dessus, ou
b) la réaction d'un composé de formule générale

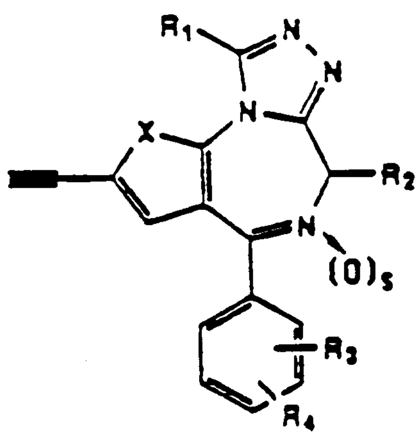

V

dans laquelle X, $R_1$, $R_2$,$R_3$,$R_4$ et s sont tels que définis ci-dessus,
avec un composé de formule générale

$R_6$-Y

dans laquelle $R_6$ et Y sont tels que définis ci-dessus, ou
c) pour la préparation d'un composé de formule I, dans laquelle s vaut 1 et $R_5$ est différent d'un fragment contenant un atome d'azote basique, la réaction d'un composé de formule I, dans laquelle s vaut 0 et $R_5$ est différent d'un fragment contenant un atome d'azote basique, avec un peroxy acide, ou
d) pour la préparation d'un composé de formule I, dans laquelle s vaut 0 et $R_2$ représente un groupe alcanoyloxy inférieur, la réaction d'un composé de formule I, dans laquelle s vaut 1 et $R_2$ représente un atome d'hydrogène, avec un anhydride d'acide alcanoïque inférieur, ou
e) pour la préparation d'un composé de formule I, dans laquelle $R_2$ représente un groupe hydroxyle, l'hydrolyse d'un composé de formule I, dans laquelle $R_2$ représente un groupe alcanoyloxy inférieur,

et

f) si on le souhaite, la conversion d'un composé de formule générale I obtenu en un sel d'addition d'acides pharmaceutiquement acceptable.

**2.** Procédé selon la revendication 1, qui comprend la préparation d'un composé de formule I défini dans la revendication 1, dans lequel X représente -CH=CH- ou S ; $R_1$ représente un groupe alkyle inférieur ou alcoxy inférieur, $R_2$ représente un atome d'hydrogène ou un groupe alkyle inférieur ou alcoxy inférieur ; $R_3$ et $R_4$ représentent, indépendamment, un atome d'hydrogène, de chlore, de fluor ou un groupe alkyle inférieur ou alcoxy inférieur ; $R_5$ représente un radical de formule $R_6$-$(CH_2)_n$-C≡C- ou $R_7$-O-$(CH_2)_m$-C≡C-, $R_6$ et $R_7$ représentent un groupe aryle ou un radical hétérocyclique, n est un nombre entier de 0 à 2 ; m est un nombre entier de 1 à 2, et s représente le nombre entier 0 ; à la condition que, lorsque n vaut 0, $R_6$ doit être fixé par l'intermédiaire d'une liaison carbone à carbone et que $R_7$ est toujours fixé par l'intermédiaire d'une liaison carbone à oxygène ; où le terme "alkyle inférieur" désigne un groupe hydrocarboné saturé, à chaîne linéaire ou ramifiée, contenant de 1 à 7 atomes de carbone , le terme "alcoxy inférieur" désigne un groupe éther alkylique dans lequel le groupe alkyle est tel que décrit ci-dessus, et le terme "radical hétéro-cyclique" désigne un radical hétérocyclique monocyclique à 5-, 6- ou 7 chaînons ou un radical hétérocyclique bi- ou tri-cyclique contenant un ou plusieurs hétéroatomes, choisis parmi l'azote, l'oxygène et le soufre, ce radical pouvant être substitué par des groupes alkyle inférieur, alcoxy inférieur, oxo, hydroxyle, l'atome de chlore ou de fluor ; et, lorsqu'au moins un atome de carbone asymétrique est présent, de leurs énantiomères et racémates et de leurs sels d'addition d'acides pharmaceutiquement acceptables conformément aux étapes a), b) ou f).

**3.** Procédé selon la revendication 1 ou 2, dans lequel $R_1$ représente un groupe méthyle ou éthyle, $R_2$ représente un atome d'hydrogène, $R_3$ représente un atome de fluor ou de chlore $R_4$ représente un atome d'hydrogène, s vaut 0 et n vaut 1 ou 2.

**4.** Procédé selon la revendication 1 ou 2, dans lequel $R_1$ représente un groupe méthyle, $R_2$ représente un atome d'hydrogène, $R_3$ représente un atome de fluor ou de chlore en position 2 du fragment phényle, $R_4$ représente un atome d'hydrogène, s vaut 0, m et n valent 1, $R_6$ représente un radical hétérocyclique bi- ou tricyclique et $R_7$ représente un groupe aryle.

**5.** Procédé selon la revendication 1 ou 2, dans lequel X représente S, $R_1$ représente un groupe méthyle, $R_2$ représente un atome d'hydrogène, $R_3$ représente un atome de chlore en position 2 du fragment phényle, $R_4$ représente un atome d'hydrogène, s vaut 0, $R_5$ représente $R_6$-$(CH_2)_n$-C≡C-, n vaut 1 et $R_6$ représente

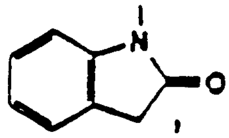, 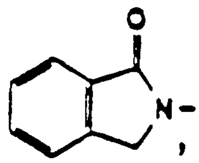, 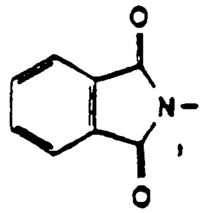,

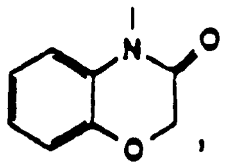, 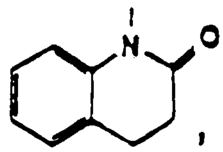, 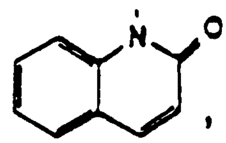,

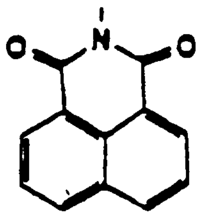, 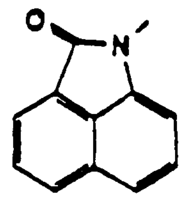, 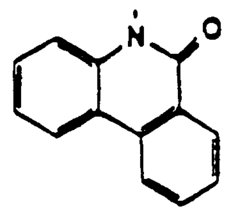 et

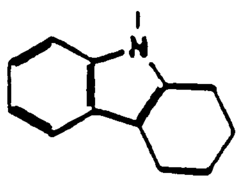

6. Procédé selon la revendication 1, dans lequel on prépare la 5-(3-(4-(2-chlorophényl)-9-méthyl-6H-thiéno(3,2-f)(1,2,4)-triazolo(4,3-a)(1,4)diazépin-2-yl)-2-propynyl)-phénanthridin-6(5H)-one.

7. Procédé selon la revendication 1, dans lequel on prépare la 4-(2-chlorophényl)-2-(3-(1,2,3,4-tétrahydro-9H-carbazol-9-yl)-1-propynyl)-9-méthyl-6H-thiéno(3,2-f)(1,2,4)triazolo (4,3-a)(1,4)diazépine.

8. Procédé selon la revendication 1, dans lequel on prépare la 1-(3-(4-(2-chlorophényl)-9-méthyl-6H-thiéno(3,2-f)(1,2,4)triazolo(4,3-a)(1,4)diazépin-2-yl)-2-propynyl)-3,4-dihydro -2(1H)-quinolinone.

9. Procédé selon la revendication 1, dans lequel on prépare la 2-(3-(4-(2-chlorophényl)-9-méthyl-6H-thiéno(3,2-f)(1,2,4)-triazolo(4,3-a)(1,4)diazépin-2-yl)-2-propynyl)-1H-benz(de)isoquinoléine-1,3(2H)-dione.

10. Procédé selon la revendication 1, dans lequel on prépare la 1-(3-(4-(2-chlorophényl)-9-méthyl-6H-thiéno(3,2-f)(1,2,4)-triazolo(4,3-a)(1,4)diazépin-2-yl)-2-propynyl)-benz(cd)-indol-2(1H)-one.

11. Procédé selon la revendication 1, dans lequel on prépare la 4-(3-(4-(2-chlorophényl)-9-méthyl-6H-thiéno(3,2-f)(1,2,4)triazolo(4,3-a)(1,4)diazépin-2-yl)-2-propynyl)-2H-1,4-benzoxazin-3(4H)-one.

12. Procédé pour la préparation d'une composition pharmaceutique, à utiliser en particulier pour maîtriser ou prévenir les états maladifs caractérisés par un excès de facteur d'activation des plaquettes ou les maladies cardiovasculaires, les maladies pulmonaires, les troubles immunologiques, les maladies inflammatoires, les troubles dermatologiques, les états de choc ou les rejets de greffon, qui consiste à amener un composé de formule I, tel que défini dans la revendication 1, ou un de ses sels d'addition d'acides pharmaceutiquement acceptable, et, si on le souhaite, une ou plusieurs autres substances thérapeutiquement actives conjointement avec un véhicule thérapeutiquement inerte, sous une forme d'administration galénique.

**13.** Composés de formules générales

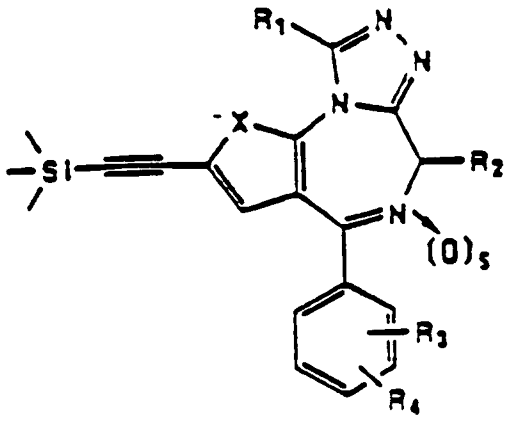

IV

et

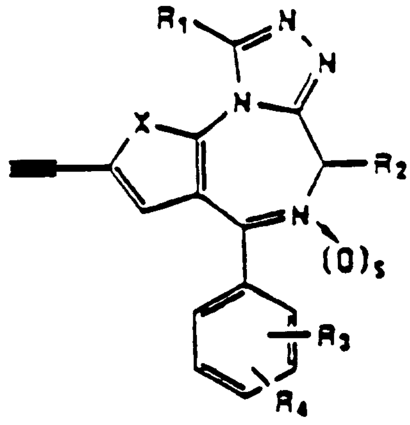

V

dans lesquels X, $R_1$, $R_2$, $R_3$, $R_4$ et s sont tels que définis dans la revendication 1.